(19) Europäisches Patentamt
European Patent Office
Office européen des brevets

(11) Publication number: **0 095 901 B1**

# (12) EUROPEAN PATENT SPECIFICATION

(45) Date of publication of patent specification: **09.09.92**

(51) Int. Cl.[5]: **C07C 59/64**, C07C 59/52, C07C 51/487

(21) Application number: **83303047.1**

(22) Date of filing: **26.05.83**

(54) **Process for the preparation of (d) 2-(d-methoxy-2-naphthyl) propionic acid and pharmaceutically acceptable salts thereof and new intermediates therefor.**

(30) Priority: **27.05.82 US 382499**
**30.09.82 US 429633**

(43) Date of publication of application:
**07.12.83 Bulletin 83/49**

(45) Publication of the grant of the patent:
**09.09.92 Bulletin 92/37**

(84) Designated Contracting States:
**AT BE CH DE FR GB IT LI LU NL SE**

(56) References cited:
**GB-A- 2 098 981**

(73) Proprietor: **SYNTEX PHARMACEUTICALS INTERNATIONAL LIMITED**
**Corner House Church Street**
**Hamilton 5(BM)**

(72) Inventor: **von Morze, Herwig**
**1220 St. Joseph Avenue**
**Los Altos California 94022(US)**

(74) Representative: **Armitage, Ian Michael et al**
**MEWBURN ELLIS & CO. 2/3 Cursitor Street**
**London EC4A 1BO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

### Field of the Invention

This invention relates to a process for the preparation of (d)-2-(6-methoxy-2-naphthyl)propionic acid and pharmaceutically acceptable salts, in the following herein described as naproxen and naproxen salts respectively.

Naproxen is a well-known anti-inflammatory, analgesic and anti-pyretic agent.

### Background of the Invention and Prior Art

Naproxen and naproxen salts are optically active substances which, in general, can be prepared from mixtures of the (d) and (l) enantiomer.

European Patent Application 79102502.6 and European Patent Application 80103828.2 describe the preparation of naproxen and naproxen salts by employing an N-R-D-glucamine or a salt thereof, where R is alkyl having 1 to 36 carbon atoms, or cycloalkyl having 3 to 8 carbon atoms as resolving agent. The resolution method described in these two European patent applications includes forming salts of mixtures of the two enantiomers with the N-R-D-glucamine which are then subjected to fractional crystallization and the less soluble naproxen salt is separated from the other, more soluble enantiomer salt by crystallization. British Patent Specification No. 1,274,271 describes the preparation of d,l-2-(5-halo-6-methoxy-2-naphthyl)-propionic acids and that these acids can be resolved with alkaloids such as cinchonidine.

GB-A-2098981 was published after the priority dates of the present application, and discloses a process involving the use of N-methyl-D-glucamine to resolve dl-2-(5-halo-6-methoxy-2-naphthyl)propionic acid by fractional crystallization of the d-salt from a specific solvent system, followed by hydrogenation of the 5-halo group. Published counterparts to GB-A-2098981 exist in other designated states, for example, CH 651289 and AT-B-390 253.

EP 7116 and EP 22529 disclose the use of N-methyl-D-glucamine and N-(higher)alkyl D-glucamine as resolving agents in the preparation of naproxen or derivatives thereof. However they do not suggest the resolution of naproxen precursors, wherein one or more additional steps have to be carried out on the resolved product to convert the precursor to naproxen.

### Brief Summary of the Invention

It has now been found that N-R-D-glucamines or salts thereof, where R is alkyl having 1 to 18 carbon atoms, preferably 2 to 18 carbon atoms or cycloalkyl with 3 to 8 carbon atoms, preferably N-R-D-glucamines containing 5 to 8 carbon atoms can be employed as resolving agents for the preparation of certain naproxen precursors which can be readily converted to naproxen. Therefore, the present invention comprises N-R-D-glucamines or salts thereof as resolving agents for the preparation of naproxen precursors. These resolving agents form diastereomeric (dl) naproxen precursor salts from which the naproxen precursor salt can be isolated. The preferred method of separation involves fractional crystallization of the diastereomeric salts.

The present invention thus entails the use of these resolving agents with naproxen precursors which, while sharing the propionic acid function with naproxen, are characterized by the presence of substituents located in that part of the naproxen molecule which is remote from the center of the chirality of the molecule. These substituents can be readily converted to the naproxen substitution. In a preferred embodiment, the distance of these convertible substituents between their point of attachment to the naphthalene ring and the center of chirality, i.e., the carbon atom to which the methyl group is attached, is between about 5.8 to 6.6A. Substituents that are specifically preferred according to the present invention are halogen substituents in position 5 of the naphthyl ring and a hydroxy group in position 6 of the molecule. Both groups can be readily converted to the substituents of naproxen (i.e., a hydrogen atom in position 5 and a methoxy group in position 6), while retaining the stereoconfiguration of the molecule.

More specifically the naproxen precursors are represented by the compounds of formula (I)

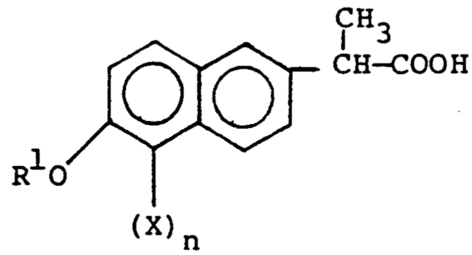

(I)

wherein $R^1$ is hydrogen or methyl when X is halogen selected from the group of chloro and bromo, and n is 1; or $R^1$ is hydrogen when n is 0. These naproxen precursors also include salts of the compounds of formula (I).

The compounds of formula (I) in form of their racemic mixtures are known compounds. For example, the compound (d,l)-2-(5-bromo-6-methoxy-2-naphthyl)propionic acid is described in German Patent 1,934,460 (this is the compound of formula (I) wherein $R^1$ is methyl, X is bromo and n is 1). The compound of formula (I) wherein $R^1$ is methyl, X is chloro and n is 1 is described, in form of its racemic mixture, in Belgian Patent 752,627. The compounds of formula (I) wherein $R^1$ is hydrogen, X is halogen and n is 0 or 1 are described, in form of their racemic mixtures, in German Application No. 1793825 and in Italian Patent Application 25778-A/76. However many of the salts of the compounds of formula (I) and the individual enantiomers of the compounds of formula (I) have not been before described. CH-A-651289 and GB-A-2098981 and other corresponding publications describe and claim certain aspects of the resolution of d- and l-2-(5-bromo-6-methoxy-2 naphthyl) proprionic acid with N-methyl-D-glucamine. However, these specifications were not published before the filing of the present application. Subject, therefore, to the terms of the appended claims;

in summary, the process of this invention comprises resolving mixtures of the (d) and (l) enantiomers of formula (I) or soluble salts thereof with an N-R-D-glucamine or salt thereof, wherein R is alkyl having 1 to 18 carbon atoms, preferably 2 to 18 carbon atoms, or 6 to 18 carbon atoms, or cycloalkyl having 3 to 8 carbon atoms to yield a product substantially enriched in the salts of the naproxen precursor with the N-R-D-glucamine which is cleaved to the free naproxen precursor, which then in turn can be converted to naproxen or a pharmaceutically acceptable naproxen salt. Racemic mixtures of the compounds of formula (I) as they occur after known chemical syntheses are the preferred starting materials for the process of the present invention.

The term "alkyl" as used herein refers to and includes straight and branched chain hydrocarbon groups having 1 to 18 carbon atoms. Typical alkyl groups include methyl, ethyl, n-propyl, i-propyl, n-butyl, i-butyl, n-hexyl, n-octyl, n-dodecyl, and n-octadecyl, and the like.

The term "cycloalkyl" as used herein refers to and includes cycloaliphatic hydrocarbon groups having 3 to 8 carbon atoms. Typical cycloalkyl groups include cyclopropyl, cyclobutyl, cyclopentyl, cyclohexyl, methylcyclohexyl, cycloheptyl and cyclooctyl. Of the cycloalkyl groups, cyclohexyl and cycloheptyl are presently preferred.

Presently preferred resolving agents include those N-alkyl glucamines wherein R is alkyl having 2 to 18 carbon atoms, and more preferred agents are wherein R is alkyl having 6 to 18 carbon atoms.

Presently particularly preferred resolving agents within the scope of this invention are N-ethyl-D-glucamine, N-n-propyl-D-glucamine, N-n-butyl-D-glucamine, and N-n-octyl-D-glucamine, N-n-decyl-D-glucamine and n-hexadecyl-D-glucamine.

A presently preferred resolving agent is N-methyl-D-glucamine in connection with the naproxen precursor of formula (I), wherein $R^1$ is hydrogen and X is halogen from the group of chloro and bromo and n is 1; or $R^1$ is hydrogen and n is 0.

The resolution step of this invention is conducted in an inert organic solvent having a pronounced difference between the solubilities of the salt of the naproxen precursor of formula (I) with the resolving agent and the salt of the other enantiomer with the resolving agent, generally at temperatures between room or ambient temperature and an elevated temperature generally up to the reflux temperature of the solvent utilized. The salt of the naproxen precursor with the resolving agent (for example, N-methyl-D-glucamine, N-n-propyl-D-glucamine, N-n-butyl-D-glucamine or N-n-octyl-D-glucamine) should be significantly less soluble in the selected solvent than is the salt of the other enantiomer thereof with the resolving agent and, accordingly, upon the cooling of a heated solution thereof, generally to or about ambient or room temperature, such salt of the naproxen precursor with the resolving agent will be preferentially crystallized therefrom. Suitable solvents include water, $C_1$ to $C_{10}$ monohydric alcohols (in particular $C_1$ to $C_{10}$ alkanols),

such as, for example, methanol, ethanol, n-propanol, isopropanol, butanol, pentanol, hexanol, cyclohexanol, 2-ethylhexanol, benzyl alcohol, furfuryl alcohol, and the like, $C_2$ to $C_6$ dihydric alcohols, such as, for example, ethylene glycol, 1,2-propylene glycol, 1,3-propylene glycol, and the like, $C_3$ to $C_4$ trihydric alcohols, such as for example, glycerol, and the like, $C_3$ to $C_{11}$ ketones,such as, for example, acetone, acetylacetone, ethyl methyl ketone, diethyl ketone, di-n-propyl ketone, diisopropyl ketone, diisobutyl ketone, and the like. Other solvents include mono- and di(lower)alkyl ether of ethylene glycol and diethylene glycol, dimethylsulfoxide, sulfolanes, formamide, dimethylformamide, N-methyl pyrrolidone, pyridine, dioxane, dimethylacetamide, and the like. The $C_1$ to $C_3$ alcohols, e.g. methanol and isopropanol, particularly methanol, are the presently preferred solvents. Sufficient water can be added to the solvent if needed to solubilize all of the materials which have been added thereto. Mixtures of polar solvents with aromatic hydrocarbons such as toluene or benzene, for example methanol and toluene, with methanol being the major component, can be used as solvent medium.

The starting material (i.e., the mixture of the naproxen precursors and the other enantiomer thereof or soluble salts thereof) is heated to an elevated temperature, generally to a temperature in the range from about 60° C to about 100° C or the reflux temperature of the solvent, in the presence of the resolving agent to solubilize all of the materials which have been added to the solvent. If desired, the solvent can be held at the elevated temperature until all of the materials have gone into solution. After the solution has been held at the elevated temperature for the desired length of time, it is slowly cooled to ambient temperature. During the cooling process, the solution is preferably seeded with a salt of the naproxen precursor and the resolving agent. The crystalline precipitate which results is enriched in the salt of the naproxen precursor with the resolving agent. The final temperature to which the solution is taken is chosen by practical considerations but generally is selected so that the temperature difference will be sufficient to provide a high yield of crystals. The crystallizing mixture can be maintained at the lower temperature until crystallization is complete, or nearly so, usually for about 30 minutes to about several hours or so. The crystalline precipitate which results is removed by filtration and washed.

The crystalline material which is obtained at this stage in the process (i.e., a material which is enriched in the salt of the naproxen precursor with the resolving agent), after separation by filtration and washing, can be charged to water and heated, if necessary, to redissolve the crystalline material. For those N-R-D-glucamines which are soluble in water, the resulting solution is acidified for example with a mineral acid, such as sulphuric acid or hydrochloric acid, or an organic acid such as acetic acid, and the crystalline precipitate so obtained is separated by filtration, washed and dried. There results a crystalline product substantially enriched in the naproxen precursor. Alternatively, for those N-R-D-glucamines which are insoluble in water, the material enriched in the salt of the naproxen precursor with such a resolving agent can be treated with a strong base, such as, for example, potassium hydroxide or other strong base having a pKa value greater than 10, to cleave the salt, followed by filtration to remove the resolving agent and acidification of the filtrate with, for example, a mineral acid such as hydrochloric acid or sulfuric acid, or an organic acid such as acetic acid, to give, after filtration, washing and drying, a crystalline product substantially enriched in the naproxen precursor.

Prior to the cleavage of the material enriched in the salt of the naproxen precursor with the resolving agent to obtain the optically pure naproxen precursor, it is generally desirable to redissolve the enriched salt material in further solvent material, heat the solvent to the desired (normally elevated) temperature, seed the resultant solution with the salt of the naproxen precursor with the resolving agent, and cool the resultant solution to effect one or more further recrystallizations. Each such recrystallization further increases the proportion of the salt of the naproxen precursor with the resolving agent in the recrystallized material. N-methyl-D-glucamine, N-ethyl-D-glucamine, N-n-propyl-D-glucamine, N-n-butyl-D-glucamine, N-n-hexyl-D-glucamine and N-n-octyl-D-glucamine are particularly suitable resolving agents within the scope of this invention because, with merely one recrystallization step prior to the redissolution of the resultant crystalline product and subsequent acidification, a product having a purity on the order of about of 97-99% of pure naproxen precursor can be obtained.

N-methyl-D-glucamine, N-ethyl-D-glucamine, and N-n-octyl-D-glucamine, however, are the presently preferred resolving agents because their naproxen precursor salts are readily recoverable by filtration, and the naproxen precursor of acceptably high optical purity can be obtained without need for one or more recrystallizations prior to the cleaving step, and the resolving agent can be recovered directly and in high yield (on the order of about 97-98%) by filtration. Additionally, the cleavage can be conducted under either alkaline or acidic conditions.

The N-n-octyl-D-glucamine is substantially insoluble in water, thus permitting recovery thereof in high yields from aqueous systems. This is also true for other resolving agents of this invention where R is alkyl having at least 6 carbon atoms, e.g., those resolving agents where R is alkyl having from 6 to 18 carbon

atoms.

The material enriched in the other enantiomer of formula (I) or the N-R-D-glucamine salt thereof (where R is as defined above) can be processed to recover the other enantiomer which can then be racemized according to known techniques. See, for example, Dyson, U.S. Patent No. 3,686,183.

The amount of resolving agent employed [on a molar basis relative to the d,l acid of formula (I) being resolved] in accordance with the present invention ranges from between about 50% and 100%. However, as only about 50% (on a molar basis relative to the d,l acid being resolved) of the resolving agent is needed to form the more insoluble salt thereof with the naproxen precursor, the remainder of the resolving agent (generally on the order of up to about 40-50 molar %) can be replaced, if desired, with a more inexpensive base, including, for example, an inorganic base such as an alkali metal hydroxide, such as sodium hydroxide or potassium hydroxide, or an organic base having a pKa value of at least 8, such as an organic tertiary amine such as triethylamine, triethanolamine, tributylamine, etc.

The terms "mixture of (d) and (l) enantiomer of formula (I)" is also intended to include those salts thereof which are soluble in the solvent utilized in the resolution process of this invention. Such salts include, for example, the corresponding sodium salts, potassium salts, lithium salts, and the like. Such salts can be prepared by the addition of base, such as an alkali metal hydroxide, for example, sodium or potassium hydroxide, to a solution of the mixture of the (d) and (l) of formula (I) acid. The resulting mixture of d 2-(6-methoxy-2-naphthyl)propionic acid and l 2-(6-methoxy-2-naphthyl)propionic acid salts can be resolved according to the present invention by use of a salt of the resolving agent which will react to form a salt of d 2-(6-methoxy-2-naphthyl)propionic acid with the N-R-D-glucamine. Suitable glucamine salts include, salts of inorganic or organic acids, for example, the hydrochloride salt and the acetate salt. Other salts include the propionate salt, butyrate salt, isobutyrate salt, sulfate salt, nitrate salt, maleate salt and the like. Accordingly, the term "N-R-D-glucamine" (where R is as defined above) is intended to include those salts thereof which, when used with an appropriate salt of the mixture of d and l 2-(6-methoxy-2-naphthyl)-propionic acid, will afford the resolution contemplated hereby.

The acids of formula (I) are prepared by known methods as described earlier. See German Patent 1,934,460, Belgian Patent 752,627, German Patent Application 1,668,654 and German Patent Application 1793825 and Italian Patent Application 25778-A/76.

After the naproxen precursor of formula (I) has been obtained in optically pure or in crude form it can be converted into naproxen or pharmaceutically acceptable salts thereof. For the compounds of formula (I) wherein $R^1$ is hydrogen or methyl and X is halogen, and n is 1 the conversion to naproxen involves dehalogenation of the compounds of formula (I). This process thus entails the replacement of halogen with hydrogen. If $R^1$ is hydrogen and X is halogen and n is 1 it is preferred to methylate the compound first, as described below, and then to remove the halogen in the 5-position. The 2-(5-halo-6-methoxy-2-naphthyl)-propionic acid precursor of naproxen can be subjected to dehalogenation reactions which do not affect the structure of the remainder of the molecule under formation of naproxen. A suitable method comprises the reaction of the resolved 5-halo compounds of formula (I) with an earth alkaline metal alcoholate such as magnesium alcoholate, for example, magnesium methylate and a tertiary amine such as tri(lower)alkyl amines, for example, triethylamine in an inert solvent such as the alcohol or alkanol from which the alcoholate or alkanolate is derived. The reaction is conducted between 30 °C and the reflux temperature of the solvent. For example, a mixture of magnesium powder, methanol and a molar excess of triethylamine are mixed and the mixture maintained under an inert atmosphere such as a nitrogen atmosphere. Resolved 2-(5-halo-6-methoxy-2-naphthyl)propionic acid is added in an anhydrous methanol solution. After the reaction is completed, i.e. after one hour at reflux temperature, hydrochloric acid is added to the reaction mixture to dissolve all the remaining magnesium. From the reaction mixture naproxen can be recovered according to known methods. For example, the reaction mixture containing naproxen can be poured into water and extracted with a suitable solvent such as methylene chloride. The organic layer is separated, washed with water and pure naproxen crystallizes out. In another variant, the naproxen precursor of formula (I) is reacted with at least 2 preferably 2 to 50 mole equivalents (calculated on the basis of aluminum) of a nickel aluminum or cobalt aluminum alloy in the presence of at least 2, preferably 2 to 50 mole equivalents of alkali metal hydroxide in an inert solvent until the 5-halo group is removed. The preferred nickel aluminum or cobalt aluminum alloys have a particle size of under one millimeter and the aluminum concentration should be at least 10% by weight of the alloy. Suitable alkali metal hydroxides are sodium hydroxide, potassium hydroxide or lithium hydroxide. The reaction is conducted in an inert solvent which is preferably methanol, ethanol, propanol, isopropanol, tertiary butanol, tetrahydrofuran, or dioxan. The reaction temperature is between 0 °C and the reflux temperature of the solvent, preferably at least 40 °C. The reaction time is 15 minutes to 12 hours.

The resolved 5-halo compounds of formula (I) can be also reacted directly with a mixture of magnesium

(preferably as a powder having a particle size of less than one millimeter), a lower alkanol and optionally a molar excess of an aliphatic amine until naproxen is formed. This reaction is conducted at a temperature of from 0°C to the reflux temperature of the reaction mixture, preferably at least 20°C and under an inert atmosphere such as nitrogen. The time required for reduction of the 5-halo group depends upon the reaction temperature. Usually from 10 minutes to 12 hours is sufficient for this reaction. At reflux temperatures, the reaction is completed within an hour.

Alternatively, the 5-halo compound of formula (I) is treated with Raney nickel in an inert organic solvent such as a lower alkanol, an ester having up to 6 carbon atoms such as ethyl acetate, or a lower alkanoic acid such as acetic acid, propionic acid etc., optionally under hydrogen. The reaction is carried out at a temperature of at least 0°C and preferably above 20°C for from 10 minutes to 24 hours. Reflux temperature is preferred.

In a further alternative, the 5-halo compounds of formula (I) are treated with palladium-on-charcoal, platinum or platinum oxide with hydrogen in an inert organic solvent such as those described for treatment with Raney nickel. The reaction is carried out at a temperature of from 0°C up to the reflux temperature of the solvent, room temperature being preferred, for from 10 minutes to 12 hours.

The crude naproxen precursor of formula (I), wherein $R^1$ is hydrogen and n is 0 can be converted directly into naproxen. Likewise, the material can be purified by crystallization to the desired optical rotation and then converted to naproxen. The conversion to naproxen is effected by treating the naproxen precursor with a suitable methylating agent.

The methylation entails the reaction of dimethylsulfate, dimethylcarbonate or a methyl halide in an alkaline medium with the naproxen precursor of formula (I), i.e. the 6-hydroxy compound. As an appropriate solvent water or lower aliphatic alcohols can be used. Sodium hydroxide, potassium hydroxide, sodium carbonate and potassium carbonate can be used for forming the alkaline medium. The reaction is carried out at the temperature in the range of from 40°C to the reflux temperature of the reaction mixture. An optimum reaction temperature would be 40 to 70°C. The reaction time depends on the reaction temperature but, in general, the reaction is completed into 1 to 10 hours. The isolation of naproxen can be accomplished by adding a solution of an organic or inorganic strong acid to the reaction mixture which has been cooled to acidify the mixture and is filtered off. The recovered precipitate will be washed and then dried at a temperature at which naproxen is unaffected by the drying procedure. The naproxen precursor of formula I, wherein R is hydrogen, X is halogen and n is 1 can be methylated according to the above described procedure and then dehalogenated as described above.

Naproxen obtained from either dehalogenation of the 5-halo compound of formula (I) or by methylation of the 6-hydroxy compound of formula (I) can be recrystallized to obtain material of very good optical rotation, i.e. with $[\alpha]_D$ being between 62-67° (chloroform).

Naproxen obtained by the method of the present invention can be converted into pharmaceutically acceptable salts by reaction of naproxen with an at least equivalent amount of organic and inorganic base preferably sodium hydroxide. This reaction is conducted in an inert solvent at a temperature between 0° and reflux temperature of the solvent and the naproxen salt is crystallized from the solvent, optionally after the removal of part or all of the solvent. Crystalline salt material can be recrystallized to increase its purity.

## EXAMPLE 1

50 g of d,l-2-(6-hydroxy-2-naphthyl)propionic acid is heated in 215 ml of methanol and 13.8 ml of toluene to dissolve the d,l-2-(6-hydroxy-2-naphthyl)propionic acid. 67.8 g of N-n-octyl-D-glucamine is added and the solution is cooled to 40°C and seeded with crystals of the salt of d 2-(6-hydroxy-2-naphthyl)-propionic acid with N-n-octyl-D-glucamine. The reaction mixture is stirred overnight at room temperature to give the salt of d 2-(6-hydroxy-2-naphthyl)propionic acid with N-n-octyl-D-glucamine which, after cleavage, affords 11.5 g of d-2-(6-hydroxy-2-naphthyl)propionic acid of specific rotation of +91.3° in chloroform in 23.0% yield.

10.5 g of the 6-hydroxy-naproxen precursor is dissolved in 60 ml of acetone and 100 ml of a 1.5 N aqueous solution of potassium hydroxide and 44 g of methyl iodide is added to the solution. The solution is maintained at 0°-20°C for 14 hours. The naproxen methyl ester obtained is hydrolyzed in situ to naproxen under the basic reaction conditions. The conversion from the 6-hydroxy-naproxen precursor to naproxen is achieved with a 90.3% yield. The specific rotation of the naproxen obtained is +63.5° in chloroform.

## EXAMPLE 2

17.0 g of d,l-2-(6-hydroxy-2-naphthyl)propionic acid and 15.34 g of N-methyl-D-glucamine are dissolved

in 150 ml of boiling methanol containing 10 ml of $H_2O$ (10% w/w). The solution is filtered to clearness and carefully cooled to 45° C with slow stirring to give the salt of the 6-hydroxy-naproxen precursor with N-methyl-D-glucamine. The salt obtained is reslurried three times from the same solvent system to afford, after cleavage, 3.1 g of d-2-(6-hydroxy-2-naphthyl)propionic acid of a specific rotation of +91.7° in chloroform in 18.3% yield.

Following the procedure described in Example 1, d-2-(6-hydroxy-2-naphthyl)propionic acid is converted to d-2-(6-methoxy-2-naphthyl)propionic acid with a specific rotation of +63.8° in chloroform.

EXAMPLE 3

25 g of d,l-2-(5-bromo-6-methoxy-2-naphthyl)propionic acid, 23.7 g of N-n-octyl-D-glucamine and 60 ml of 10% toluene in methanol is brought to reflux to dissolve the crystalline materials. The reaction mixture is held at reflux for 20 minutes and then allowed to slowly cool. At 30° C, the solution is seeded with a small amount of the N-n-octyl-D-glucamine salt of d-2-(5-bromo-6-methoxy-2-naphthyl)propionic acid. The reaction mixture is then allowed to stand overnight at room temperature. The precipitate is isolated by vacuum filtration and washed with 30 ml of cold methanol to yield 16.7 g of a material enriched in the N-n-octyl-D-glucamine salt of d-2-(5-bromo-6-methoxy-2-naphthyl)propionic acid. A 1.0 g sample of the salt is suspended in 50 ml of water, heated to 80° C and then acidified with concentrated HCl. The resultant precipitate which is enriched in d-2-(5-bromo-6-methoxy-2-naphthyl)propionic acid is isolated by filtration at 50° C, washed with water, and dried in a vacuum oven, $[\alpha]_D^{25}$ = +28.2° (C = 0.5% in $CHCl_3$).

The remaining 15.7 g of the material enriched in the salt of d-2-(5-bromo-6-methoxy-2-naphthyl)-propionic acid is dissolved in 2 ml of toluene and 18 ml of methanol at the reflux temperature of the solvent. The solution is cooled to 25° C, seeded with the N-n-octyl-D-glucamine salt of d-2-(5-bromo-6-methoxy-2-naphthyl)propionic acid and allowed to stand overnight. The precipitate is isolated by filtration and washed with 20 ml of cold methanol. The isolated material is suspended in 100 ml of water, heated to 80° C, and acidified with HCl. Isolation of the precipitate gives 5.5 g of essentially pure d-2-(5-bromo-6-methoxy-2-naphthyl)propionic acid ($[\alpha]_D^{25}$ = + 42.5°, C = 0.5% in $CHCl_3$).

The 5-bromo-naproxen precursor obtained is debrominated to yield naproxen with a specific optical rotation of 64° in chloroform, following the procedures as previously described.

EXAMPLE 4

15 g of d,l-2-(5-bromo-6-methoxy-2-naphthyl)propionic acid is heated to reflux with 2.42 g of triethylamine, 7.03 g of N-n-octyl-D-glucamine and 30 ml of 10% toluene in methanol. The reaction mixture is held at reflux for 20 minutes and then allowed to slowly cool. At 30° C, the solution is seeded with a small amount of the N-n-octyl-D-glucamine salt of d-2-(5-bromo-6-methoxy-2-naphthyl)propionic acid. The reaction is then allowed to stand overnight at room temperature. The precipitate is isolated by vacuum filtration and washed with 20 ml of cold methanol to yield 4.47 g of a material enriched in the N-n-octyl-D-glucamine salt of d-2-(5-bromo-6-methoxy-2-naphthyl)propionic acid.

The 4.47 g of the material enriched in the salt of d-2-(5-bromo-6-methoxy-2-naphthyl)propionic acid is dissolved in 1 ml of toluene and 9 ml of methanol at the reflux temperature of the solvent. The solution is cooled to 30° C, seeded with the N-n-octyl-D-glucamine salt of d-2-(5-bromo-6-methoxy-2-naphthyl)-propionic acid and allowed to stand overnight. The precipitate is isolated by filtration and washed with 10 ml of cold methanol. The isolated material is suspended in 75 ml of water, heated to 80° C, and acidified with HCl. Isolation of the precipitate gives 1.2 g of a material substantially enriched in d-2-(5-bromo-6-methoxy-2-naphthyl)propionicacid ($[\alpha]_D^{25}$ = + 42°, (C = 0.5% in $CHCl_3$).

The 5-bromo-naproxen precursor obtained is debrominated to yield naproxen following the procedures described previously.

EXAMPLE 5

5.30 g of d,l 2-(5-chloro-6-methoxy-2-naphthyl)propionic acid is heated with 1.01 g of triethylamine (0.5 equivalent) in 35 ml of isopropanol to about the reflux temperature of the solvent to dissolve the d,l acid. 2.09 G of N-ethyl-D-glucamine (0.5 equivalent) are added and the solution is cooled to room temperature (i.e. about 20-23° C) to give 4 g of a material enriched in the salt of the less soluble naproxen precursor acid with N-ethyl-D-glucamine. A sample of the latter is dissolved in about 25 ml of water heated to about 80° C, treated with hydrochloric acid until acidic at which time a material enriched in the naproxen precursor acid precipitates out of solution and is recovered by filtration.

1.00 g of the material enriched in the N-ethyl-D-glucamine salt is dissolved in 19.5 ml of isopropanol and 1.5 ml of water at about the reflux temperature of the solvent. The solution is cooled to room temperature to give 0.50 g of a recrystallized salt. This material is treated with hydrochloric acid as set forth above in the preceding paragraph to give a material further enriched in the naproxen precursor acid. A solution of 1 g of (d)-2-(5-chloro-6-methoxy-2-naphthyl)propionic acid in 20 ml of 10% aqueous sodium hydroxide solution at 90° C is treated with 3 g of a nickel-aluminum alloy in small portions. After stirring the mixture for two hours, it is filtered, diluted with excess diluted hydrochloric acid and extracted with methylene chloride. The organic phase is evaporated to dryness and the residue recrystallized from methylene chloride hexane to yield 2-(6-methoxy-2-naphthyl)propionic acid ($[\alpha]_D$ 60° in chloroform) after recrystallization.

Repeating the above procedure with the corresponding 5-bromo compound yields naproxen of an $[\alpha]_D$ of 61° (chloroform) after recrystallization.

EXAMPLE 6

6.2 g of d,l 2-(5-bromo-6-methoxy-2-naphthyl)propionic acid is heated with 1.01 g of triethylamine (0.5 equivalent) in 30 ml of isopropanol and 1.5 ml of water to about the reflux temperature of the solvent to dissolve the d,l acid. 2.09 g of N-ethyl-D-glucamine are added and the solution is cooled to room temperature to give 4.32 g of a material enriched in the N-ethyl-D-glucamine salt. A sample of the latter is dissolved in about 25 ml of water heated to about 80° C, treated with hydrochloric acid until acidic at which time a material enriched in the naproxen precursor acid precipitates out of solution and is recovered by filtration.

1.00 g of the material enriched in the N-ethyl-D-glucamine salt is dissolved in 20 ml of 5% aqueous isopropanol and 1.0 ml of water at about the reflux temperature of the solvent. The solution is cooled to room temperature to give 0.6 g of a first recrystallized material. A sample of the latter is treated with hydrochloric acid as set forth in the preceding paragraph to give a material further enriched in the naproxen precursor salt.

0.50 g of the first recrystallized material from the preceding paragraph is dissolved in 10 ml of 5% aqueous isopropanol and 0.5 ml of water at about the reflux temperature of the solvent. The solution is cooled to room temperature to give 0.43 g of a second recrystallized material. A sample of the latter is treated with hydrochloric acid as set forth in the first paragraph of this Example to give a material even further optically enriched.

0.33 g of the second recrystallized material from the preceding paragraph is dissolved in 12 ml of 5% aqueous isopropanol and 1.0 ml of water at about the reflux temperature of the solvent. The solution is cooled to room temperature to give 0.27 g of a third recrystallized material. The latter is treated with hydrochloric acid as set forth in the first paragraph of this Example to give substantially pure 5-bromo-naproxen precursor.

To a 250 ml flask equipped with a reflux condenser and a nitrogen bubbler magnesium powder (60 g) are added, further anhydrous methanol (50 ml) and triethylamine (10 g) . The flask is swept with nitrogen, and the nitrogen atmosphere is maintained throughout the reaction.

The 5-bromo-naproxen precursor (0.1 mole) in methanol (15 g) is slowly added, and the mixture is heated under reflux for one additional hour after the addition of the 5-bromo-naproxen precursor is complete. The cooled mixture is mixed with 6N hydrochloric acid until no magnesium remains. The mixture is poured into water and extracted with methylene chloride. The organic layer is separated, washed with water and crystallized by concentrating the solution and adding hexane. After recrystallization naproxen is obtained with a specific optical rotation $[\alpha]_D$ of 64° in chloroform.

Repeating this procedure with the corresponding 5-chloro-naproxen precursor naproxen is obtained with a specific optical rotation of 62° in chloroform.

EXAMPLE 7

3 g of d,l 2-(5-chloro-6-methoxy-2-naphthyl)propionic acid is heated with 0.50 g of triethylamine (0.5 equivalent) in 15 ml of isopropanol and 0.5 ml of water to about the reflux temperature of the solvent to dissolve the propionic acid. There is an immediate precipitate when 1.32 g of N-cyclohexyl-D-glucamine (0.5 equivalent) is added to the heated solution. An additional 15 ml of isopropanol and 1.5 ml of water are added and the mixture is heated to about the reflux temperature of the solvent to dissolve the precipitate. The solution is cooled to room temperature to give 2 g of a material enriched in the salt of the naproxen precursor acid with N-cyclohexyl-D-glucamine. A sample of the latter is dissolved in about 25 ml of water

heated to about 80°C, treated with hydrochloric acid until acidic at which time a material enriched in the naproxen precursor acid precipitates out of solution and is recovered by filtration.

1.00 g of the material enriched in the salt of the naproxen precursor acid with N-cyclohexyl-D-glucamine is dissolved in 20 ml of 5% aqueous isopropanol at about the reflux temperature of the solvent. The solution is cooled to room temperature to give 0.79 g of a first recrystallized material. A sample of the latter is treated with hydrochloric acid as set forth in the preceding paragraph to give a material further enriched in the 5-chloro-naproxen precursor.

A solution of one g of the 5-chloro-naproxen precursor in 10 ml of anhydrous methanol is mixed with one g of Raney nickel and heated at reflux temperature for 12 hours. The mixture is filtered, and the filtrate is diluted with water. The precipitate is dried and recrystallized from acetone/hexane to yield naproxen.

Repeating the above procedure with the corresponding 5-bromo compound yields naproxen, optical rotation $[\alpha]_D$ 60° in chloroform.

Repeating the above procedure with N-methyl-D-glucamine and N-n-octyl-D-glucamine as a resolving agent yields naproxen with a specific optical rotation between 60 and 65° (chloroform).

EXAMPLE 8

6.2 g of d,l 2-(5-bromo-6-methoxy-2-naphthyl)propionic acid is heated with 1.01 g of triethylamine (0.5 equivalent) in 30 ml of isopropanol and 1.5 ml of water to about the reflux temperature of the solvent to dissolve the d,l acid. 2.93 g of N-n-octyl-D-glucamine (0.5 equivalent) are added and the solution is cooled to room temperature to give 4.5 g of a material enriched in the naproxen precursor salt with N-n-octyl-D-glucamine. A sample of the latter is dissolved in about 25 ml of water heated to about 80°C, treated with hydrochloric acid until acidic at which time a material enriched in the naproxen precursor propionic acid precipitates out of solution and is recovered by filtration.

1.00 g of the material enriched in the salt of the naproxen precursor propionic acid with N-n-octyl-D-glucamine is dissolved in 9.5 ml of isopropanol and 0.5 ml of water at about the reflux temperature of the solvent. The solution is cooled to room temperature to give 0.85 g of a recrystallized salt. This material is treated with hydrochloric acid as set forth in the preceding paragraph to give substantially pure product.

In a 200 ml flask equipped with a reflux condenser and a nitrogen inlet 60 g of magnesium powder, 50 ml of anhydrous methanol and 10 g of triethylamine are added. The flask is being kept under nitrogen during the entire reaction. 0.1 mole of the 5-bromo-naproxen precursor are added dropwise in form of a solution in 15 g methanol. The mixture is heated for another hour after the completion of the addition of the 5-bromo-naproxen precursor under reflux. The mixture is cooled to room temperature and 6N hydrochloric acid until no magnesium remained. Then the mixture is diluted with water, methylene chloride added and the mixture treated with the methylene chloride. The organic layer is separated washed with water and naproxen crystallized by concentration of the solution and addition of hexane. After recrystallization naproxen is obtained having a melting point of 155° and an $[\alpha]_D$ of 65°.

The above reaction sequence is repeated with d,l-2-(5-chloro-6-methoxy-2-naphthyl)propionic acid whereby naproxen of a melting point of 154°C and an $[\alpha]_D$ of 62° in chloroform is obtained.

EXAMPLE 9

2.16 g of d,l 2-(6-hydroxy-2-naphthyl)propionic acid is heated with 0.50 g of triethylamine (0.5 equivalent) in 30 ml. of ethylene glycol to 90°C to dissolve the d,l 2-(6-hydroxy-2-naphthyl)propionic acid. 1.47 g of N-n-octyl-D-glucamine are added and the solution is cooled to 40°C and seeded with crystals of the salt of d 2-(6-hydroxy-2-naphthyl)propionic acid with N-n-octyl-D-glucamine. The reaction mixture is stirred overnight at room temperature to give 1.47 g of a material enriched in the salt of d 2-(6-hydroxy-2-naphthyl)propionic acid with N-n-octyl-D-glucamine. A sample of the latter is dissolved in about 25 ml of water heated to about 80°C, treated with hydrochloric acid until acidic at which time a material enriched in d 2-(6-hydroxy-2-naphthyl)propionic acid precipitates out of solution and is recovered by filtration.

2 g of the 6-hydroxy-naproxen precursor are dissolved in 30 ml of a 1N aqueous solution of sodium hydroxide and 3.8 g of dimethylsulfate are added to the solution. The solution is heated to 60°C for 2 hours and cooled to room temperature and then the reaction mixture acidified by the addition of dilute hydrochloric acid. The obtained precipitate is filtered and washed with water until it is neutral to obtain 1.5 g of naproxen which after crystallization had an optical rotation of $[\alpha]_D$ of 62°.

d,l-2-(6-hydroxy-5-bromo-2-naphthyl)propionic acid is resolved and methylated according to the procedure of the preceding paragraphs and then dehalogenated according to the procedure of Example 4 to yield naproxen of $[\alpha]_D$ being 60°.

EXAMPLE 10

3.1 g of d,l 2-(5-bromo-6-methoxy-2-naphthyl)propionic acid is heated with 0.50 g. of triethylamine (0.5 equivalent) in 15 ml of isopropanol and 0.75 ml of water to about the reflux temperature of the solvent to dissolve the d,l 2-(5-bromo-6-methoxy-2-naphthyl)propionic acid. 1.75 g of N-n-dodecyl-D-glucamine (0.5 equivalent) are added and the solution is cooled to room temperature to give 2.50 g. of a material enriched in the naproxen precursor salt of N-n-dodecyl-D-glucamine. A sample of the latter is suspended in about 25 ml of water at room temperature in the presence of 300 mg of potassium hydroxide and held at that temperature for 60 minutes. The precipitate is removed by filtration. The filtrate is treated with hydrochloric acid until acidic at which time a material enriched in the 5-bromo-naproxen precursor propionic acid precipitates out of solution and is recovered by filtration.

1.00 g of the material enriched in the salt of d 2-(5-bromo-6-methoxy-2-naphthyl)propionic acid with N-n-dodecyl-D-glucamine is dissolved in 20 ml of 5% aqueous isopropanol at about the reflux temperature of the solvent. The solution is cooled to room temperature to give 0.88 g of a first recrystallized material. A sample of the latter is treated with potassium hydroxide then hydrochloric acid as set forth in the preceding paragraph to give a further enriched material.

0.62 g of the first recrystallized material from the preceding paragraph [i.e. the material also enriched in the salt of d 2-(5-bromo-6-methoxy-2-naphthyl)propionic acid with N-n-dodecyl-D-glucamine] is dissolved in 15 ml of 5% aqueous isopropanol at about the reflux temperature of the solvent. The solution is cooled to room temperature to give 0.5 g of a second recrystallized material.

0.1 mole of the 5-bromo-naproxen precursor in methanol (15 g) is slowly added, and the mixture is heated on reflux for one additional hour after the addition of the 5-bromo-naproxen precursor, to a 250 ml flask equipped with a reflux condenser with 60 g magnesium, 50 ml anhydrous methanol and 10 g triethylamine. During the entire reaction the flask is swept with nitrogen. After completion of the addition of the 5-bromo-naproxen precursor the cooled mixture is mixed with 6N hydrochloric acid until no magnesium remains. The mixture is poured into water and then extracted with methylene chloride. The organic layer is separated washed with water and crystallized by concentrating the solution and added hexane. Naproxen of $[\alpha]_D$ of 62° is obtained after recrystallization.

Repeating this procedure with the corresponding 5-chloro-naproxen precursor naproxen is obtained with an optical rotation of 60°.

EXAMPLE 11

135 mg of the sodium salt of d,l-2-(5-bromo-6-methoxy-2-naphthyl)propionic acid is heated with 100 mg of the hydrochloride salt of N-n-butyl-D-glucamine in 2 ml of 5% aqueous isopropanol to about the reflux temperature of the solvent. The reaction mixture is slowly cooled, seeded with a small amount of the salt of the 5-bromo-naproxen precursor with N-n-butyl-D-glucamine, and then further cooled to room temperature to give, after recovery by filtration and washing, 1.10 g of a material enriched in the salt of the 5-bromo-naproxen precursor with N-n-butyl-D-glucamine. A sample of the latter is dissolved in water, and treated with hydrochloric acid until acidic at which time a material enriched in d 2-(6-methoxy-2-naphthyl)propionic acid precipitates out of solution and is recovered by filtration.

A solution of one g of the 5-bromo precursor of naproxen and 10 ml of anhydrous methanol is mixed with one g of Raney nickel and heated at reflux temperature for 14 hours while hydrogen is introduced. The mixture is filtered and the filtrate diluted with water. The precipitate is dried and recrystallized from acetone hexane to yield naproxen of an optical rotation of 65° after recrystallization.

EXAMPLE 12

2.1 g of d,l 2-(6-hydroxy-2-naphthyl)propionic acid is heated with 1.46 g of N-n-hexyl-D-glucamine (0.55 equivalent) in 15 ml of water to about 80°C. 0.17 g of potassium hydroxide (0.45 equivalent) are added and the pH of the solution adjusted to 8 with potassium carbonate. The solution is seeded with a small amount of the salt of d 2-(6-hydroxy-2-naphthyl)propionic acid with N-n-hexyl-D-glucamine and cooled to give 1.25 g of a material enriched in the salt of d 2-(6-hydroxy-2-naphthyl)propionic acid with N-n-hexyl-D-glucamine.

The N-n-hexyl-D-glucamine salt of d 2-(6-hydroxy-2-naphthyl)propionic acid is dissolved in 15 ml of dioxan and 15 ml of aqueous sodium hydroxide (20%) added, the mixture is heated and saturated with methyl bromide. Then cooled to room temperature whereby continuously methyl bromide is introduced. The reaction mixture is acidified by the addition of aqueous 1N hydrochloric acid after cooling and extracted with diethyl ether. The extracts are collected and washed with water, dried over sodium sulfate, filtered and

evaporated and gave naproxen of an optical rotation of 58°.

EXAMPLE 13

4.60 g of d,l 2-(6-hydroxy-2-naphthyl)propionic acid is slurried with 25 ml of water and 0.60 g of potassium hydroxide (0.45 equivalent) at 70°C for 10 minutes. 3.22 g of N-n-octyl-D-glucamine (0.55 equivalent) are added and the solution is cooled slowly to 50°C. A small amount of the salt of d 2-(6-hydroxy-2-naphthyl)propionic acid with N-n-octyl-D-glucamine is added and the solution cooled overnight, with stirring, to room temperature to give, after recovery by filtration, 4.0 g of a material enriched in the salt of d 2-(6-hydroxy-2-naphthyl)propionic acid with N-n-octyl-D-glucamine. A sample of the latter is suspended in about 25 ml of water at room temperature in the presence of 300 mg of potassium hydroxide and held at that temperature for about 60 minutes. The resultant precipitate is removed by filtration. The filtrate is treated with hydrochloric acid until acidic at which time a material enriched in d 2-(6-hydroxy-2-naphthyl)-propionic acid precipitates out of solution and is recovered by filtration.

2 g of the 6-hydroxy-naproxen precursor is dissolved in 35 ml of 1N aqueous solution of sodium hydroxide and 4 g of dimethyl sulfate is added to the solution. Then the solution is heated to 65°C for 2 1/2 hours then cooled to room temperature. The reaction mixture is then acidified by the addition of dilute hydrochloric acid. The precipitate formed after the additional hydrochloric acid is filtered and washed with water until it became neutral to obtain 1.6 g of naproxen of an optical purity of 64° in chloroform.

EXAMPLE 14

433 g of racemic 2-(6-hydroxy-2-naphthyl)propionic acid (2 mol) and 390.5 g of N-methyl-D-glucamine (2 mol) are dissolved in 4 liters of boiling methanol.

The solution is filtered to clearness and carefully cooled to 45°C with slow stirring. 1 g of the 6-hydroxy-naproxen precursor N-methyl-D-glucamine salt crystals (obtained in a preliminary test by cooling and rubbing with a glass rod, filtering under suction and washing with some methanol) is now added. Massive crystallization occurred immediately after seeding. The temperature is held at 45°C and then lowered slowly to 15°C.

The precipitated crystals are filtered off and washed with a little methanol.

Yield: 300 g of (d)-6-hydroxy-naproxen precursor N-methyl-D-glucamine salt.

The residue from precipitation is dissolved in water and dilute hydrochloric acid is added to acidify the salt solution. The (d)-6-hydroxy-naproxen precursor is precipitated.

200 g of the (d)-2-(6-hydroxy-2-naphthyl)propionic acid are dissolved in 350 ml of an 1N aqueous solution of sodium hydroxide, and 40 g of dimethyl sulfate added to the solution. Then the solution is heated at 60°C for 2 hours and cooled to room temperature and reaction mixture is acidified by the addition of dilute hydrochloric acid. The formed precipitate is filtered off, washed with water until it became neutral to obtain 175 g of naproxen, optical rotation 62°, after recrystallization.

EXAMPLE 15

6.2 g of d,l 2-(5-bromo-6-methoxy-2-naphthyl)propionic acid is heated with 1.01 g of triethylamine (0.5 equivalent) in 20 ml of 6% toluene in methanol to the reflux temperature of the solvent to dissolve the d,l acid. 1.95 g of N-methyl-D-glucamine (0.5 equivalent) are added and the solution is cooled to room temperature (i.e. about 20-23°C) to give 3.52 g of a material enriched in the salt of the 5-bromo-naproxen precursor with N-methyl-D-glucamine. The salt is dissolved in about 25 ml of water, treated with hydrochloric acid until acidic at which time a material enriched in the 5-bromo-naproxen precursor precipitates out of solution and is recovered by filtration.

1.00 g of the material enriched in the salt of the 5-bromo-naproxen precursor with N-methyl-D-glucamine is recrystallized from 10 ml of methanol and 20 ml of ethanol, concentrated at reflux to remove 5 ml of solvent, and cooled to give 0.85 g of a recrystallized salt. This material is treated with hydrochloric acid as set forth in the preceding paragraph to give substantially pure 5-bromo-naproxen precursor.

A solution of one gram of the 5-bromo naproxen precursor in 10 ml of anhydrous methanol is mixed with 100 mg of palladium-on-charcoal and stirred at room temperature under hydrogen atmosphere for 12 hours. The mixture is filtered, diluted with water and extracted with methylene chloride. The organic phase is evaporated by dryness and residue is recrystallized from methyl chloride/hexane to yield naproxen of an optical rotation of 58°.

In another variant, to a 250 ml flask equipped with a reflux condenser having an nitrogen bubbler is

added magnesium powder (60 g), anhydrous methanol (50 ml) and triethylamine (10 g). The flask is saturated and swept with nitrogen and the nitrogen atmosphere is maintained through the reaction.

The 5-bromo naproxen precursor prepared in the first part of Example 11, 0.1 mole, is dissolved in 15 g methanol and slowly added to the above reaction mixture which is heated under reflux for one additional hour after the addition of the 5-bromo naproxen precursor. The cooled mixture is mixed with 6N hydrochloric acid until no magnesium remains. The mixture is poored then into water and extracted with methylene chloride. The organic layer is separated, washed with water and crystallized by concentrating the solution and adding hexane. Naproxen isolated after recrystallization with an optical rotation of 64° in chloroform.

Repeating these procedures with the corresponding 5-chloro naproxen precursor, naproxen is obtained after recrystallization, $[\alpha]_D$ of 62°.

EXAMPLE 16

A mixture prepared by adding 23 g of d-2-(6-methoxy-2-naphthyl)propionic acid as prepared in Example 1 to 4 g of sodium hydroxide in 500 ml of aqueous methanol is stirred for 3 hours at room temperature. Then the mixture is evaporated to yield sodium 2-(6-methoxy-2-naphthyl)propionate. The product is replaced into toluene then isolated by centrifugation and washed with hexane prior to drying. The product melts at about 255°C with decomposition and its infrared spectrum exhibits maxima at 1260, 1600, 1625 and 1725 $cm^{-1}$. The yield is 95% based on d-2-(6-methoxy-2-naphthyl)propionic acid.

## Claims

**Claims for the following Contracting States : BE, DE, SE, NL, GB, FR, IT, LU**

**1.** An N-R-D-glucamine salt of a d or l compound of formula I

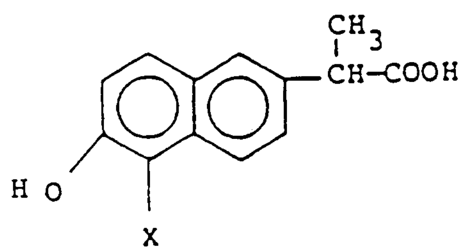

(I)

wherein R is methyl and X is hydrogen, chloro or bromo.

**2.** An N-R-D-glucamine salt of a d or l compound of formula I

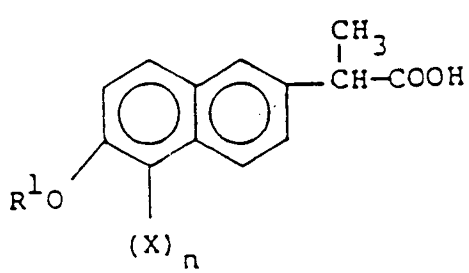

(I)

wherein R is alkyl from 2 to 18 carbon atoms or cycloalkyl from 3 to 8 carbon atoms and $R^1$ is hydrogen or methyl when X is halogen selected from chloro and bromo and n is 1; or $R^1$ is hydrogen when n is 0.

**3.** An N-R-D-glucamine salt of a d or l compound of formula I

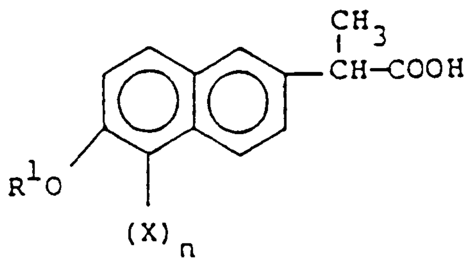

(I)

wherein R is alkyl from 6 to 18 carbon atoms or cycloalkyl from 3 to 8 carbon atoms, and $R^1$ is hydrogen or methyl when X is halogen selected from chloro and bromo and n is 1; or $R^1$ is hydrogen when n is 0.

**4.** The N-R-D-glucamine salt of claim 1 wherein R is methyl; and X is bromo.

**5.** The N-R-D-glucamine salt of claim 2 or claim 3 wherein R is n-octyl.

**6.** A mixture of the N-R-D-glucamine salts of the (d,l) acids of the formula I

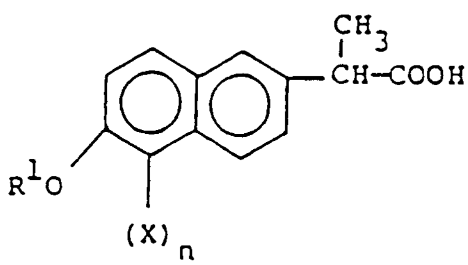

(I)

wherein $R^1$ is hydrogen or methyl when X is halogen selected from bromo and chloro and n is 1; or $R^1$ is hydrogen when n is 0, and R is methyl; with the proviso that when $R^1$ is methyl and X is halogen, then the mixture is other than in a solvent system comprising a mixture of toluene and methanol.

**7.** A mixture of the N-R-D-glucamine salts of the (d,l) acids of the formula I

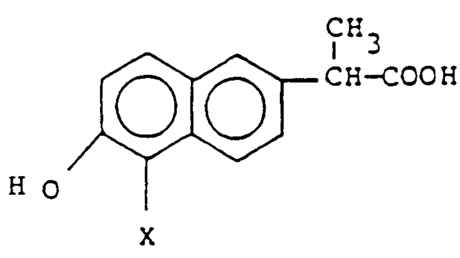

(I)

wherein X is hydrogen, bromo or chloro and R is methyl.

**8.** A mixture of the N-R-D-glucamine salts of the (d,l) acids of the formula I

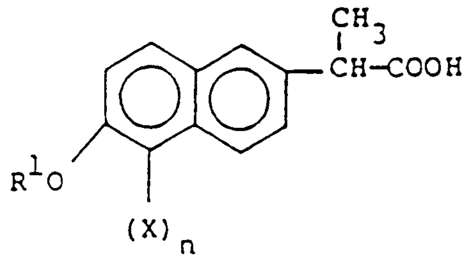

(I)

wherein $R^1$ is hydrogen or methyl when X is halogen selected from bromo and chloro and n is 1; or $R^1$ is hydrogen when n is 0, and R is alkyl with 2 to 18 carbon atoms or cycloalkyl with 3 to 8 carbon atoms.

**9.** A mixture of the N-R-D-glucamine salts of the (d,l) acids of the formula I

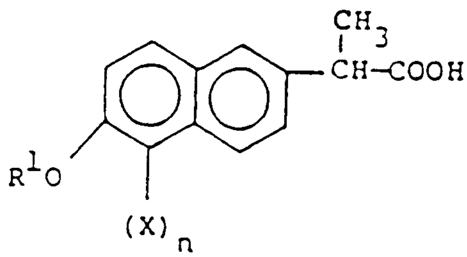

(I)

wherein $R^1$ is hydrogen or methyl when X is halogen selected from bromo and chloro and n is 1; or $R^1$ is hydrogen when n is 0, and R is alkyl with 6 to 18 carbon atoms or cycloalkyl with 3 to 8 carbon atoms.

**10.** A resolving medium comprising a mixture of the N-R-D-glucamine salts of the (d,l) acids of the formula I

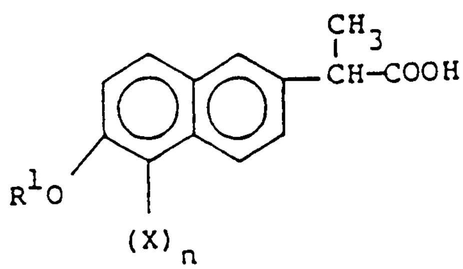

(I)

wherein $R^1$ is hydrogen or methyl when X is halogen selected from bromo and chloro and n is 1, or $R^1$ is hydrogen when n is 0, and R is methyl, and a solvent in which the solubility of the corresponding d-2-(6-methoxy-2-naphthyl)propionic acid precursor salt is considerably less than the solubility of the other diastereomeric salt at the resolution temperature; with the proviso that when $R^1$ is methyl and X is halogen, then the mixture is other than in a solvent system comprising a mixture of toluene and methanol.

**11.** A resolving medium comprising a mixture of the N-R-D-glucamine salts of the (d,l) acids of the formula I

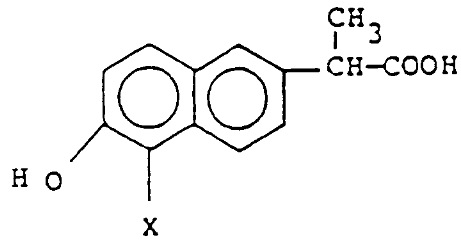

(I)

wherein X is hydrogen, bromo or chloro, and R is methyl, and a solvent in which the solubility of the corresponding d-2-(6-methoxy-2-naphthyl)propionic acid precursor salt is considerably less than the solubility of the other diastereomeric salt at the resolution temperature.

**12.** A resolving medium comprising a mixture of the N-R-D-glucamine salts of the (d,l) acids of the formula I

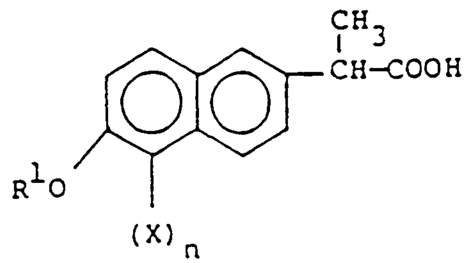

(I)

wherein $R^1$ is hydrogen or methyl when X is halogen selected from bromo and chloro and n is 1, or $R^1$ is hydrogen when n is 0, and R is alkyl having 2 to 18 carbon atoms or a cycloalkyl having 3 to 8 carbon atoms, and a solvent in which the solubility of the corresponding d-2-(6-methoxy-2-naphthyl)-propionic acid precursor salt is considerably less than the solubility of the other diastereomeric salt at the resolution temperature.

**13.** A resolving medium comprising a mixture of the N-R-D-glucamine salts of the (d,l) acids of the formula I

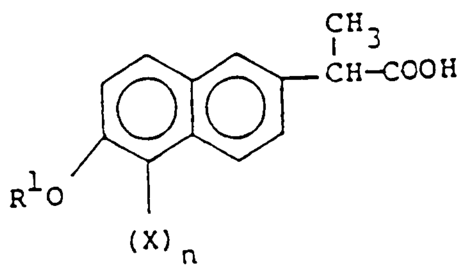

(I)

wherein $R^1$ is hydrogen or methyl when X is halogen selected from bromo and chloro and n is 1, or $R^1$ is hydrogen when n is 0, and R is alkyl having 6 to 18 carbon atoms or a cycloalkyl having 3 to 8 carbon atoms, and a solvent in which the solubility of the corresponding d-2-(6-methoxy-2-naphthyl)-propionic acid precursor salt is considerably less than the solubility of the other diastereomeric salt at the resolution temperature.

**14.** The d-isomer of a compound of formula I

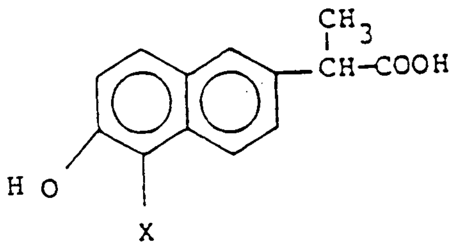

(I)

wherein X is hydrogen, chloro or bromo.

**15.** The l-isomer of a compound of formula I

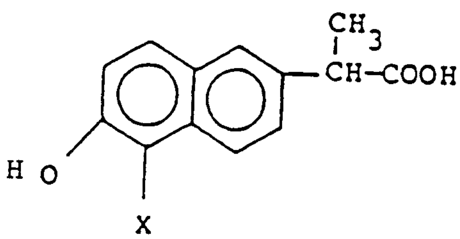

(I)

wherein X is hydrogen, chloro or bromo.

**16.** A process for the production of (d)-2-(6-methoxy-2-naphthyl)propionic acid, which process comprises conversion of an N-R-D-glucamine salt of any one of claims 1 to 5, wherein said N-R-D-glucamine salt is that of the d-isomer of a compound of formula I, to (d)-2-(6-methoxy-2-naphthyl)propionic acid.

**17.** A process according to claim 16 wherein (d)-2-(6-methoxy-2-naphthyl)propionic acid is converted to a pharmaceutically acceptable salt thereof.

**18.** Process for resolving mixtures of (d)- and (l)-2-(6-methoxy-2-naphthyl)propionic acid precursors of formula I

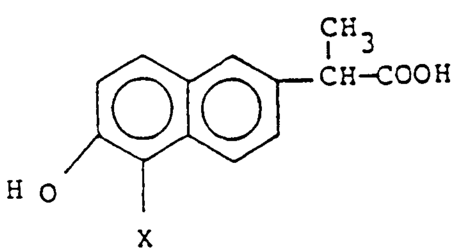

(I)

wherein X is hydrogen, chloro or bromo; or salts thereof;

into the enantiomers thereof, characterized in that N-R-D-glucamines or salts thereof are used as the resolving agent, wherein R is methyl.

**19.** Process for resolving mixtures of (d) and (l)-2-(6-methoxy-2-naphthyl)propionic acid precursors of formula I

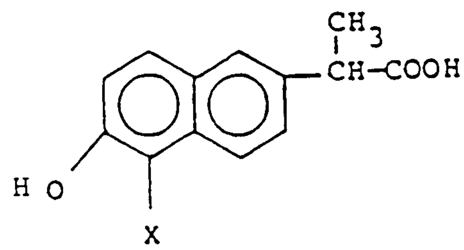

(I)

wherein X is hydrogen, chloro or bromo; or salts thereof;
into the enantiomers thereof characterized in that N-R-D-glucamines or salts thereof are used as the resolving agent, wherein R is octyl.

**20.** Process for resolving mixtures of (d)- and (l)-2-(6-methoxy-2-naphthyl)propionic acid precursors of formula I

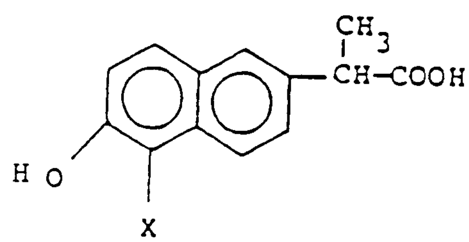

(I)

wherein X is hydrogen, chloro or bromo; or salts thereof;
into the enantiomers thereof characterized in that N-R-D-glucamines or salts thereof are used as the resolving agent, wherein R is alkyl of 2 to 18 carbon atoms or cycloalkyl of 3 to 8 carbon atoms.

**21.** Process for resolving mixtures of (d)- and (l)-2-(6-methoxy-2-naphthyl)propionic acid precursors of formula I

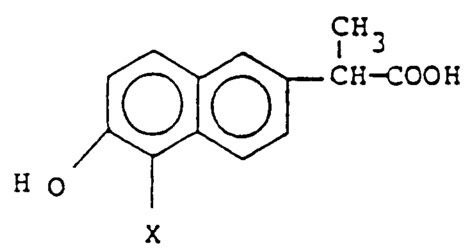

(I)

wherein X is hydrogen, chloro or bromo; or salts thereof;
into the enantiomers thereof characterized in that N-R-D-glucamines or salts thereof are used as the resolving agent, wherein R is alkyl of 6 to 18 carbon atoms or cycloalkyl of 3 to 8 carbon atoms.

**22.** The process of any one of claims 18 to 21 wherein the resulting pair of (d)- and (l)-acid N-R-D-glucamine salts is separated by fractional crystallization.

**23.** Process for preparing (d)-2-(6-methoxy-2-naphthyl)propionic acid or pharmaceutically acceptable salts thereof which process comprises:
(a) resolving a precursor of formula I

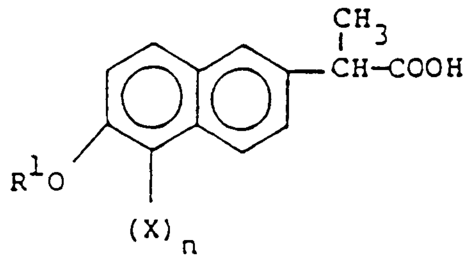

(I)

wherein $R^1$ is hydrogen or methyl when X is halogen selected from bromo and chloro and n is 1, or wherein $R^1$ is hydrogen when n is 0, or a salt of a compound of formula (I), with an N-R-D-glucamine wherein R is methyl or a salt thereof; with the proviso that when the resolving agent is N-methyl-D-glucamine and said precursor is dl-2-(5-halo-6-methoxy-2-naphthyl)propionic acid, and are reacted together in a solvent system from which N-methyl-D-glucamine d-2-(5-halo-6-methoxy-2-naphthyl)-propionate is separated, then said solvent system is other than a mixture of toluene and methanol; and

(b) converting the resultant d-2-(6-methoxy-2-naphthyl)propionic acid precursor to d-2-(6-methoxynaphthyl)propionic acid.

**24.** Process for preparing (d)-2-(6-methoxy-2-naphthyl)propionic acid or pharmaceutically acceptable salts thereof which process comprises:

(a) resolving a compound of formula I

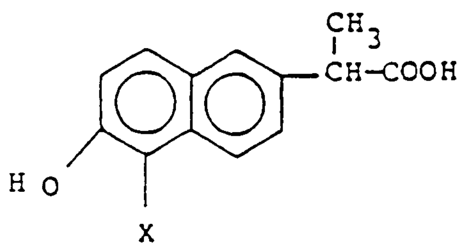

(I)

wherein X is hydrogen, bromo or chloro, or a salt of a compound of formula (I), with an N-R-D-glucamine wherein R is methyl, or a salt thereof; and

(b) converting the resultant d-2-(6-methoxy-2-naphthyl)propionic acid precursor to d-2-(6-methoxy-2-naphthyl)propionic acid.

**25.** Process for preparing (d)-2-(6-methoxy-2-naphthyl)propionic acid or pharmaceutically acceptable salts thereof which process comprises:

(a) resolving a compound of formula I

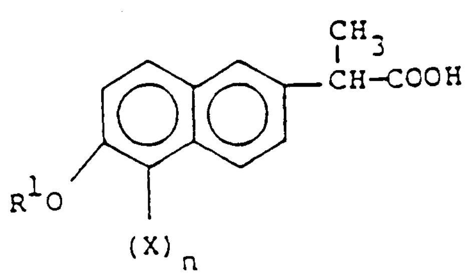

(I)

wherein R is hydrogen or methyl when X is halogen selected from bromo or chloro and n is 1, or wherein $R^1$ is hydrogen when n is 0, or a salt of a compound of formula (I), with an N-R-D-glucamine wherein R is alkyl of 2 to 18 carbon atoms or cycloalkyl of 3 to 8 carbon atoms or a salt thereof; and

(b) converting the resultant d-2-(6-methoxy-2-naphthyl)propionic acid precursor to d-2-(6-methoxy-2-naphthyl)propionic acid.

**26.** Process for preparing (d)-2-(6-methoxy-2-naphthyl)propionic acid or pharmaceutically acceptable salts thereof which process comprises:
(a) resolving a compound of formula I

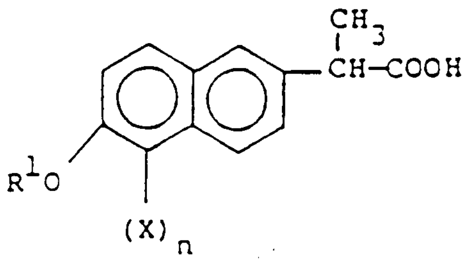

(I)

wherein R is hydrogen or methyl when X is halogen selected from bromo or chloro and n is 1, or wherein $R^1$ is hydrogen when n is 0, or a salt of a compound of formula (I), with an N-R-D-glucamine wherein R is alkyl of 6 to 18 carbon atoms or cycloalkyl of 3 to 8 carbon atoms or a salt thereof; and
(b) converting the resultant d-2-(6-methoxy-2-naphthyl)propionic acid precursor to d-2-(6-methoxy-2-naphthyl)propionic acid.

**27.** The process of any one of claims 23 to 26 wherein d-2-(6-hydroxy-2-naphthyl)propionic acid or a salt thereof is converted to d-2-(6-methoxy-2-naphthyl)propionic acid.

**28.** The process of any one of claims 23 to 26 wherein d-2-(5-halo-6-methoxy-2-naphthyl)propionic acid, d-2-(5-halo-6-hydroxy-2-naphthyl)propionic acid or a salt thereof is converted to d-2-(6-methoxy-2-naphthyl)propionic acid.

**29.** The process of claim 27 or 28 wherein d-2-(6-methoxy-2-naphthyl)propionicacid is converted to a pharmaceutically acceptable salt thereof.

**30.** A process for resolving a (d)- and (l)-2-(6-methoxy-2-naphthyl)propionic acid precursor of formula I

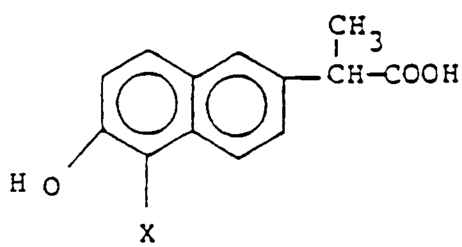

(I)

wherein X is hydrogen, chloro or bromo, or salts thereof,

into the enantiomers thereof characterized in that an N-R-D-glucamine or salt thereof wherein R is alkyl having 1 to 18 carbon atoms or cycloalkyl having 3 to 8 carbon atoms is used as the resolving agent to isolate said (d)-acid precursor and that said (l)-acid precursor is racemized followed by resolution.

**31.** A process for preparing (d)-2-(6-methoxy-2-naphthyl)propionic acid or pharmaceutically acceptable salts thereof which process comprises:
(a) resolving a precursor compound of formula I

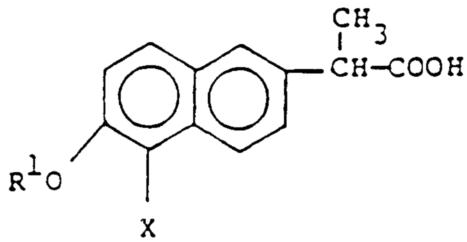

(I)

wherein $R^1$ is hydrogen or methyl when X is bromo or chloro; or $R^1$ is hydrogen when X is hydrogen; or a salt of a compound of formula I, with an N-R-D-glucamine wherein R is alkyl of 1 to 18 carbon atoms or cycloalkyl of 3 to 8 carbon atoms, or a salt of said glucamine, to isolate the (d)-form of said precursor compound;
(b) converting said (d)-form to (d)-2-(6-methoxy-2-naphthyl)propionic acid or a pharmaceutically acceptable salt thereof; and
(c) racemizing the (l)-form of said precursor compound and resolving the resultant racemic form of said precursor compound.

**32.** A process for resolving a (d)- and (l)-2-(6-methoxy-2-naphthyl)propionic acid precursor of formula I

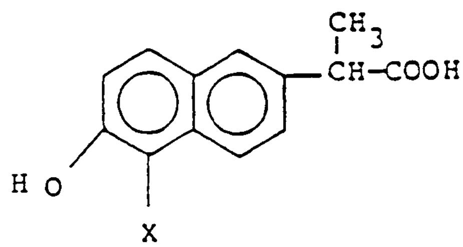

(I)

wherein X is hydrogen, chloro or bromo, or salts thereof,
into the enantiomers thereof characterized in that a N-R-D-glucamine or salt thereof wherein R is alkyl having 2 to 8 carbon atoms or cycloalkyl having 3 to 8 carbon atoms is used as the resolving agent to isolate said (d)-acid precursor and that said (l)-acid precursor is racemized followed by resolution.

**33.** A process for preparing (d)-2-(6-methoxy-2-naphthyl)propionic acid or pharmaceutically acceptable salts thereof which process comprises:
(a) resolving a precursor compound of formula I

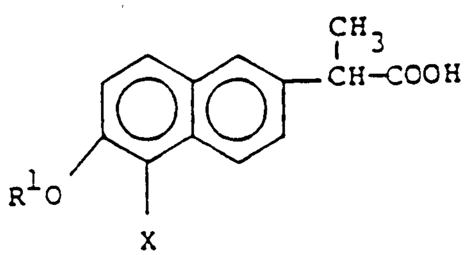

(I)

wherein $R^1$ is hydrogen or methyl when X is bromo or chloro; or
$R^1$ is hydrogen when X is hydrogen,
or a salt of a compound of formula I,
with an N-R-D-glucamine wherein R is alkyl of 2 to 8 carbon atoms or cycloalkyl of 3 to 8 carbon atoms,
or a salt of said glucamine,
to isolate the (d)-form of said precursor compound;
(b) converting said (d)-form to (d)-2-(6-methoxy-2-naphthyl)propionic acid or a pharmaceutically

acceptable salt thereof; and

(c) racemizing the (l)-form of said precursor compound and resolving the resultant racemic form of said precursor compound.

**Claims for the following Contracting State : AT**

**1.** A process for the separation of the d- and l-isomers of formula I

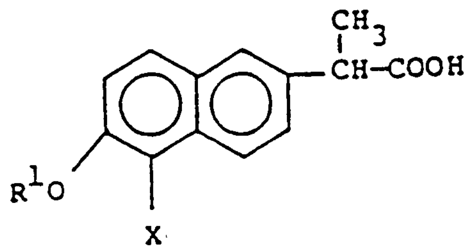

(I)

wherein $R^1$ is methyl and X is bromo; which process is characterised by resolving diastereomeric salts of N-R-D-glucamine, where R is methyl, and a mixture of d- and l-isomers of formula I.

**2.** A process which comprises resolving the diastereomeric salts of N-R-D-glucamine and a mixture of d- and l-isomers of formula I

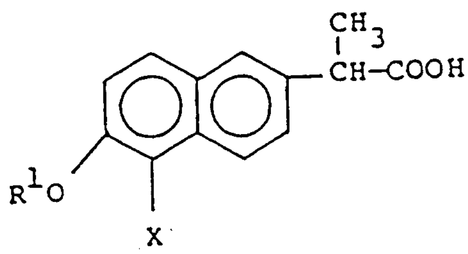

(I)

wherein R is methyl, $R^1$ is methyl and X is chloro.

**3.** A process which comprises resolving diastereomeric salts of N-R-D-glucamine and a mixture of d- an l-isomers of formula I

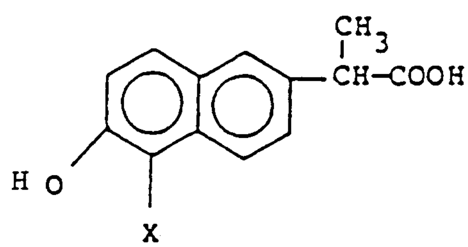

(I)

wherein R is methyl and X is hydrogen, chloro or bromo.

**4.** A process which comprises resolving diastereomeric salts of N-R-D-glucamine and a mixture of d- and l-isomers of formula I

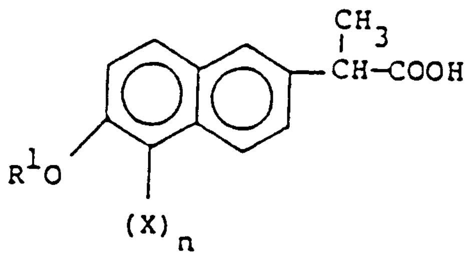

(I)

wherein R is alkyl from 2 to 18 carbon atoms or cycloalkyl from 3 to 8 carbon atoms and $R^1$ is hydrogen or methyl when X is halogen selected from chloro and bromo and n is 1; or $R^1$ is hydrogen when n is 0.

5. A process which comprises resolving diastereomeric salts of N-R-D-glucamine and a mixture of d- and l-isomers of formula I

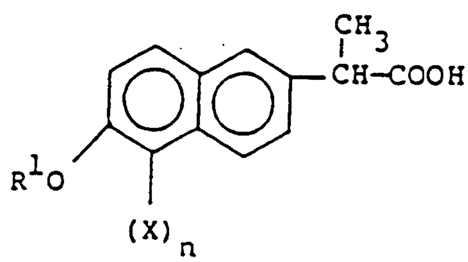

(I)

wherein R is alkyl from 6 to 18 carbon atoms or cycloalkyl from 3 to 8 carbon atoms, and $R^1$ is hydrogen or methyl when X is halogen selected from chloro and bromo and n is 1; or $R^1$ is hydrogen when n is 0.

6. The process of claim 3 wherein X is bromo.

7. The process of claim 5 wherein R is n-octyl.

8. A process for the separation of the d- and l-isomers of formula I

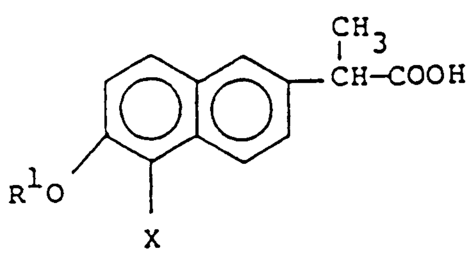

(I)

wherein $R^1$ is methyl and X is bromo; which process is characterised by the reaction of N-R-D-glucamine, where R is methyl, with a mixture of d- and l-isomers of formula I.

9. A process which comprises the reaction of N-R-D-glucamine with a mixture of d- and l-isomers of formula I

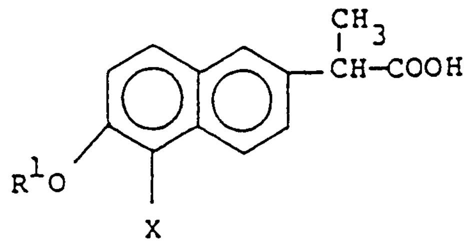

(I)

wherein $R^1$ is methyl, X is chloro and R is methyl.

**10.** A process which comprises the reaction of N-R-D-glucamine with a mixture of d- an l-isomers of formula I

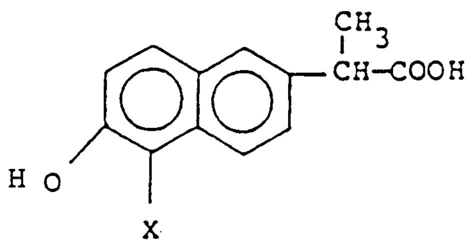

(I)

wherein X is hydrogen, bromo or chloro and R is methyl.

**11.** A process which comprises the reaction of N-R-D-glucamine with a mixture of d- and l-isomers of formula I

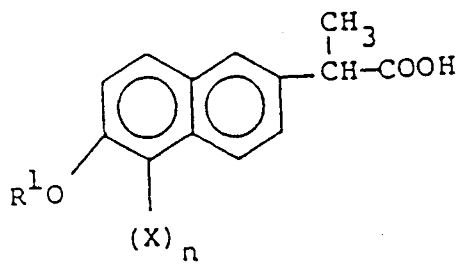

(I)

wherein $R^1$ is hydrogen or methyl when X is halogen selected from bromo and chloro and n is 1; or $R^1$ is hydrogen when n is 0, and R is alkyl with 2 to 18 carbon atoms or cycloalkyl with 3 to 8 carbon atoms.

**12.** A process which comprises the reaction of N-R-D-glucamine with a mixture of d- and l-isomers of formula I

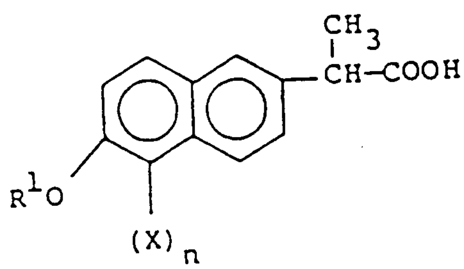

(I)

wherein $R^1$ is hydrogen or methyl when X is halogen selected from bromo and chloro and n is 1; or

$R^1$ is hydrogen when n is 0, and R is alkyl with 6 to 18 carbon atoms or cycloalkyl with 3 to 8 carbon atoms.

**13.** A process for the separation of the d- and l-isomers of formula I

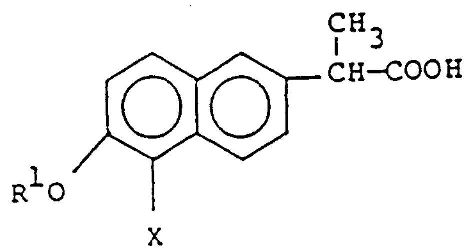

(I)

wherein $R^1$ is methyl when X is bromo, which process is characterised by the preparation of a resolving medium comprising a mixture of diastereomeric salts of N-R-D-glucamine, where R is methyl, and the d- and l-isomers of formula I, and a solvent in which the solubility of the corresponding d-2-(6-methoxy-2-naphthyl)propionic acid precursor salt is considerably less than the solubility of the other diastereomeric salt at the resolution temperature.

**14.** A process which comprises the preparation of a resolving medium comprising a mixture of the N-R-D-glucamine salts of the (d,l) acids of the formula I

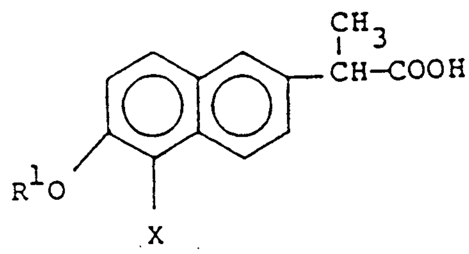

(I)

wherein $R^1$ is methyl, X is chloro and R is methyl, and a solvent in which the solubility of the corresponding d-2-(6-methoxy-2-naphthyl)propionic acid precursor salt is considerably less than the solubility of the other diastereomeric salt at the resolution temperature.

**15.** A process which comprises the preparation of a resolving medium comprising a mixture of the N-R-D-glucamine salts of the (d,l) acids of the formula I

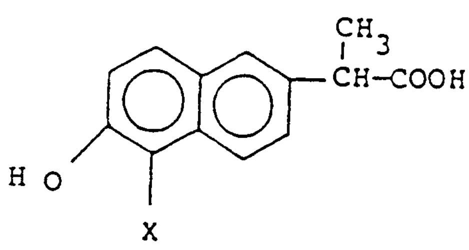

(I)

wherein X is hydrogen, bromo or chloro, and R is methyl, and a solvent in which the solubility of the corresponding d-2-(6-methoxy-2-naphthyl)propionic acid precursor salt is considerably less than the solubility of the other diastereomeric salt at the resolution temperature.

**16.** A process which comprises the preparation of a resolving medium comprising a mixture of the N-R-D-glucamine salts of the (d,l) acids of the formula I

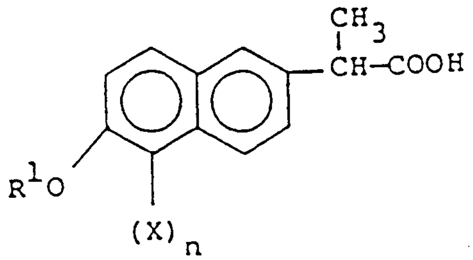

(I)

wherein $R^1$ is hydrogen or methyl when X is halogen selected from bromo and chloro and n is 1, or $R^1$ is hydrogen when n is 0, and R is alkyl having 2 to 18 carbon atoms or a cycloalkyl having 3 to 8 carbon atoms, and a solvent in which the solubility of the corresponding d-2-(6-methoxy-2-naphthyl)-propionic acid precursor salt is considerably less than the solubility of the other diastereomeric salt at the resolution temperature.

**17.** A process which comprises the preparation of a resolving medium comprising a mixture of the N-R-D-glucamine salts of the (d,l) acids of the formula I

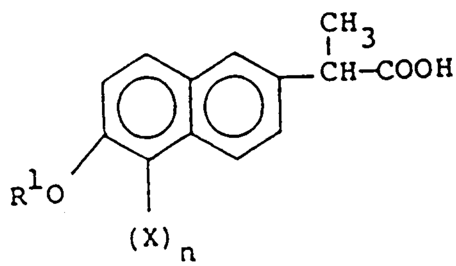

(I)

wherein $R^1$ is hydrogen or methyl when X is halogen selected from bromo and chloro and n is 1, or $R^1$ is hydrogen when n is 0, and R is alkyl having 6 to 18 carbon atoms or a cycloalkyl having 3 to 8 carbon atoms, and a solvent in which the solubility of the corresponding d-2-(6-methoxy-2-naphthyl)-propionic acid precursor salt is considerably less than the solubility of the other diastereomeric salt at the resolution temperature.

**18.** A process which comprises the use of N-R-D-glucamine, where R is alkyl of 2 to 18 carbon atoms or cycloalkyl of 3 to 8 carbon atoms, as a resolving agent in the preparation of the d-isomer of a compound of formula I

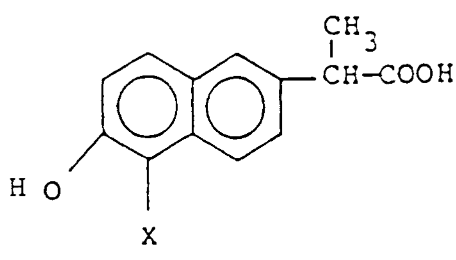

(I)

wherein X is hydrogen, chloro or bromo.

**19.** A process which comprises the use of N-R-D-glucamine, where R is alkyl of 2 to 18 carbon atoms or cycloalkyl of 3 to 8 carbon atoms, as a resolving agent in the preparation of the l-isomer of a compound of formula I

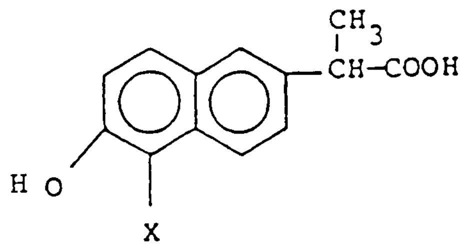

(I)

wherein X is hydrogen, chloro or bromo.

**20.** A process for the production of (d)-2-(6-methoxy-2-naphthyl)propionic acid, which process comprises conversion of an N-R-D-glucamine salt of any one of claims 1 to 7, wherein said N-R-D-glucamine salt is that of the d-isomer of a compound of formula I, to (d)-2-(6-methoxy-2-naphthyl)propionic acid.

**21.** A process according to claim 20 wherein (d)-2-(6-methoxy-2-naphthyl)propionic acid is converted to a pharmaceutically acceptable salt thereof.

**22.** Process for resolving mixtures of (d)- and (l)-2-(6-methoxy-2-naphthyl)propionic acid precursors of formula I

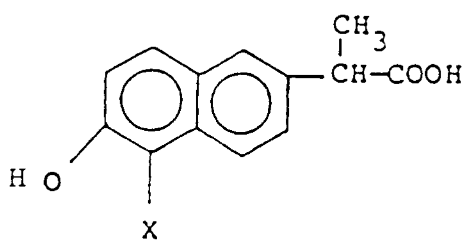

(I)

wherein X is hydrogen, chloro or bromo; or salts thereof;
into the enantiomers thereof characterized in that N-R-D-glucamines or salts thereof are used as the resolving agent, wherein R is methyl.

**23.** Process for resolving mixtures of (d) and (l)-2-(6-methoxy-2-naphthyl)propionic acid precursors of formula I

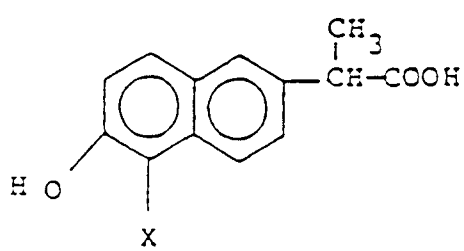

(I)

wherein X is hydrogen, chloro or bromo; or salts thereof;
into the enantiomers thereof characterized in that N-R-D-glucamines or salts thereof are used as the resolving agent, wherein R is octyl.

**24.** Process for resolving mixtures of (d)- and (l)-2-(6-methoxy-2-naphthyl)propionic acid precursors of formula I

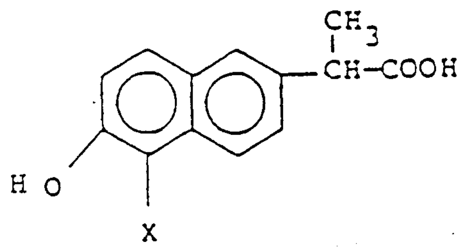

(I)

wherein X is hydrogen, chloro or bromo; or salts thereof;
into the enantiomers thereof characterized in that N-R-D-glucamines or salts thereof are used as the resolving agent, wherein R is alkyl of 2 to 18 carbon atoms or cycloalkyl of 3 to 8 carbon atoms.

**25.** Process for resolving mixtures of (d)- and (l)-2-(6-methoxy-2-naphthyl)propionic acid precursors of formula I

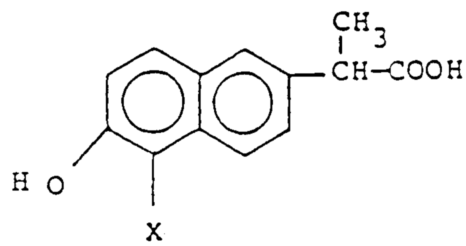

(I)

wherein X is hydrogen, chloro or bromo; or salts thereof;
into the enantiomers thereof characterized in that N-R-D-glucamines or salts thereof are used as the resolving agent, wherein R is alkyl of 6 to 18 carbon atoms or cycloalkyl of 3 to 8 carbon atoms.

**26.** The process of any one of claims 22 to 25 wherein the resulting pair of (d)- and (l)-acid N-R-D-glucamine salts is separated by fractional crystallization.

**27.** Process for preparing (d)-2-(6-methoxy-2-naphthyl)propionic acid or pharmaceutically acceptable salts thereof which process comprises:
(a) resolving a compound of formula I

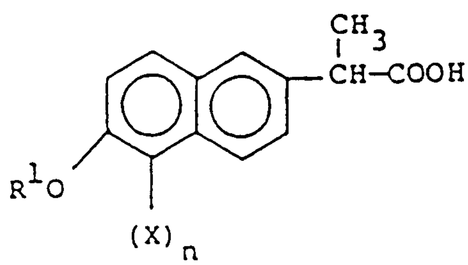

(I)

wherein $R^1$ is hydrogen or methyl when X is halogen selected from bromo and chloro and n is 1, or wherein $R^1$ is hydrogen when n is 0, or a salt of a compound of formula (I), with an N-R-D-glucamine wherein R is methyl or a salt thereof; and
(b) converting the resultant d-2-(6-methoxy-2- naphthyl)propionic acid precursor to d-2-(6-methoxynaphthyl)propionic acid; with the proviso that when R and $R^1$ are methyl, and X is bromo and n is 1, then the resolution is carried out other than in a toluene and methane solvent.

**28.** Process for preparing (d)-2-(6-methoxy-2-naphthyl)propionic acid or pharmaceutically acceptable salts thereof which process comprises:
(a) resolving a compound of formula I

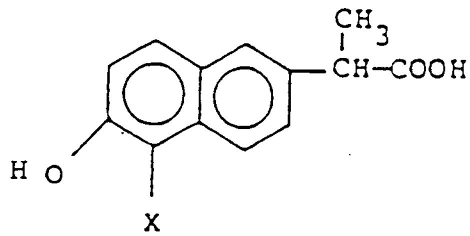

(I)

wherein X is hydrogen, bromo or chloro, or a salt of a compound of formula (I), with an N-R-D-glucamine wherein R is methyl, or a salt thereof; and

(b) converting the resultant d-2-(6-methoxy-2-naphthyl)propionic acid precursor to d-2-(6-methoxy-2-naphthyl)propionic acid.

**29.** Process for preparing (d)-2-(6-methoxy-2-naphthyl)propionic acid or pharmaceutically acceptable salts thereof which process comprises:

(a) resolving a compound of formula I

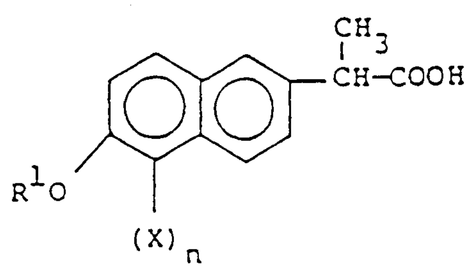

(I)

wherein R is hydrogen or methyl when X is halogen selected from bromo or chloro and n is 1, or wherein $R^1$ is hydrogen when n is 0, or a salt of a compound of formula (I), with an N-R-D-glucamine wherein R is alkyl of 2 to 18 carbon atoms or cycloalkyl of 3 to 8 carbon atoms or a salt thereof; and

(b) converting the resultant d-2-(6-methoxy-2-naphthyl)propionic acid precursor to d-2-(6-methoxy-2-naphthyl)propionic acid.

**30.** Process for preparing (d)-2-(6-methoxy-2 naphthyl)propionic acid or pharmaceutically acceptable salts thereof which process comprises:

(a) resolving a compound of formula I

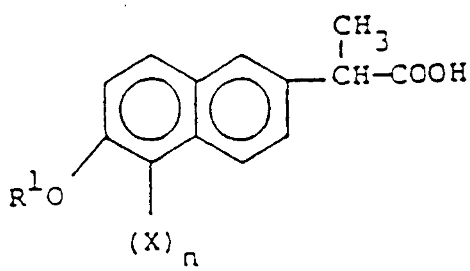

(I)

wherein R is hydrogen or methyl when X is halogen selected from bromo or chloro and n is 1, or wherein $R^1$ is hydrogen when n is 0, or a salt of a compound of formula (I), with an N-R-D-glucamine wherein R is alkyl of 6 to 18 carbon atoms or cycloalkyl of 3 to 8 carbon atoms or a salt thereof; and

(b) converting the resultant d-2-(6-methoxy-2-naphthyl)propionic acid precursor to d-2-(6-methoxy-2-naphthyl)propionic acid.

**31.** The process of any one of claims 27 to 30 wherein d-2-(6-hydroxy-2-naphthyl)propionic acid or a salt thereof is converted to d-2-(6-methoxy-2-naphthyl)propionic acid.

**32.** The process of any one of claims 27 to 30 wherein d-2-(5-halo-6-methoxy-2-naphthyl)propionic acid, d-2-(5-halo-6-hydroxy-2-naphthyl)propionic acid or a salt thereof is converted to d-2-(6-methoxy-2-naphthyl)propionic acid.

**33.** The process of claim 31 or 32 wherein d-2-(6-methoxy-2-naphthyl)propionicacid is converted to a pharmaceutically acceptable salt thereof.

**34.** A process for resolving a (d)- and (l)-2-(6-methoxy-2-naphthyl)propionic acid precursor of formula I

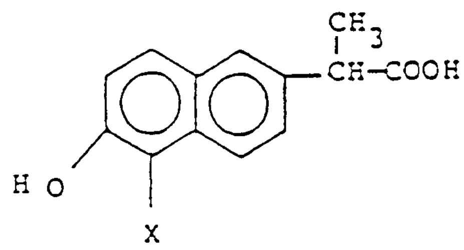

(I)

wherein X is hydrogen, chloro or bromo, or salts thereof,
into the enantiomers thereof characterized in that a N-R-D-glucamine or salt thereof wherein R is alkyl having 1 to 18 carbon atoms or cycloalkyl having 3 to 8 carbon atoms is used as the resolving agent to isolate said (d)-acid precursor and that said (l)-acid precursor is racemized followed by resolution.

**35.** A process for preparing (d)-2-(6-methoxy-2-naphthyl)propionic acid or pharmaceutically acceptable salts thereof which process comprises:
(a) resolving a precursor compound of formula I

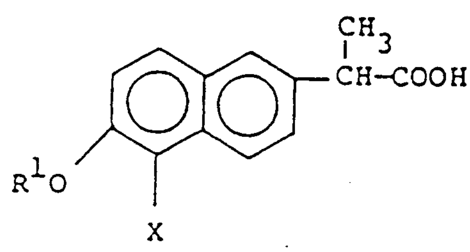

(I)

wherein $R^1$ is hydrogen or methyl when X is bromo or chloro; or $R^1$ is hydrogen when X is hydrogen; or a salt of a compound of formula I, with an N-R-D-glucamine wherein R is alkyl of 1 to 18 carbon atoms or cycloalkyl of 3 to 8 carbon atoms, or a salt of said glucamine, to isolate the (d)-form of said precursor compound;
(b) converting said (d)-form to (d)-2-(6-methoxy-2-naphthyl)propionic acid or a pharmaceutically acceptable salt thereof; and
(c) racemizing the (l)-form of said precursor compound and resolving the resultant racemic form of said precursor compound.

**36.** A process for resolving a (d)- and (l)-2-(6-methoxy-2-naphthyl)propionic acid precursor of formula I

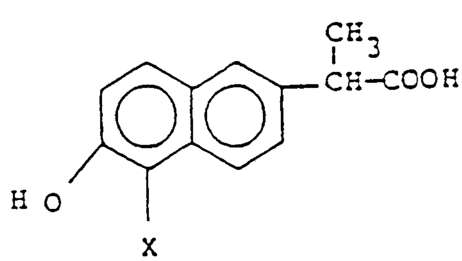

(I)

wherein X is hydrogen, chloro or bromo, or salts thereof,
into the enantiomers thereof characterized in that a N-R-D-glucamine or salt thereof wherein R is alkyl having 2 to 8 carbon atoms or cycloalkyl having 3 to 8 carbon atoms is used as the resolving agent to isolate said (d)-acid precursor and that said (l)-acid precursor is racemized followed by resolution.

**37.** A process for preparing (d)-2-(6-methoxy-2-naphthyl)propionic acid or pharmaceutically acceptable salts thereof which process comprises:
(a) resolving a precursor compound of formula I

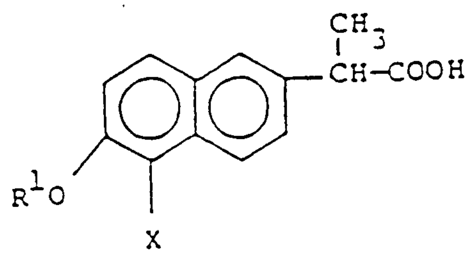

(I)

wherein $R^1$ is hydrogen or methyl when X is bromo or chloro; or
$R^1$ is hydrogen when X is hydrogen,
or a salt of a compound of formula I,
with an N-R-D-glucamine wherein R is alkyl of 2 to 8 carbon atoms or cycloalkyl of 3 to 8 carbon atoms,
or a salt of said glucamine,
to isolate the (d)-form of said precursor compound;
(b) converting said (d)-form to (d)-2-(6-methoxy-2-naphthyl)propionic acid or a pharmaceutically acceptable salt thereof; and
(c) racemizing the (l)-form of said precursor compound and resolving the resultant racemic form of said precursor compound.

**Claims for the following Contracting State : CH**

**1.** An N-R-D-glucamine salt of a d or l compound of formula I

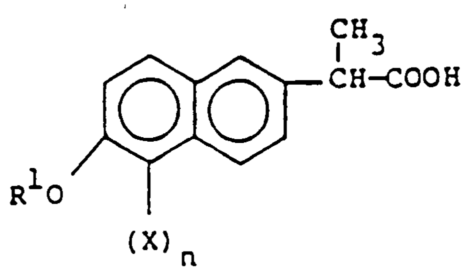

(I)

wherein R is methyl and $R^1$ is hydrogen or methyl when X is halogen selected from chloro and bromo and n is 1; or $R^1$ is hydrogen when n is 0.

**2.** An N-R-D-glucamine salt of a d or l compound of formula I

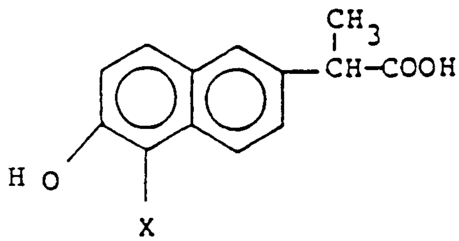

(I)

wherein R is methyl and X is hydrogen, chloro or bromo.

3. An N-R-D-glucamine salt of a d or l compound of formula I

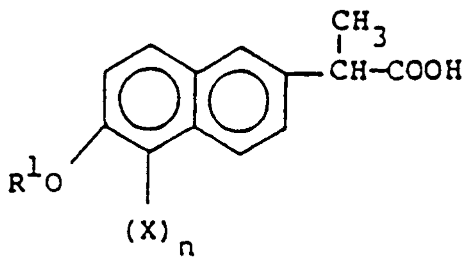

(I)

wherein R is alkyl from 2 to 18 carbon atoms or cycloalkyl from 3 to 8 carbon atoms and $R^1$ is hydrogen or methyl when X is halogen selected from chloro and bromo and n is 1; or $R^1$ is hydrogen when n is 0.

4. An N-R-D-glucamine salt of a d or l compound of formula I

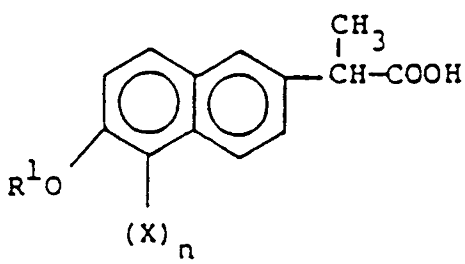

(I)

wherein R is alkyl from 6 to 18 carbon atoms or cycloalkyl from 3 to 8 carbon atoms, and $R^1$ is hydrogen or methyl when X is halogen selected from chloro and bromo and n is 1; or $R^1$ is hydrogen when n is 0.

5. The N-R-D-glucamine salt of claim 1 or claim 2 wherein R is methyl; and X is bromo.

6. The N-R-D-glucamine salt of claim 3 or claim 4 wherein R is n-octyl.

7. A mixture of the N-R-D-glucamine salts of the (d,l) acids of the formula I

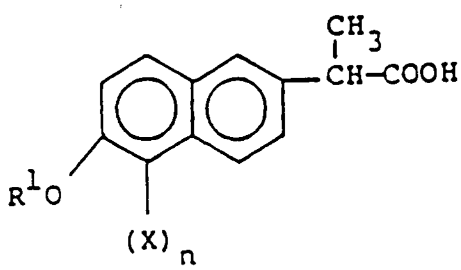

(I)

wherein $R^1$ is hydrogen or methyl when X is halogen selected from bromo and chloro and n is 1; or $R^1$ is hydrogen when n is 0, and R is methyl.

**8.** A mixture of the N-R-D-glucamine salts of the (d,l) acids of the formula I

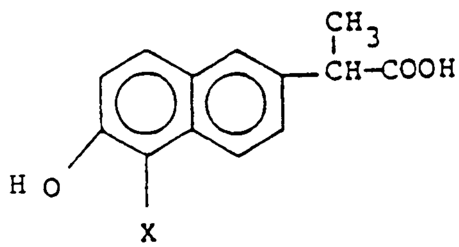

(I)

wherein X is hydrogen, bromo or chloro and R is methyl.

**9.** A mixture of the N-R-D-glucamine salts of the (d,l) acids of the formula I

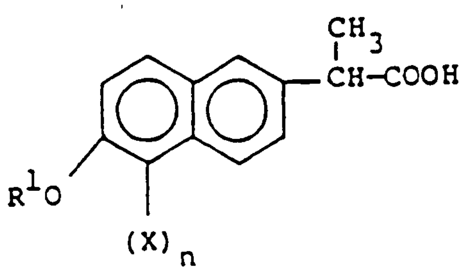

(I)

wherein $R^1$ is hydrogen or methyl when X is halogen selected from bromo and chloro and n is 1; or $R^1$ is hydrogen when n is 0, and R is alkyl with 2 to 18 carbon atoms or cycloalkyl with 3 to 8 carbon atoms.

**10.** A mixture of the N-R-D-glucamine salts of the (d,l) acids of the formula I

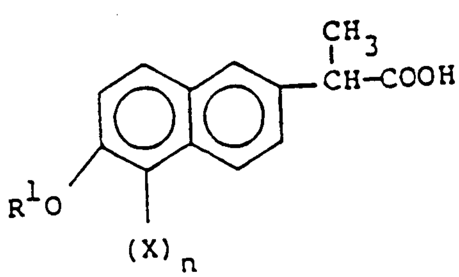

(I)

wherein $R^1$ is hydrogen or methyl when X is halogen selected from bromo and chloro and n is 1; or $R^1$ is hydrogen when n is 0, and R is alkyl with 6 to 18 carbon atoms or cycloalkyl with 3 to 8 carbon atoms.

11. A resolving medium comprising a mixture of the N-R-D-glucamine salts of the (d,l) acids of the formula I

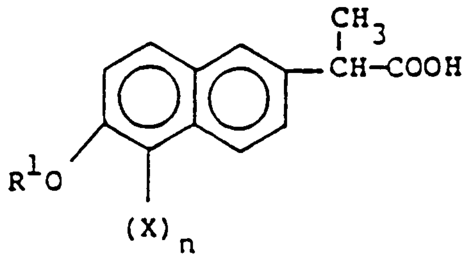

(I)

wherein $R^1$ is hydrogen or methyl when X is halogen selected from bromo and chloro and n is 1, or $R^1$ is hydrogen when n is 0, and R is methyl, and a solvent in which the solubility of the corresponding d-2-(6-methoxy-2-naphthyl)propionic acid precursor salt is considerably less than the solubility of the other diastereomeric salt at the resolution temperature.

12. A resolving medium comprising a mixture of the N-R-D-glucamine salts of the (d,l) acids of the formula I

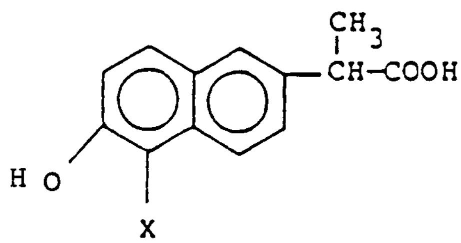

(I)

wherein X is hydrogen, bromo or chloro, and R is methyl, and a solvent in which the solubility of the corresponding d-2-(6-methoxy-2-naphthyl)propionic acid precursor salt is considerably less than the solubility of the other diastereomeric salt at the resolution temperature.

13. A resolving medium comprising a mixture of the N-R-D-glucamine salts of the (d,l) acids of the formula I

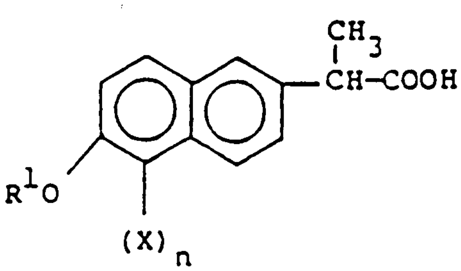

(I)

wherein $R^1$ is hydrogen or methyl when X is halogen selected from bromo and chloro and n is 1, or $R^1$ is hydrogen when n is 0, and R is alkyl having 2 to 18 carbon atoms or a cycloalkyl having 3 to 8 carbon atoms, and a solvent in which the solubility of the corresponding d-2-(6-methoxy-2-naphthyl)-propionic acid precursor salt is considerably less than the solubility of the other diastereomeric salt at the resolution temperature.

14. A resolving medium comprising a mixture of the N-R-D-glucamine salts of the (d,l) acids of the formula I

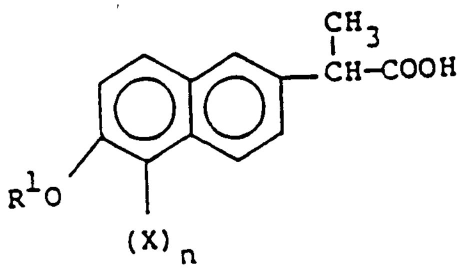

(I)

wherein $R^1$ is hydrogen or methyl when X is halogen selected from bromo and chloro and n is 1, or $R^1$ is hydrogen when n is 0, and R is alkyl having 6 to 18 carbon atoms or a cycloalkyl having 3 to 8 carbon atoms, and a solvent in which the solubility of the corresponding d-2-(6-methoxy-2-naphthyl)-propionic acid precursor salt is considerably less than the solubility of the other diastereomeric salt at the resolution temperature.

**15.** The d-isomer of a compound of formula I

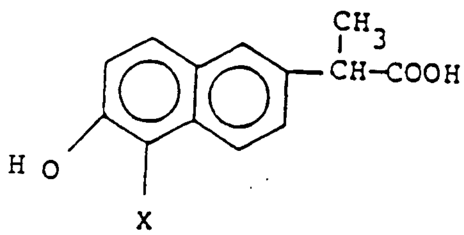

(I)

wherein X is hydrogen, chloro or bromo.

**16.** The l-isomer of a compound of formula I

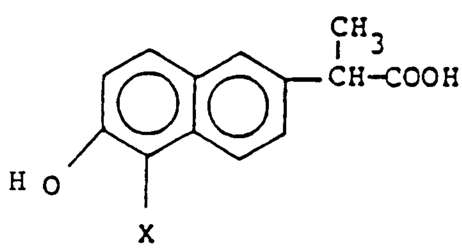

(I)

wherein X is hydrogen, chloro or bromo.

**17.** A process for the production of (d)-2-(6-methoxy-2-naphthyl)propionic acid, which process comprises conversion of an N-R-D-glucamine salt of any one of claims 1-6, wherein said N-R-D-glucamine salt is that of the d-isomer of a compound of formula I, to (d)-2-(6-methoxy-2-naphthyl)propionic acid.

**18.** A process according to claim 17 wherein (d)-2-(6-methoxy-2-naphthyl)propionic acid is converted to a pharmaceutically acceptable salt thereof.

**19.** Process for resolving mixtures of (d)- and (l)-2-(6-methoxy-2-naphthyl)propionic acid precursors of formula I

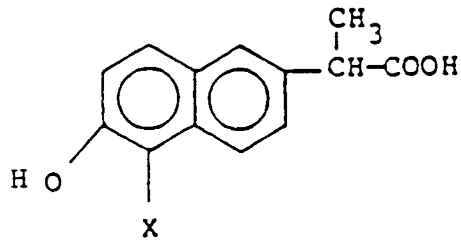

(I)

wherein X is hydrogen, chloro or bromo; or salts thereof;

into the enantiomers thereof characterized in that N-R-D-glucamines or salts thereof are used as the resolving agent, wherein R is methyl.

**20.** Process for resolving mixtures of (d) and (l)-2-(6-methoxy-2-naphthyl)propionic acid precursors of formula I

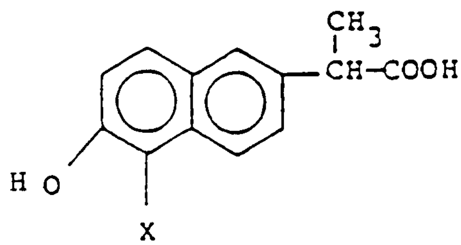

(I)

wherein X is hydrogen, chloro or bromo; or salts thereof;

into the enantiomers thereof characterized in that N-R-D-glucamines or salts thereof are used as the resolving agent, wherein R is octyl.

**21.** Process for resolving mixtures of (d)- and (l)-2-(6-methoxy-2-naphthyl)propionic acid precursors of formula I

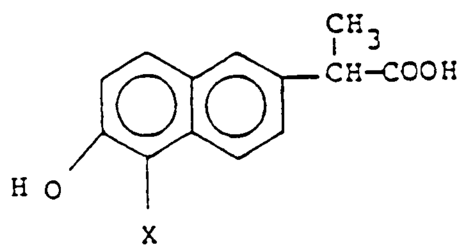

(I)

wherein X is hydrogen, chloro or bromo; or salts thereof;

into the enantiomers thereof characterized in that N-R-D-glucamines or salts thereof are used as the resolving agent, wherein R is alkyl or 2 to 18 carbon atoms or cycloalkyl of 3 to 8 carbon atoms.

**22.** Process for resolving mixtures of (d)- and (l)-2-(6-methoxy-2-naphthyl)propionic acid precursors of formula I

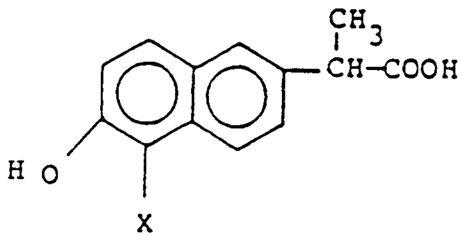

(I)

wherein X is hydrogen, chloro or bromo; or salts thereof;

into the enantiomers thereof characterized in that N-R-D-glucamines or salts thereof are used as the resolving agent, wherein R is alkyl of 6 to 18 carbon atoms or cycloalkyl of 3 to 8 carbon atoms.

**23.** The process of any one of claims 19 to 22 wherein the resulting pair of (d)- and (l)-acid N-R-D-glucamine salts is separated by fractional crystallization.

**24.** Process for preparing (d)-2-(6-methoxy-2-naphthyl)propionic acid or pharmaceutically acceptable salts thereof which process comprises:

(a) resolving a precursor of formula I

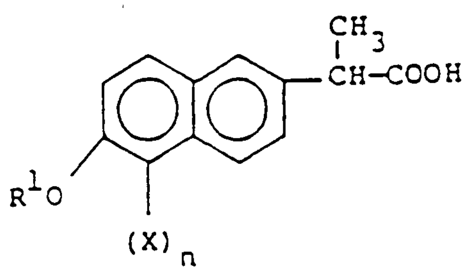

(I)

wherein $R^1$ is hydrogen or methyl when X is halogen selected from bromo and chloro and n is 1, or wherein $R^1$ is hydrogen when n is 0, or a salt of a compound of formula (I), with an N-R-D-glucamine wherein R is methyl or a salt thereof; and

(b) converting the resultant d-2-(6-methoxy-2-naphthyl)propionic acid precursor to d-2-(6-methoxynaphthyl)propionic acid:

except the process wherein the resolving agent N-methyl-D-glucamine and said precursor dl-2-(5-halo-6-methoxy-2-naphthyl)propionic acid are reacted in a solvent medium selected from mixtures of toluene and methanol in the presence of an optically inactive organic or inorganic base at a temperature between room temperature and 65 ° C, the less soluble N-methyl-D-glucamine salt of the d-isomer being obtained and contacted with a strong mineral acid to obtain d-2-(5-halo-6-methoxy-2-naphthyl)-propionic acid, which is catalytically dehalogenated by treatment with a suitable hydrogenation sytem which is capable of complete replacement of the 5-halo with hydrogen in an alkaline medium at a temperature of between room temperature and 100 ° C for a period between 1 and 4 hours.

**25.** Process for preparing (d)-2-(6-methoxy-2-naphthyl)propionic acid or pharmaceutically acceptable salts thereof which process comprises:

(a) resolving a compound of formula I

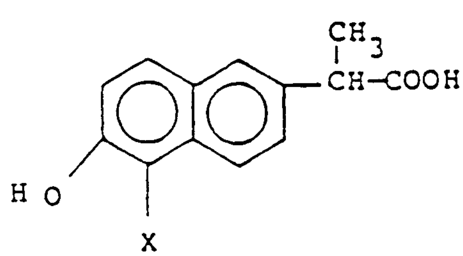

(I)

wherein X is hydrogen, bromo or chloro, or a salt of a compound of formula (I), with an N-R-D-glucamine wherein R is methyl, or a salt thereof; and

(b) converting the resultant d-2-(6-methoxy-2-naphthyl)propionic acid precursor to d-2-(6-methoxy-2-naphthyl)propionic acid.

**26.** Process for preparing (d)-2-(6-methoxy-2-naphthyl)propionic acid or pharmaceutically acceptable salts thereof which process comprises:

(a) resolving a compound of formula I

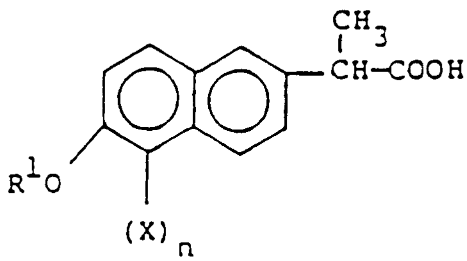

(I)

wherein $R^1$ is hydrogen or methyl when X is halogen selected from bromo or chloro and n is 1, or wherein $R^1$ is hydrogen when n is 0, or a salt of a compound of formula (I), with an N-R-D-glucamine wherein R is alkyl of 2 to 18 carbon atoms or cycloalkyl of 3 to 8 carbon atoms or a salt thereof; and

(b) converting the resultant d-2-(6-methoxy-2-naphthyl)propionic acid precursor to d-2-(6-methoxy-2-naphthyl)propionic acid.

**27.** Process for preparing (d)-2-(6-methoxy-2-naphthyl)propionic acid or pharmaceutically acceptable salts thereof which process comprises:

(a) resolving a compound of formula I

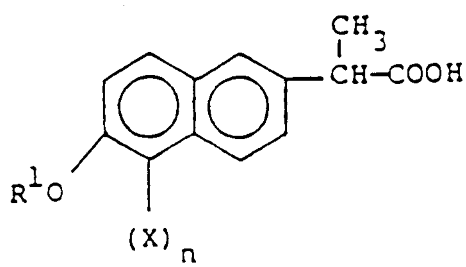

(I)

wherein R is hydrogen or methyl when X is halogen selected from bromo or chloro and n is 1, or wherein $R^1$ is hydrogen when n is 0, or a salt of a compound of formula (I), with an N-R-D-glucamine wherein R is alkyl of 6 to 18 carbon atoms or cycloalkyl or 3 to 8 carbon atoms or a salt thereof; and

(b) converting the resultant d-2-(6-methoxy-2-naphthyl)propionic acid precursor to d-2-(6-methoxy-2-naphthyl)propionic acid.

**28.** The process of any one of claims 24 to 27 wherein d-2-(6-hydroxy-2-naphthyl)propionic acid or a salt thereof is converted to d-2-(6-methoxy-2-naphthyl)propionic acid.

**29.** The process of any one of claims 24 to 27 wherein d-2-(5-halo-6-methoxy-2-naphthyl)propionic acid, d-2-(5-halo-6-hydroxy-2-naphthyl)propionic acid or a salt thereof is converted to d-2-(6-methoxy-2-naphthyl)propionic acid.

**30.** The process of claim 28 or 29 wherein d-2-(6-methoxy-2-naphthyl)propionicacid is converted to a pharmaceutically acceptable salt thereof.

**31.** A process for resolving a (d)- and (l)-2-(6-methoxy-2-naphthyl)propionic acid precursor of formula I

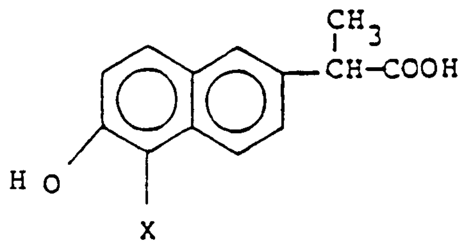

(I)

wherein X is hydrogen, chloro or bromo, or salts thereof,

into the enantiomers thereof characterized in that a N-R-D-glucamine or salt thereof wherein R is alkyl having 1 to 18 carbon atoms or cycloalkyl having 3 to 8 carbon atoms is used as the resolving agent to isolate said (d)-acid precursor and that said (l)-acid precursor is racemized followed by resolution.

**32.** A process for preparing (d)-2-(6-methoxy-2-naphthyl)propionic acid or pharmaceutically acceptable salts thereof which process comprises:

(a) resolving a precursor compound of formula I

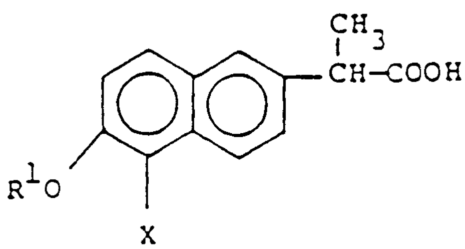

(I)

wherein $R^1$ is hydrogen or methyl when X is bromo or chloro; or $R^1$ is hydrogen when X is hydrogen; or a salt of a compound of formula I, with an N-R-D-glucamine wherein R is alkyl of 1 to 18 carbon atoms or cycloalkyl of 3 to 8 carbon atoms, or a salt of said glucamine, to isolate the (d)-form of said precursor compound;

(b) converting said (d)-form to (d)-2-(6-methoxy-2-naphthyl)propionic acid or a pharmaceutically acceptable salt thereof; and

(c) racemizing the (l)-form of said precursor compound and resolving the resultant racemic form of said precursor compound.

**33.** A process for resolving a (d)- and (l)-2-(6-methoxy-2-naphthyl)propionic acid precursor of formula I

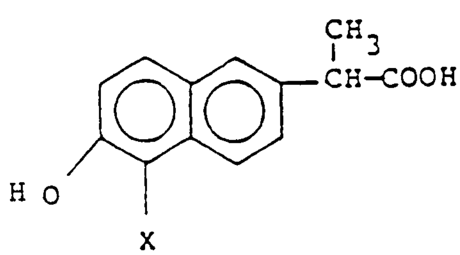

(I)

wherein X is hydrogen, chloro or bromo, or salts thereof,

into the enantiomers thereof characterized in that an N-R-D-glucamine or salt thereof wherein R is alkyl having 2 to 8 carbon atoms or cycloalkyl having 3 to 8 carbon atoms is used as the resolving agent to isolate said (d)-acid precursor and that said (l)-acid precursor is racemized followed by resolution.

**34.** A process for preparing (d)-2-(6-methoxy-2-naphthyl)propionic acid or pharmaceutically acceptable salts thereof which process comprises:

(a) resolving a precursor compound of formula I

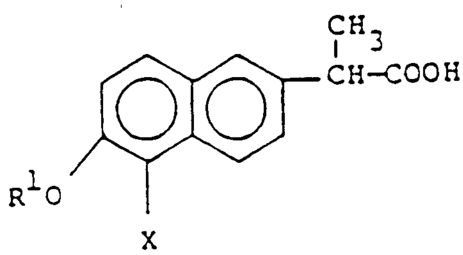

(I)

wherein $R^1$ is hydrogen or methyl when X is bromo or chloro; or
$R^1$ is hydrogen when X is hydrogen,
or a salt of a compound of formula I,
with an N-R-D-glucamine wherein R is alkyl of 2 to 8 carbon atoms or cycloalkyl of 3 to 8 carbon atoms,
or a salt of said glucamine,
to isolate the (d)-form of said precursor compound;
(b) converting said (d)-form to (d)-2-(6-methoxy-2-naphthyl)propionic acid or a pharmaceutically acceptable salt thereof; and
(c) racemizing the (l)-form of said precursor compound and resolving the resultant racemic form of said precursor compound.

**Patentansprüche**
**Patentansprüche für folgende Vertragstaaten : BE, DE, SE, NL, GB, FR, IT, LU**

**1.** N-R-D-Glucaminsalz einer d- oder l-Verbindung der Formel I

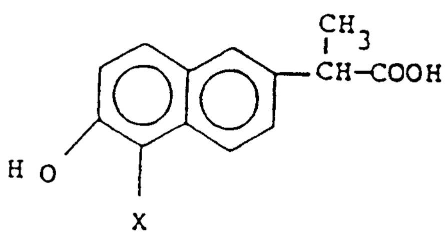

(I)

worin R Methyl ist und X Wasserstoff, Chlor oder Brom ist.

**2.** N-R-D-Glucaminsalz einer d- oder l-Verbindung der Formel I

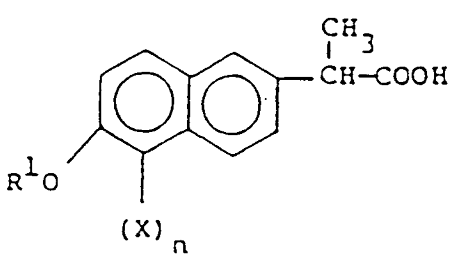

(I)

worin R Alkyl mit 2 bis 18 Kohlenstoffatomen oder Cycloalkyl mit 3 bis 8 Kohlenstoffatomen ist und $R^1$ Wasserstoff oder Methyl ist, wenn X Halogen, ausgewählt aus Chlor und Brom, ist und n 1 ist; oder $R^1$ Wasserstoff ist, wenn n 0 ist.

**3.** N-R-D-Glucaminsalz einer d- oder l-Verbindung der Formel I

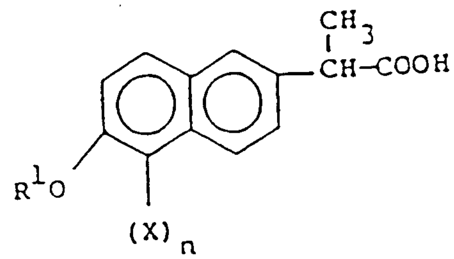

(I)

worin R Alkyl mit 6 bis 18 Kohlenstoffatomen oder Cycloalkyl mit 3 bis 8 Kohlenstoffatomen ist, und $R^1$ Wasserstoff oder Methyl ist, wenn X Halogen, ausgewählt aus Chlor und Brom, ist und n 1 ist; oder $R^1$ Wasserstoff ist, wenn n 0 ist.

**4.** N-R-D-Glucaminsalz nach Anspruch 1, worin R Methyl ist; und X Brom ist.

**5.** N-R-D-Glucaminsalz nach Anspruch 2 oder Anspruch 3, worin R n-Octyl ist.

**6.** Mischung der M-R-D-Glucaminsalze der (d,l)-Säuren der Formel I

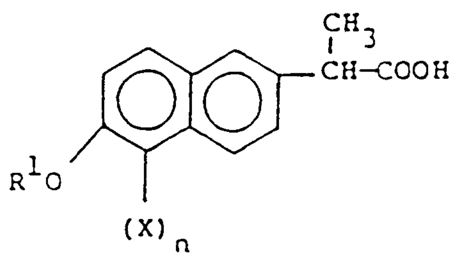

(I)

worin $R^1$ Wasserstoff oder Methyl ist, wenn X Halogen, ausgewählt aus Brom und Chlor, ist und n 1 ist; oder $R^1$ Wasserstoff ist, wenn n 0 ist, und R Methyl ist; mit der Maßgabe, daß, wenn $R^1$ Methyl ist und X Halogen ist, die Mischung nicht in einem Lösungsmittelsystem, das eine Mischung aus Toluol und Methanol umfaßt, ist.

**7.** Mischung der M-R-D-Glucaminsalze der (d,l)-Säuren der Formel I

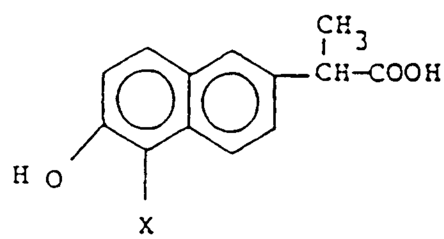

(I)

worin X Wasserstoff, Brom oder Chlor ist und R Methyl ist.

**8.** Mischung der N-R-D-Glucaminsalze der (d,l)-Säuren der Formel I

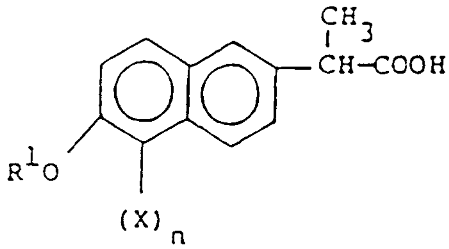

(I)

worin $R^1$ Wasserstoff oder Methyl ist, wenn X Halogen, ausgewählt aus Brom und Chlor, ist und n 1 ist; oder $R^1$ Wasserstoff ist, wenn n 0 ist, und R Alkyl mit 2 bis 18 Kohlenstoffatomen oder Cycloalkyl mit 3 bis 8 Kohlenstoffatomen ist.

**9.** Mischung der N-R-D-Glucaminsalze der (d,l)-Säuren der Formel I

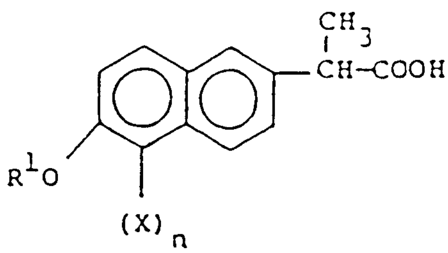

(I)

worin $R^1$ Wasserstoff oder Methyl ist, wenn X Halogen, ausgewählt aus Brom und Chlor, ist und n 1 ist; oder $R^1$ Wasserstoff ist, wenn n 0 ist, und R Alkyl mit 6 bis 18 Kohlenstoffatomen oder Cycloalkyl mit 3 bis 8 Kohlenstoffatomen ist.

**10.** Ein optische Isomere auftrennendes Medium, umfassend eine Mischung der N-R-D-Glucaminsalze der (d,l)-Säuren der Formel I

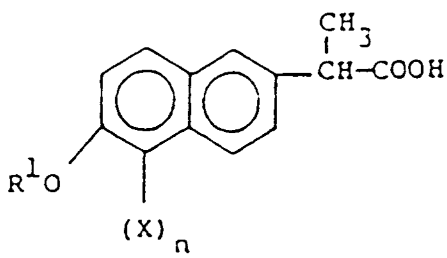

(I)

worin $R^1$ Wasserstoff oder Methyl ist, wenn X Halogen, ausgewählt aus Brom und Chlor, ist und n 1 ist, oder $R^1$ Wasserstoff ist, wenn n 0 ist, und R Methyl ist, und ein Lösungsmittel, in dem bei der Auftrennungstemperatur die Löslichkeit des entsprechenden d-2-(6-Methoxy-2-naphthyl)propionsäure-Vorläufersalzesbeträchtlich geringer ist als die Löslichkeit des anderen diastereomeren Salzes; mit der Maßgabe daß, wenn $R^1$ Methyl ist und X Halogen ist, die Mischung nicht in einem Lösungsmittelsystem, das eine Mischung von Toluol und Methanol umfaßt, vorliegt.

**11.** Ein optische Isomere auftrennendes Medium, umfassend eine Mischung der N-R-D-Glucaminsalze der (d,l)-Säuren der Formel I

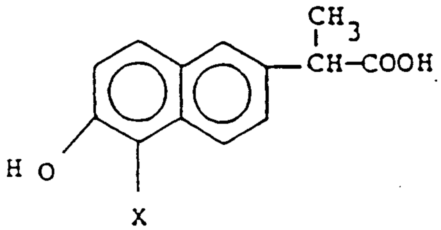

(I)

worin X Wasserstoff, Brom oder Chlor ist, und R Methyl ist, und ein Lösungsmittel, in dem bei der Auftrennungstemperatur die Löslichkeit des entsprechenden d-2-(6-Methoxy-2-naphthyl)propionsäure-Vorläufersalzesbeträchtlich geringer als die Löslichkeit des anderen diastereomeren Salzes ist.

**12.** Ein optische Isomere auftrennendes Medium, umfassend eine Mischung der N-R-D-Glucaminsalze der (d,l)-Säuren der Formel I

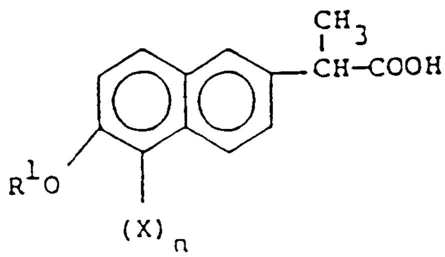

(I)

worin $R^1$ Wasserstoff oder Methyl ist, wenn X Halogen, ausgewählt aus Brom und Chlor, ist und n 1 ist, oder $R^1$ Wasserstoff ist, wenn n 0 ist, und R Alkyl mit 2 bis 18 Kohlenstoffatomen oder ein Cycloalkyl mit 3 bis 8 Kohlenstoffatomen ist, und ein Lösungsmittel, in dem bei der Auftrennungstemperatur die Löslichkeit des entsprechenden d-2-(6-Methoxy-2-naphthyl)propionsäure-Vorläufersalzesbeträchtlich geringer als die Löslichkeit des anderen diastereomeren Salzes ist.

**13.** Ein optische Isomere auftrennendes Medium, umfassend eine Mischung der N-R-D-Glucaminsalze der (d,l)-Säuren der Formel I

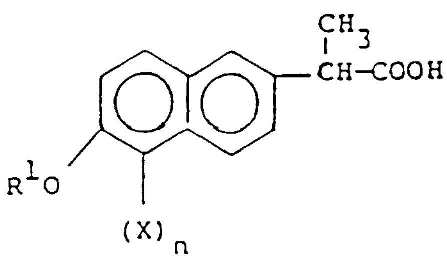

(I)

worin $R^1$ Wasserstoff oder Methyl ist, wenn X Halogen, ausgewählt aus Brom und Chlor, ist und n 1 ist, oder $R^1$ Wasserstoff ist, wenn n 0 ist, und R Alkyl mit 6 bis 18 Kohlenstoffatomen oder ein Cycloalkyl mit 3 bis 8 Kohlenstoffatomen ist, und ein Lösungsmittel, in dem bei der Auftrennungstemperatur die Löslichkeit des entsprechenden d-2-(6-Methoxy-2-naphthyl)propionsäure-Vorläufersalzesbeträchtlich geringer als die Löslichkeit des anderen diastereomeren Salzes ist.

**14.** d-Isomer einer Verbindung der Formel I

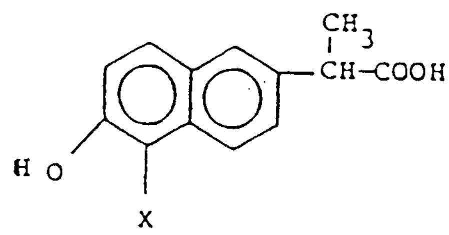

(I)

worin X Wasserstoff, Chlor oder Brom ist.

**15.** l-Isomer einer Verbindung der Formel I

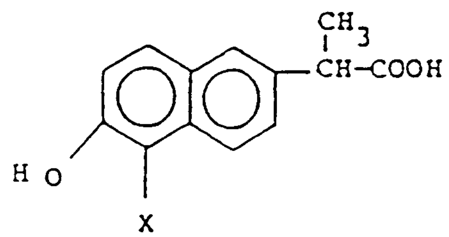

(I)

worin X Wasserstoff, Chlor oder Brom ist.

**16.** Verfahren zur Herstellung von (d)-2-(6-Methoxy-2-naphthyl)propionsäure, umfassend die Überführung eines N-R-D-Glucaminsalzes nach irgendeinem der Ansprüche 1 bis 5, worin das N-R-D-Glucaminsalz das des d-Isomers einer Verbindung der Formel I ist, in (d)-2-(6-Methoxy-2-naphthyl)propionsäure.

**17.** Verfahren nach Anspruch 16, worin (d)-2-(6-Methoxy-2-naphthyl)propionsäure in ein pharmazeutisch annehmbares Salz davon überführt wird.

**18.** Verfahren zur Auftrennung in optische Isomere von Mischungen von (d)- und (l)-2-(6-Methoxy-2-naphthyl)propionsäure-Vorläufern der Formel I

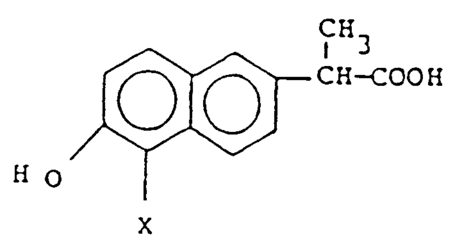

(I)

worin X Wasserstoff, Chlor oder Brom ist; oder von Salzen davon;
in die Enantiomere davon, dadurch gekennzeichnet, daß N-R-D-Glucamine oder Salze davon als das Auftrennungsmittel, worin R Methyl ist, verwendet werden.

**19.** Verfahren zur Auftrennung in optische Isomere von Mischungen von (d)- und (l)-2-(6-Methoxy-2-naphthyl)propionsäure-Vorlaufern der Formel I

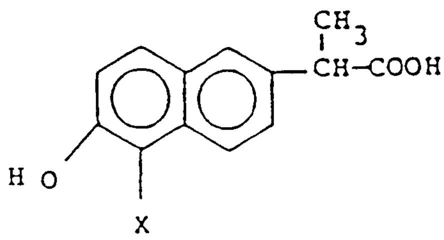

(I)

worin X Wasserstoff, Chlor oder Brom ist; oder von Salzen davon;
in die Enantiomere davon, dadurch gekennzeichnet, daß N-R-D-Glucamine oder Salze davon als das Auftrennungsmittel, worin R Octyl ist, verwendet werden.

**20.** Verfahren zur Auftrennung in optische Isomere von Mischungen von (d)- und (l)-2-(6-Methoxy-2-naphthyl)propionsäure-Vorläufern der Formel I

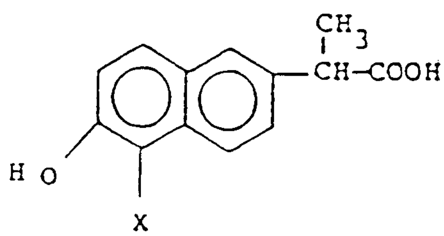

(I)

worin X Wasserstoff, Chlor oder Brom ist; oder von Salzen davon;
in die Enantiomere davon, dadurch gekennzeichnet, daß N-R-D-Glucamin oder Salze davon als das Auftrennungsmittel, worin R Alkyl mit 2 bis 18 Kohlenstoffatomen oder Cycloalkyl mit 3 bis 8 Kohlenstoffatomen ist, verwendet werden.

**21.** Verfahren zur Auftrennung in optische Isomere von Mischungen von (d)- und (l)-2-(6-Methoxy-2-naphthyl)propionsäure-Vorläufern der Formel I

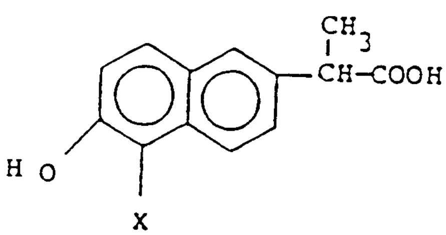

(I)

worin X Wasserstoff, Chlor oder Brom ist; oder von Salzen davon;
in die Enantiomere davon, dadurch gekennzeichnet, daß N-R-D-Glucamine oder Salze davon als das Auftrennungsmittel, worin R Alkyl mit 6 bis 18 Kohlenstoffatomen oder Cycloalkyl mit 3 bis 8 Kohlenstoffatomen ist, verwendet werden.

**22.** Verfahren nach irgendeinem der Ansprüche 18 bis 21, worin das resultierende Paar von (d)- und (l)-Säure-N-R-D-Glucaminsalzen durch fraktionierte Kristallisation aufgetrennt wird.

**23.** Verfahren zur Herstellung von (d)-2-(6-Methoxy-2-naphthyl)propionsäure oder pharmazeutisch annehmbaren Salzen davon, umfassend:
(a) das Auftrennen in optische Isomere eines Vorläufers der Formel I

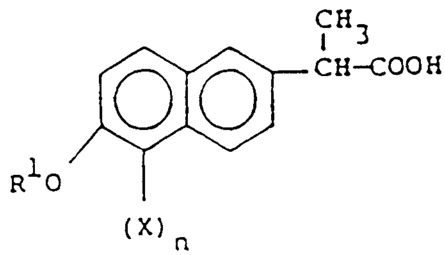

(I)

worin $R^1$ Wasserstoff oder Methyl ist, wenn X Halogen, ausgewählt aus Brom und Chlor, ist und n 1 ist, oder worin $R^1$ Wasserstoff ist, wenn n 0 ist, oder eines Salzes einer Verbindung der Formel (I), mit einem N-R-D-Glucamin, worin R Methyl ist, oder einem Salz davon; mit der Maßgabe, daß, wenn das Auftrennungsmittel N-Methyl-D-glucamin ist und der Vorläufer dl-2-(5-Halogen-6-methoxy-2-naphthyl)propionsäure ist, und sie zusammen in einem Lösungsmittelsystem, aus dem N-Methyl-D-glucamin-d-2-(5-halogen-6-methoxy-2-naphthyl)propionat abgetrennt wird, umgesetzt werden, das Lösungsmittelsystem nicht eine Mischung von Toluol und Methanol ist; und

(b) das Überführen des resultierenden d-2-(6-Methoxy-2-naphthyl)propionsäure-Vorläufers in d-2-(6-Methoxy-naphthyl)propionsäure.

**24.** Verfahren zur Herstellung von (d)-2-(6-Methoxy-2-naphthyl)propionsäure oder pharmazeutisch annehmbaren Salzen davon, umfassend:

(a) das Auftrennen in optische Isomere einer Verbindung der Formel I

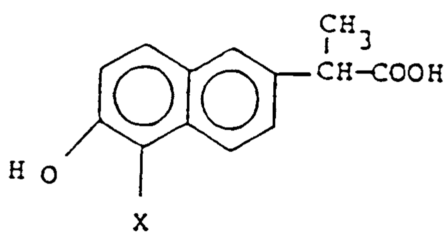

(I)

worin X Wasserstoff, Brom oder Chlor ist, oder eines Salzes einer Verbindung der Formel (I), mit einem N-R-D-Glucamin, worin R Methyl ist, oder einem Salz davon; und

(b) das Überführen des resultierenden d-2-(6-Methoxy-2-naphthyl)propionsäure-Vorläufer in d-2-(6-Methoxy-2-naphthyl)propionsäure.

**25.** Verfahren zur Herstellung von (d)-2-(6-Methoxy-2-naphthyl)propionsäure oder pharmazeutisch annehmbaren Salzen davon, umfassend:

(a) das Auftrennen in optische Isomere einer Verbindung der Formel I

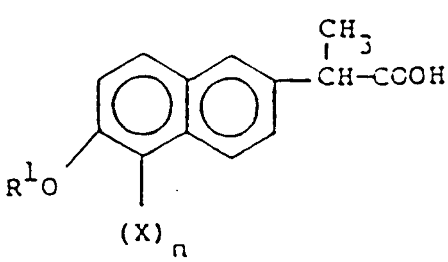

(I)

worin R Wasserstoff oder Methyl ist, wenn X Halogen, ausgewählt aus Brom oder Chlor, ist und n 1 ist, oder worin $R^1$ Wasserstoff ist, wenn n 0 ist, oder eines Salzes einer Verbindung der Formel (I), mit einem N-R-D-Glucamin, worin R Alkyl mit 2 bis 18 Kohlenstoffatomen oder Cycloalkyl mit 3 bis 8 Kohlenstoffatomen ist, oder einem Salz davon; und

(b) das Überführen des resultierenden d-2-(6-Methoxy-2-naphthyl)propionsäure-Vorläufers in d-2-(6-Methoxy-2-naphthyl)propionsäure.

**26.** Verfahren zur Herstellung von (d)-2-(6-Methoxy-2-naphthyl)propionsäure oder pharmazeutisch annehmbaren Salzen davon, umfassend:
(a) das Auftrennen in optische Isomere einer Verbindung der Formel I

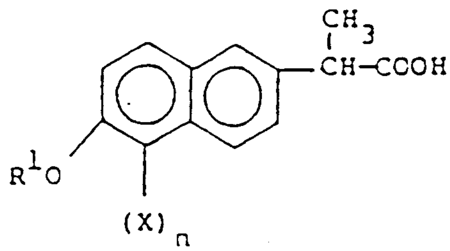

(I)

worin R Wasserstoff oder Methyl ist, wenn X Halogen, ausgewählt aus Brom oder Chlor, ist und n 1 ist, oder worin $R^1$ Wasserstoff ist, wenn n 0 ist, oder eines Salzes einer Verbindung der Formel (I), mit einem N-R-D-Glucamin, worin R Alkyl mit 6 bis 18 Kohlenstoffatomen oder Cycloalkyl mit 3 bis 8 Kohlenstoffatomen ist, oder einem Salz davon; und
(b) das Überführen des resultierenden d-2-(6-Methoxy-2-naphthyl)propionsäure-Vorläufers in d-2-(6-Methoxy-2-naphthyl)propionsäure.

**27.** Verfahren nach irgendeinem der Ansprüche 23 bis 26, worin d-2-(6-Hydroxy-2-naphthyl)propionsäure oder ein Salz davon in d-2-(6-Methoxy-2-naphthyl)propionsäure überführt wird.

**28.** Verfahren nach irgendeinem der Ansprüche 23 bis 26, worin d-2-(5-Halogen-6-methoxy-2-naphthyl)-propionsäure, d-2-(5-Halogen-6-hydroxy-2-naphthyl)propionsäure oder ein Salz davon in d-2-(6-Methoxy-2-naphthyl)propionsäure überführt wird.

**29.** Verfahren nach Anspruch 27 oder 28, worin d-2-(6-Methoxy-2-naphthyl)propionsäure in ein pharmazeutisch annehmbares Salz davon überführt wird.

**30.** Verfahren zur Auftrennung in optische Isomere eines (d)- und (l)-2-(6-Methoxy-2-naphthyl)propionsäure-Vorläufers der Formel I

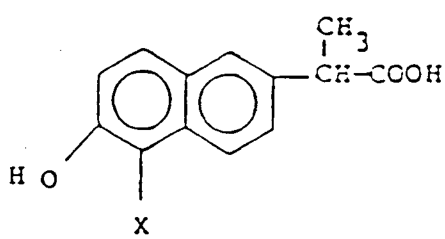

(I)

worin X Wasserstoff, Chlor oder Brom ist, oder von Salzen davon,
in die Enantiomere davon, dadurch gekennzeichnet, daß ein N-R-D-Glucamin oder Salz davon, worin R Alkyl mit 1 bis 18 Kohlenstoffatomen oder Cycloalkyl mit 3 bis 8 Kohlenstoffatomen ist, als Auftrennungsmittel verwendet wird, um den (d)-Säurevorläufer zu isolieren, und daß der (l)-Säurevorläufer racemisiert wird, gefolgt von Auftrennung in optische Isomere.

**31.** Verfahren zur Herstellung von (d)-2-(6-Methoxy-2-naphthyl)propionsäure oder pharmazeutisch annehmbaren Salzen davon, umfassend:
(a) das Auftrennen in optische Isomere einer Vorläuferverbindung der Formel I

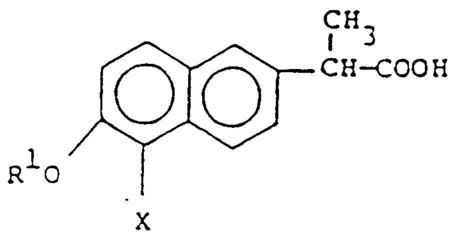

(I)

worin $R^1$ Wasserstoff oder Methyl ist, wenn X Brom oder Chlor ist; oder $R^1$ Wasserstoff ist, wenn X Wasserstoff ist; oder eines Salzes einer Verbindung der Formel I, mit einem N-R-D-Glucamin, worin R Alkyl mit 1 bis 18 Kohlenstoffatomen oder Cycloalkyl mit 3 bis 8 Kohlenstoffatomen ist, oder einem Salz des Glucamins, um die (d)-Form der Vorläuferverbindung zu isolieren;
(b) das Überführen der (d)-Form in (d)-2-(6-Methoxy-2-naphthyl)propionsäure oder ein pharmazeutisch annehmbares Salz davon; und
(c) das Racemisieren der (l)-Form der Vorläuferverbindung und das Auftrennen in optische Isomere der resultierenden racemischen Form der Vorläuferverbindung.

**32.** Verfahren zur Auftrennung in optische Isomere eines (d)- und (l)-2-(6-Methoxy-2-naphthyl)propionsäure-Vorläufers der Formel I

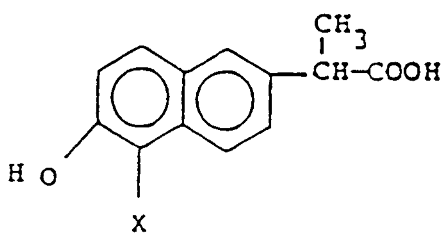

(I)

worin X Wasserstoff, Chlor oder Brom ist, oder von Salzen davon,
in die Enantiomere davon, dadurch gekennzeichnet, daß ein N-R-D-Glucamin oder Salz davon, worin R Alkyl mit 2 bis 8 Kohlenstoffatomen oder Cycloalkyl mit 3 bis 8 Kohlenstoffatomen ist, als das Auftrennungsmittel verwendet wird, um den (d)-Säurevorläufer zu isolieren, und daß der (l)-Säurevorläufer racemisiert wird, gefolgt von Auftrennung in optische Isomere.

**33.** Verfahren zur Herstellung von (d)-2-(6-Methoxy-2-naphthyl)propionsäure oder pharmazeutisch annehmbaren Salzen davon, umfassend:
(a) das Auftrennen in optische Isomere einer Vorläuferverbindung der Formel I

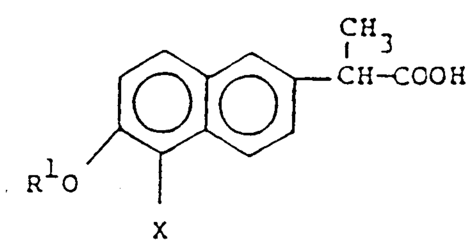

(I)

worin $R^1$ Wasserstoff oder Methyl ist, wenn X Brom oder Chlor ist; oder
$R^1$ Wasserstoff ist, wenn X Wasserstoff ist,
oder eines Salzes einer Verbindung der Formel I,
mit einem N-R-D-Glucamin, worin R Alkyl mit 2 bis 8 Kohlenstoffatomen oder Cycloalkyl mit 3 bis 8 Kohlenstoffatomen ist,
oder einem Salz des Glucamins,
um die (d)-Form der Vorläuferverbindung zu isolieren;

(b) das Überführen der (d)-Form in (d)-2-(6-Methoxy-2-naphthyl)propionsäure oder ein pharmazeutisch annehmbares Salz davon; und

(c) das Racemisieren der (l)-Form der Vorläuferverbindung und das Auftrennen in optische Isomere der resultierenden racemischen Form der Vorläuferverbindung.

**Patentansprüche für folgenden Vertragsstaat : AT**

**1.** Verfahren für die Auftrennung der d- und l-Isomeren der Formel I

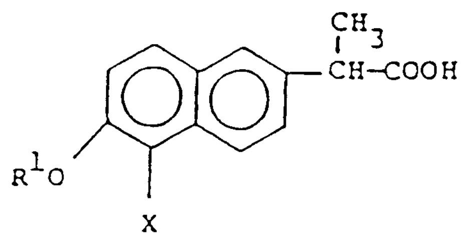

(I)

worin $R^1$ Methyl ist und X Brom ist; gekennzeichnet durch die Auftrennung in optische Isomere von diastereomeren Salzen von N-R-D-Glucamin, worin R Methyl ist, und einer Mischung von d- und l-Isomeren der Formel I.

**2.** Verfahren, das die Auftrennung in optische Isomere der diastereomeren Salze von N-R-D-Glucamin und einer Mischung von d- und l-Isomeren der Formel I

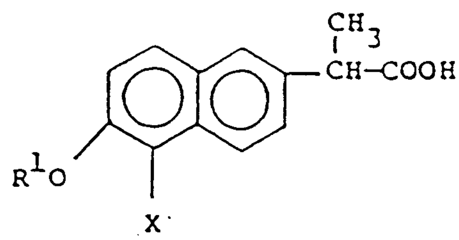

(I)

worin R Methyl ist, $R^1$ Methyl ist und X Chlor ist, umfaßt.

**3.** Verfahren, das die Auftrennung in optische Isomere von diastereomeren Salzen von N-R-D-Glucamin und einer Mischung von d- und l-Isomeren der Formel I

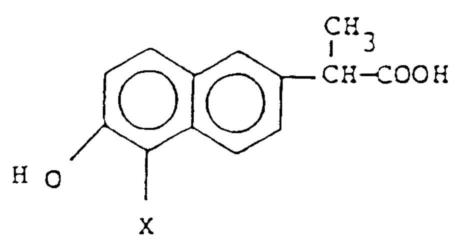

(I)

worin R Methyl ist und X Wasserstoff, Chlor oder Brom ist, umfaßt.

**4.** Verfahren, das die Auftrennung in optische Isomere der diastereomeren Salze von N-R-D-Glucamin und einer Mischung von d- und l-Isomeren der Formel I

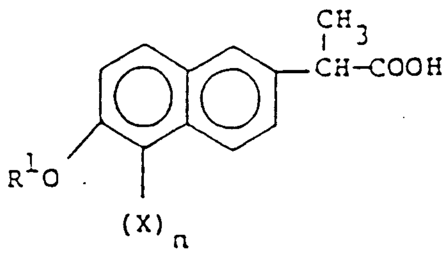

(I)

worin R Alkyl mit 2 bis 18 Kohlenstoffatomen oder Cycloalkyl mit 3 bis 8 Kohlenstoffatomen ist und $R^1$ Wasserstoff oder Methyl ist, wenn X Halogen, ausgewählt aus Chlor und Brom, ist und n 1 ist; oder $R^1$ Wasserstoff ist, wenn n 0 ist, umfaßt.

**5.** Verfahren, das die Auftrennung in optische Isomere von diastereomeren Salzen von N-R-D-Glucamin und einer Mischung von d- und l-Isomeren der Formel I

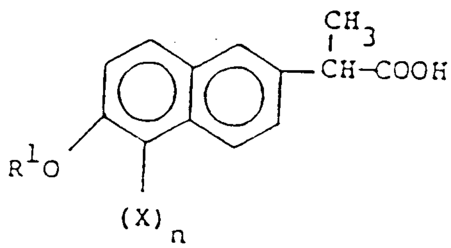

(I)

worin R Alkyl mit 6 bis 18 Kohlenstoffatomen oder Cycloalkyl mit 3 bis 8 Kohlenstoffatomen ist und $R^1$ Wasserstoff oder Methyl ist, wenn X Halogen, ausgewählt aus Chlor und Brom, ist und n 1 ist; oder $R^1$ Wasserstoff ist, wenn n 0 ist, umfaßt.

**6.** Verfahren nach Anspruch 3, worin X Brom ist.

**7.** Verfahren nach Anspruch 5, worin R n-Octyl ist.

**8.** Verfahren zur Trennung der d- und l-Isomeren der Formel I

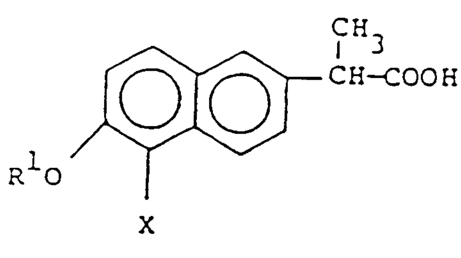

(I)

worin $R^1$ Methyl ist und X Brom ist; gekennzeichnet durch die Umsetzung von N-R-D-Glucamin, worin R Methyl ist, mit einer Mischung von d- und l-Isomeren der Formel I.

**9.** Verfahren, umfassend die Umsetzung von N-R-D-Glucamin mit einer Mischung von d- und l-Isomeren der Formel I

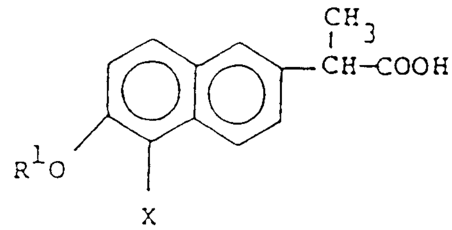

(I)

worin $R^1$ Methyl ist, X Chlor ist und R Methyl ist.

**10.** Verfahren, umfassend die Umsetzung von N-R-D-Glucamin mit einer Mischung von d- und l-Isomeren der Formel I

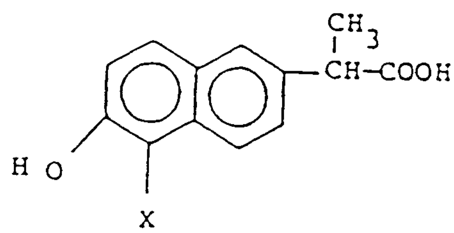

(I)

worin X Wasserstoff, Brom oder Chlor ist und R Methyl ist.

**11.** Verfahren, umfassend die Umsetzung von N-R-D-Glucamin mit einer Mischung von d- und l-Isomeren der Formel I

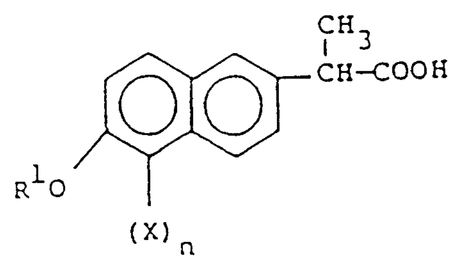

(I)

worin $R^1$ Wasserstoff oder Methyl ist, wenn X Halogen, ausgewählt aus Brom und Chlor, ist und n 1 ist; oder $R^1$ Wasserstoff ist, wenn n 0 ist, und R Alkyl mit 2 bis 18 Kohlenstoffatomen oder Cycloalkyl mit 3 bis 8 Kohlenstoffatomen ist.

**12.** Verfahren, umfassend die Umsetzung von N-R-D-Glucamin mit einer Mischung von d- und l-Isomeren der Formel I

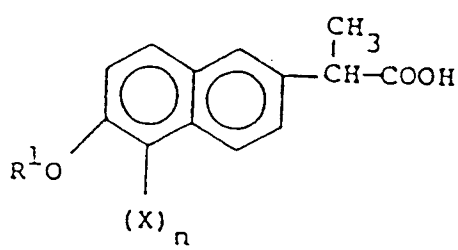

(I)

worin $R^1$ Wasserstoff oder Methyl ist, wenn X Halogen, ausgewählt aus Brom und Chlor, ist und n 1 ist; oder $R^1$ Wasserstoff ist, wenn n 0 ist, und R Alkyl mit 6 bis 18 Kohlenstoffatomen oder Cycloalkyl mit 3 bis 8 Kohlenstoffatomen ist.

**13.** Verfahren zur Auftrennung der d- und l-Isomeren der Formel I

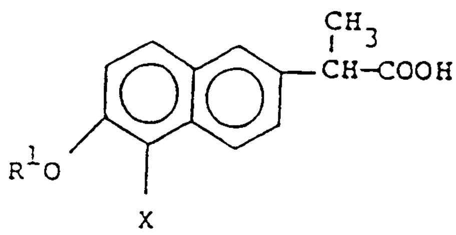

(I)

worin $R^1$ Methyl ist, wenn X Brom ist, gekennzeichnet durch die Herstellung eines Auftrennungsmediums, das eine Mischung von diastereomeren Salzen von N-R-D-Glucamin, worin R Methyl ist, und den d- und l-Isomeren der Formel I und ein Lösungsmittel, in dem bei der Auftrennungstemperatur die Löslichkeit des entsprechenden d-2-(6-Methoxy-2-naphthyl)propionsäure-Vorläufersalzesbeträchtlich geringer als die Löslichkeit des anderen diastereomeren Salzes ist, umfaßt.

**14.** Verfahren, umfassend die Herstellung eines Mediums zur Autrennung in optische Isomere, das eine Mischung der N-R-D-Glucaminsalze der (d,l)-Säuren der Formel I

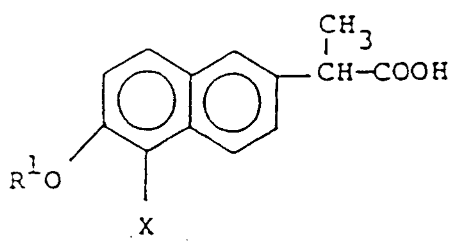

(I)

worin $R^1$ Methyl ist, X Chlor ist und R Methyl ist, und ein Lösungsmittel, in dem bei der Auftrennungstemperatur die Löslichkeit des entsprechenden d-2-(6-Methoxy-2-naphthyl)propionsäure-Vorläufersalzesbeträchtlich geringer als die Löslichkeit des anderen diastereomeren Salzes ist, umfaßt.

**15.** Verfahren, umfassend die Herstellung eines Mediums zur Auftrennung in optische Isomere, das eine Mischung der N-R-D-Glucaminsalze der (d,l)-Säuren der Formel I

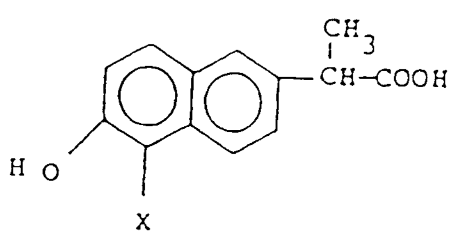

(I)

worin X Wasserstoff, Brom oder Chlor ist und R Methyl ist, und ein Lösungsmittel, in dem bei der Auftrennungstemperatur die Löslichkeit des entsprechenden d-2-(6-Methoxy-2-naphthyl)propionsäure-Vorläufersalzesbeträchtlich geringer als die Löslichkeit des anderen diastereomeren Salzes ist, umfaßt.

**16.** Verfahren, umfassend die Herstellung eines Mediums zur Auftrennung in optische Isomere, das eine Mischung der N-R-D-Glucaminsalze der (d,l)-Säuren der Formel I

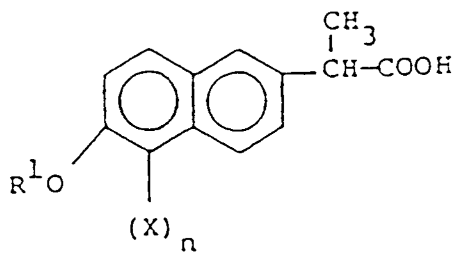

(I)

worin $R^1$ Wasserstoff oder Methyl ist, wenn X Halogen, ausgewählt aus Brom und Chlor, ist und n 1 ist, oder $R^1$ Wasserstoff ist, wenn n 0 ist, und R Alkyl mit 2 bis 18 Kohlenstoffatomen oder ein Cycloalkyl mit 3 bis 8 Kohlenstoffatomen ist, und ein Lösungsmittel, in dem bei der Auftrennungstemperatur die Löslichkeit des entsprechenden d-2-(6-Methoxy-2-naphthyl)propionsäure-Vorläufersalzesbeträchtlich geringer als die Löslichkeit des anderen diastereomeren Salzes ist, umfaßt.

**17.** Verfahren, umfassend die Herstellung eines Mediums zur Auftrennung in optische Isomere, das eine Mischung der N-R-D-Glucaminsalze der (d,l)-Säuren der Formel I

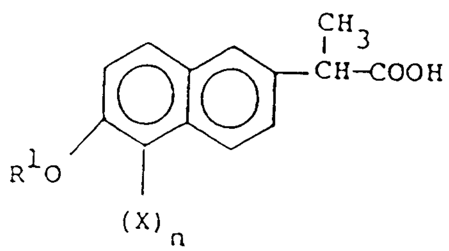

(I)

worin $R^1$ Wasserstoff oder Methyl ist, wenn X Halogen, ausgewählt aus Brom und Chlor, ist und n 1 ist, oder $R^1$ Wasserstoff ist, wenn n 0 ist, und R Alkyl mit 6 bis 18 Kohlenstoffatomen oder ein Cycloalkyl mit 3 bis 8 Kohlenstoffatomen ist, und ein Lösungsmittel, in dem bei der Auftrennungstemperatur die Löslichkeit des entsprechenden d-2-(6-Methoxy-2-naphthyl)propionsäure-Vorläufersalzesbeträchtlich geringer als die Löslichkeit des anderen diastereomeren Salzes ist, umfaßt.

**18.** Verfahren, umfassend die Verwendung von N-R-D-Glucamin, worin R Alkyl mit 2 bis 18 Kohlenstoffatomen oder Cycloalkyl mit 3 bis 8 Kohlenstoffatomen ist, als Mittel zur Auftrennung in optische Isomere bei der Herstellung des d-Isomers einer Verbindung der Formel I

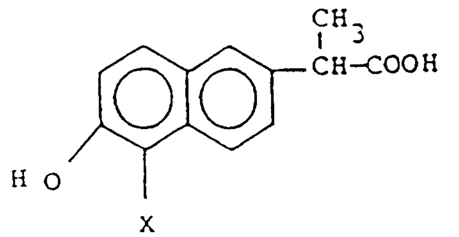

(I)

worin X Wasserstoff, Chlor oder Brom ist.

**19.** Verfahren, umfassend die Verwendung von N-R-D-Glucamin, worin R Alkyl mit 2 bis 18 Kohlenstoffatomen oder Cycloalkyl mit 3 bis 8 Kohlenstoffatomen ist, als Mittel zur Auftrennung in optische Isomere bei der Herstellung des l-Isomers einer Verbindung der Formel I

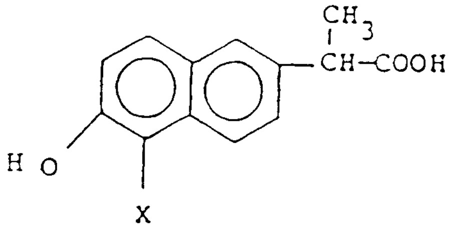

(I)

worin X Wasserstoff, Chlor oder Brom ist.

**20.** Verfahren zur Herstellung von (d)-2-(6-Methoxy-2-naphthyl)propionsäure, umfassend die Überführung eines N-R-D-Glucaminsalzes nach irgendeinem der Ansprüche 1 bis 7, worin das N-R-D-Glucaminsalz das des d-Isomers einer Verbindung der Formel I ist, in (d)-2-(6-Methoxy-2-naphthyl)propionsäure.

**21.** Verfahren nach Anspruch 20, worin (d)-2-(6-Methoxy-2-naphthyl)propionsäure in ein pharmazeutisch annehmbares Salz davon überführt wird.

**22.** Verfahren zur Auftrennung in optische Isomere von Mischungen von (d)- und (l)-2-(6-Methoxy-2-naphthyl)propionsäure-Vorläufern der Formel I

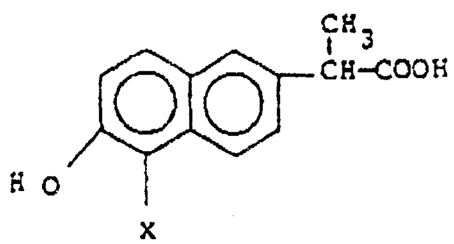

(I)

worin X Wasserstoff, Chlor oder Brom ist; oder von Salzen davon;
in die Enantiomere davon, dadurch gekennzeichnet, daß N-R-D-Glucamine oder Salze davon als das Auftrennungsmittel, worin R Methyl ist, verwendet werden.

**23.** Verfahren zur Auftrennung in optische Isomere von Mischungen von (d)- und (l)-2-(6-Methoxy-2-naphthyl)propionsäure-Vorläufern der Formel I

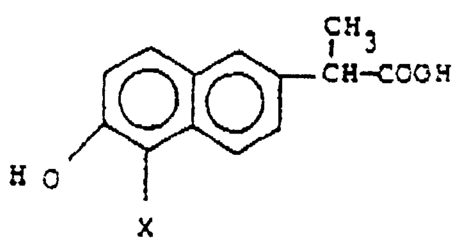

(I)

worin X Wasserstoff, Chlor oder Brom ist; oder von Salzen davon;
in die Enantiomere davon, dadurch gekennzeichnet, daß N-R-D-Glucamine oder Salze davon als Auftrennungsmittel, worin R Octyl ist, verwendet werden.

**24.** Verfahren zur Auftrennung in optische Isomere von Mischungen von (d)- und (l)-2-(6-Methoxy-2-naphthyl)propionsäure-Vorläufern der Formel I

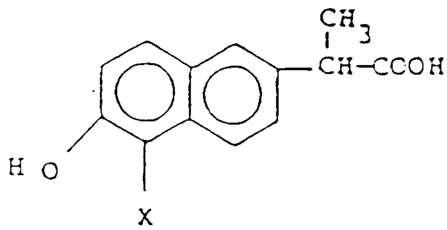

(I)

worin X Wasserstoff, Chlor oder Brom ist; oder von Salzen davon; in die Enantiomere davon, dadurch gekennzeichnet, daß N-R-D-Glucamine oder Salze davon als das Auftrennungsmittel, worin R Alkyl mit 2 bis 18 Kohlenstoffatomen oder Cycloalkyl mit 3 bis 8 Kohlenstoffatomen ist, verwendet werden.

**25.** Verfahren zur Auftrennung in optische Isomere von Mischungen von (d)- und (l)-2-(6-Methoxy-2-naphthyl)propionsäure-Vorläufern der Formel I

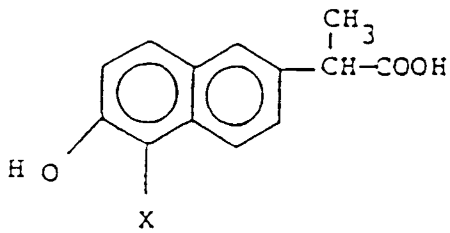

(I)

worin X Wasserstoff, Chlor oder Brom ist; oder von Salzen davon; in die Enantiomere davon, dadurch gekennzeichnet, daß N-R-D-Glucamine oder Salze davon als das Auftrennungsmittel, worin R Alkyl mit 6 bis 18 Kohlenstoffatomen oder Cycloalkyl mit 3 bis 8 Kohlenstoffatomen ist, verwendet werden.

**26.** Verfahren nach irgendeinem der Ansprüche 22 bis 25, worin das resultierende Paar von (d)- und (l)-Säure-N-R-D-Glucaminsalzen durch fraktionierte Kristallisation aufgetrennt wird.

**27.** Verfahren zur Herstellung von (d)-2-(6-Methoxy-2-naphthyl)propionsäure oder pharmazeutisch annehmbaren Salzen davon, umfassend:

(a) das Auftrennen in optische Isomere einer Verbindung der Formel I

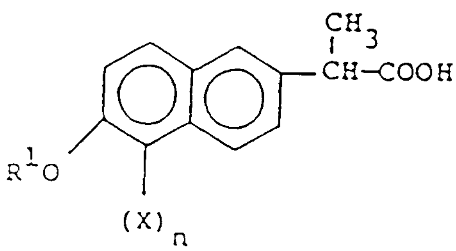

(I)

worin $R^1$ Wasserstoff oder Methyl ist, wenn X Halogen, ausgewählt aus Brom und Chlor, ist und n 1 ist, oder worin $R^1$ Wasserstoff ist, wenn n 0 ist, oder eines Salzes einer Verbindung der Formel (I), mit einem N-R-D-Glucamin, worin R Methyl ist, oder einem Salz davon; und

(b) das Überführen des resultierenden d-2-(6-Methoxy-2-naphthyl)propionsäure-Vorläufers in d-2-(6-Methoxy-naphthyl)propionsäure;

mit der Maßgabe, daß, wenn R und $R^1$ Methyl sind und X Brom ist und n 1 ist, die Auftrennung nicht in einem Toluol- und Methanollösungsmittel durchgeführt wird.

**28.** Verfahren zur Herstellung von (d)-2-(6-Methoxy-2-naphthyl)propionsäure oder pharmazeutisch annehmbaren Salzen davon, umfassend:

(a) das Auftrennen in optische Isomere einer Verbindung der Formel I

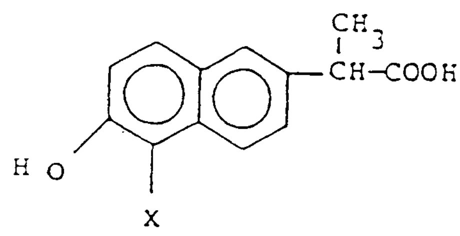

(I)

worin X Wasserstoff, Brom oder Chlor ist, oder eines Salzes einer Verbindung der Formel (I), mit einem N-R-D-Glucamin, worin R Methyl ist, oder einem Salz davon; und
(b) das Überführen des resultierenden d-2-(6-Methoxy-2-naphthyl)propionsäure-Vorläufers in d-2-(6-Methoxy-2-naphthyl)propionsäure.

**29.** Verfahren zur Herstellung von (d)-2-(6-Methoxy-2-naphthyl)propionsäure oder pharmazeutisch annehmbaren Salzen davon, umfassend:
(a) das Auftrennen in optische Isomere einer Verbindung der Formel I

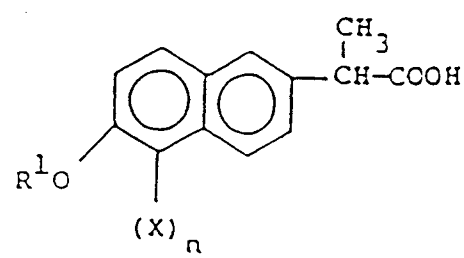

(I)

worin R Wasserstoff oder Methyl ist, wenn X Halogen, ausgewählt aus Brom oder Chlor, ist und n 1 ist, oder worin $R^1$ Wasserstoff ist, wenn n 0 ist, oder eines Salzes einer Verbindung der Formel (I), mit einem N-R-D-Glucamin, worin R Alkyl mit 2 bis 18 Kohlenstoffatomen oder Cycloalkyl mit 3 bis 8 Kohlenstoffatomen ist, oder einem Salz davon; und
(b) das Überführen des resultierenden d-2-(6-Methoxy-2-naphthyl)propionsäure-Vorläufers in d-2-(6-Methoxy-2-naphthyl)propionsäure.

**30.** Verfahren zur Herstellung von (d)-2-(6-Methoxy-2-naphthyl)propionsäure oder pharmazeutisch annehmbaren Salzen davon, umfassend:
(a) das Auftrennen in optische Isomere einer Verbindung der Formel I

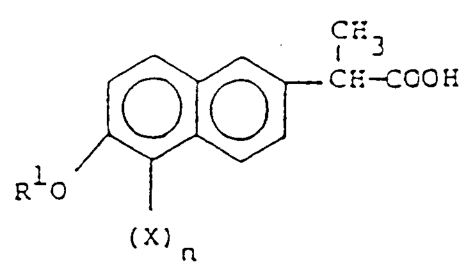

(I)

worin R Wasserstoff oder Methyl ist, wenn X Halogen, ausgewählt aus Brom oder Chlor, ist und n 1 ist, oder worin $R^1$ Wasserstoff ist, wenn n 0 ist, oder eines Salzes einer Verbindung der Formel (I), mit einem N-R-D-Glucamin, worin R Alkyl mit 6 bis 18 Kohlenstoffatomen oder Cycloalkyl mit 3 bis 8 Kohlenstoffatomen ist, oder einem Salz davon; und
(b) das Überführen des resultierenden d-2-(6-Methoxy-2-naphthyl)propionsäure-Vorläufers in d-2-(6-Methoxy-2-naphthyl)propionsäure.

**31.** Verfahren nach irgendeinem der Ansprüche 27 bis 30, worin d-2-(6-Hydroxy-2-naphthyl)propionsäure oder ein Salz davon in d-2-(6-Methoxy-2-naphthyl)propionsäure überführt wird.

**32.** Verfahren nach irgendeinem der Ansprüche 27 bis 30, worin d-2-(5-Halogen-6-methoxy-2-naphthyl)-propionsäure, d-2-(5-Halogen-6-hydroxy-2-naphthyl)propionsäure oder ein Salz davon in d-2-(6-Methoxy-2-naphthyl)propionsäure überführt wird.

**33.** Verfahren nach Anspruch 31 oder 32, worin d-2-(6-Methoxy-2-naphthyl)propionsäure in ein pharmazeutisch annehmbares Salz davon überführt wird.

**34.** Verfahren zur Auftrennung in optische Isomere eines (d)- und (l)-2-(6-Methoxy-2-naphthyl)propionsäure-Vorläufers der Formel I

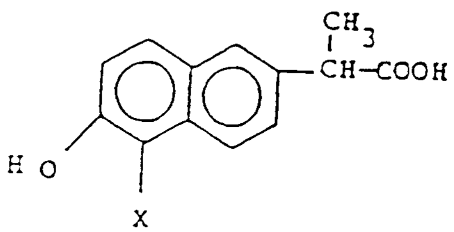

(I)

worin X Wasserstoff, Chlor oder Brom ist, oder von Salzen davon,
in die Enantiomere davon, dadurch gekennzeichnet, daß ein N-R-D-Glucamin oder Salz davon, worin R Alkyl mit 1 bis 18 Kohlenstoffatomen oder Cycloalkyl mit 3 bis 8 Kohlenstoffatomen ist, als das Auftrennungsmittel verwendet wird, um den (d)-Säurevorläufer zu isolieren, und daß der (l)-Säurevorläufer racemisiert wird, gefolgt von Auftrennung in optische Isomere.

**35.** Verfahren zur Herstellung von (d)-2-(6-Methoxy-2-naphthyl)propionsäure oder pharmazeutisch annehmbaren Salzen davon, umfassend:
(a) das Auftrennen in optische Isomere einer Vorläuferverbindung der Formel I

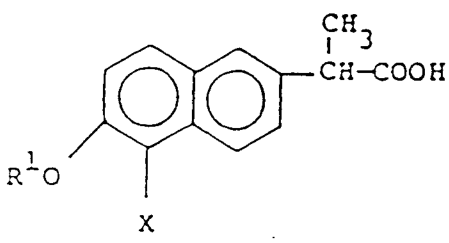

(I)

worin $R^1$ Wasserstoff oder Methyl ist, wenn X Brom oder Chlor ist; oder $R^1$ Wasserstoff ist, wenn X Wasserstoff ist; oder eines Salzes einer Verbindung der Formel I, mit einem N-R-D-Glucamin, worin R Alkyl mit 1 bis 18 Kohlenstoffatomen oder Cycloalkyl mit 3 bis 8 Kohlenstoffatomen ist, oder einem Salz des Glucamins, um die (d)-Form der Vorläuferverbindung zu isolieren;
(b) das Überführen der (d)-Form in (d)-2-(6-Methoxy-2-naphthyl)propionsäure oder ein pharmazeutisch annehmbares Salz davon; und
(c) das Racemisieren der (l)-Form der Vorläuferverbindung und das Auftrennen in optische Isomere der resultierenden racemischen Form der Vorläuferverbindung.

**36.** Verfahren zur Auftrennung in optische Isomere eines (d)- und (l)-2-(6-Methoxy-2-naphthyl)propionsäure-Vorläufers der Formel I

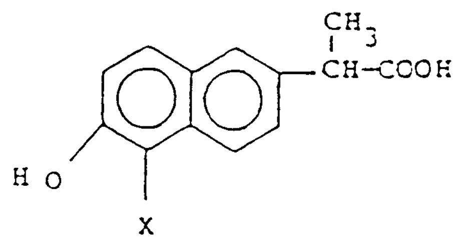

(I)

worin X Wasserstoff, Chlor oder Brom ist, oder von Salzen davon,
in die Enantiomere davon, dadurch gekennzeichnet, daß ein N-R-D-Glucamin oder Salz davon, worin R Alkyl mit 2 bis 8 Kohlenstoffatomen oder Cycloalkyl mit 3 bis 8 Kohlenstoffatomen ist, als das Auftrennungsmittel verwendet wird, um den (d)-Säurevorläufer zu isolieren, und daß der (l)-Säurevorläufer racemisiert wird, gefolgt von Auftrennung in optische Isomere.

**37.** Verfahren zur Herstellung von (d)-2-(6-Methoxy-2-naphthyl)propionsäure oder pharmazeutisch annehmbaren Salzen davon, umfassend:
(a) das Auftrennen in optische Isomere einer Vorläuferverbindung der Formel I

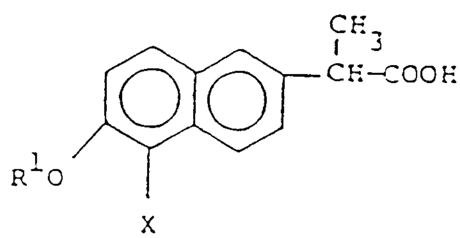

(I)

worin $R^1$ Wasserstoff oder Methyl ist, wenn x Brom oder Chlor ist; oder
$R^1$ Wasserstoff ist, wenn X Wasserstoff ist,
oder eines Salzes einer Verbindung der Formel I,
mit einem N-R-D-Glucamin, worin R Alkyl mit 2 bis 8 Kohlenstoffatomen oder Cycloalkyl mit 3 bis 8 Kohlenstoffatomen ist,
oder einem Salz des Glucamins,
um die (d)-Form der Vorläuferverbindung zu isolieren;
(b) das Überführen der (d)-Form in (d)-2-(6-Methoxy-2-naphthyl)propionsäure oder ein pharmazeutisch annehmbares Salz davon; und
(c) das Racemisieren der (l)-Form der Vorläuferverbindung und das Auftrennen in optische Isomere der resultierenden racemischen Form der Vorläuferverbindung.

**Patentansprüche für folgenden Vertragsstaat : CH**

**1.** N-R-D-Glucaminsalz einer d- oder l-Verbindung der Formel I

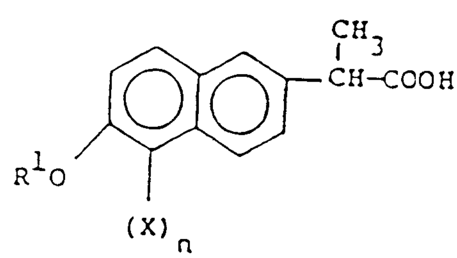

(I)

worin R Methyl ist und $R^1$ Wasserstoff oder Methyl ist, wenn X Halogen, ausgewählt aus Chlor und Brom, ist und n 1 ist; oder $R^1$ Wasserstoff ist, wenn n 0 ist.

2. N-R-D-Glucaminsalz einer d- oder l-Verbindung der Formel I

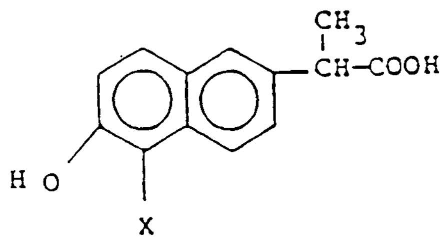

(I)

worin R Methyl ist und X Wasserstoff, Chlor oder Brom ist.

3. N-R-D-Glucaminsalz einer d- oder l-Verbindung der Formel I

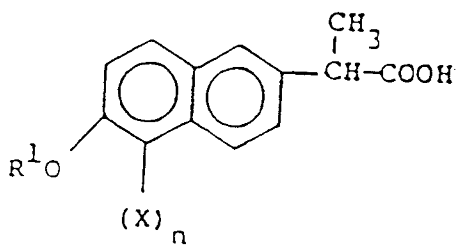

(I)

worin R Alkyl mit 2 bis 18 Kohlenstoffatomen oder Cycloalkyl mit 3 bis 8 Kohlenstoffatomen ist und $R^1$ Wasserstoff oder Methyl ist, wenn X Halogen, ausgewählt aus Chlor und Brom, ist und n 1 ist; oder $R^1$ Wasserstoff ist, wenn n 0 ist.

4. N-R-D-Glucaminsalz einer d- oder l-Verbindung der Formel I

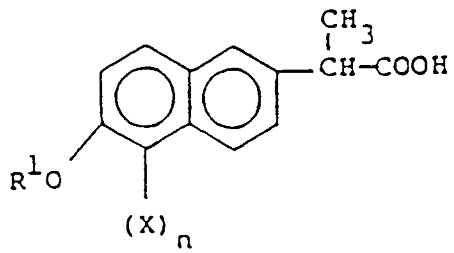

(I)

worin R Alkyl mit 6 bis 18 Kohlenstoffatomen oder Cycloalkyl mit 3 bis 8 Kohlenstoffatomen ist, und $R^1$ Wasserstoff oder Methyl ist, wenn X Halogen, ausgewählt aus Chlor und Brom, ist und n 1 ist; oder $R^1$ Wasserstoff ist, wenn n 0 ist.

5. N-R-D-Glucaminsalz nach Anspruch 1 oder Anspruch 2, worin R Methyl ist; und X Brom ist.

6. N-R-D-Glucaminsalz nach Anspruch 3 oder Anspruch 4, worin R n-Octyl ist.

7. Mischung der N-R-D-Glucaminsalze der (d,l)-Säuren der Formel I

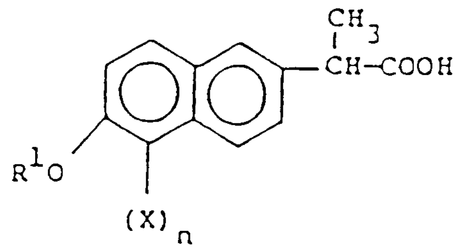

(I)

worin $R^1$ Wasserstoff oder Methyl ist, wenn X Halogen, ausgewählt aus Brom und Chlor, ist und n 1 ist; oder $R^1$ Wasserstoff ist, wenn n 0 ist,und R Methyl ist.

**8.** Mischung der N-R-D-Glucaminsalze der (d,l)-Säuren der Formel I

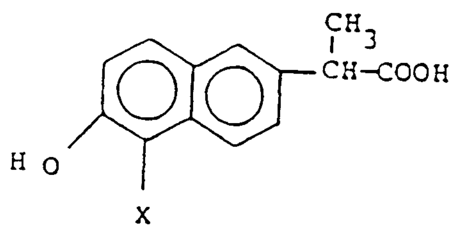

(I)

worin X Wasserstoff, Brom oder Chlor ist und R Methyl ist.

**9.** Mischung der N-R-D-Glucaminsalze der (d,l)-Säuren der Formel I

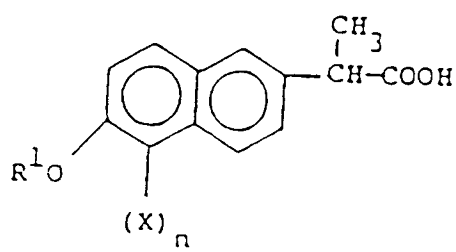

(I)

worin $R^1$ Wasserstoff oder Methyl ist, wenn X Halogen, ausgewählt aus Brom und Chlor, ist und n 1 ist; oder $R^1$ Wasserstoff ist, wenn n 0 ist, und R Alkyl mit 2 bis 18 Kohlenstoffatomen oder Cycloalkyl mit 3 bis 8 Kohlenstoffatomen ist.

**10.** Mischung der N-R-D-Glucaminsalze der (d,l)-Säuren der Formel I

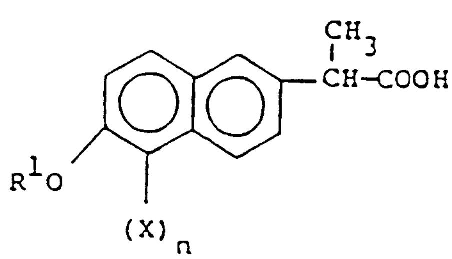

(I)

worin $R^1$ Wasserstoff oder Methyl ist, wenn X Halogen, ausgewählt aus Brom und Chlor, ist und n 1 ist; oder $R^1$ Wasserstoff ist, wenn n 0 ist, und R Alkyl mit 6 bis 18 Kohlenstoffatomen oder Cycloalkyl mit 3 bis 8 Kohlenstoffatomen ist.

**11.** Ein optische Isomere auftrennendes Medium, umfassend eine Mischung der N-R-D-Glucaminsalze der (d,l)-Säuren der Formel I

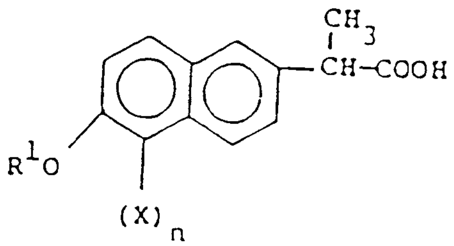

(I)

worin $R^1$ Wasserstoff oder Methyl ist, wenn X Halogen, ausgewählt aus Brom und Chlor, ist und n 1 ist, oder $R^1$ Wasserstoff ist, wenn n 0 ist, und R Methyl ist, und ein Lösungsmittel, in dem bei der Auftrennungstemperatur die Löslichkeit des entsprechenden d-2-(6-Methoxy-2-naphthyl)propionsäure-Vorläufersalzesbeträchtlich geringer ist als die Löslichkeit des anderen diastereomeren Salzes.

**12.** Ein optische Isomere auftrennendes Medium, umfassend eine Mischung der N-R-D-Glucaminsalze der (d,l)-Säuren der Formel I

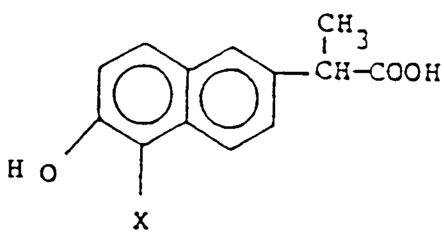

(I)

worin X Wasserstoff, Brom oder Chlor ist, und R Methyl ist, und ein Lösungsmittel, in dem bei der Auftrennungstemperatur die Löslichkeit des entsprechenden d-2-(6-Methoxy-2-naphthyl)propionsäure-Vorläufersalzesbeträchtlich geringer als die Löslichkeit des anderen diastereomeren Salzes ist.

**13.** Ein optische Isomere auftrennendes Medium umfassend eine Mischung der N-R-D-Glucaminsalze der (d,l)-Säuren der Formel I

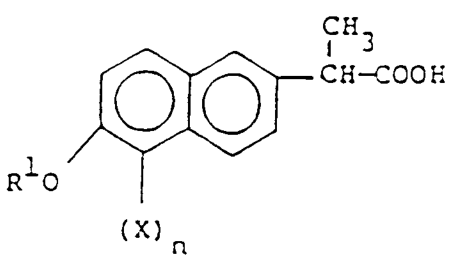

(I)

worin $R^1$ Wasserstoff oder Methyl ist, wenn X Halogen, ausgewählt aus Brom und Chlor, ist und n 1 ist, oder $R^1$ Wasserstoff ist, wenn n 0 ist, und R Alkyl mit 2 bis 18 Kohlenstoffatomen oder ein Cycloalkyl mit 3 bis 8 Kohlenstoffatomen ist, und ein Lösungsmittel, in dem bei der Auftrennungstemperatur die Löslichkeit des entsprechenden d-2-(6-Methoxy-2-naphthyl)propionsäure-Vorläufersalzesbeträchtlich geringer als die Löslichkeit des anderen diastereomeren Salzes ist.

**14.** Ein optische Isomere auftrennendes Medium, umfassend eine Mischung der N-R-D-Glucaminsalze der (d,l)-Säuren der Formel I

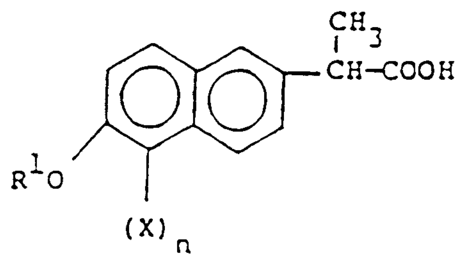

(I)

worin $R^1$ Wasserstoff oder Methyl ist, wenn X Halogen, ausgewählt aus Brom und Chlor, ist und n 1 ist, oder $R^1$ Wasserstoff ist, wenn n 0 ist, und R Alkyl mit 6 bis 18 Kohlenstoffatomen oder ein Cycloalkyl mit 3 bis 18 Kohlenstoffatomen ist, und ein Lösungsmittel, in dem bei der Auftrennungstemperatur die Löslichkeit des entsprechenden d-2-(6-Methoxy-2-naphthyl)propionsäure-Vorläufersalzesbeträchtlich geringer als die Löslichkeit des anderen diastereomeren Salzes ist.

**15.** d-Isomer einer Verbindung der Formel I

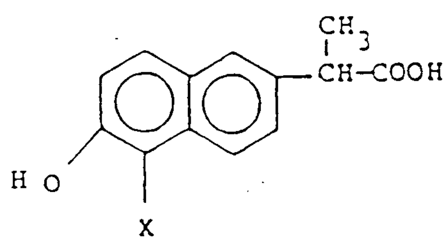

(I)

worin X Wasserstoff, Chlor oder Brom ist.

**16.** l-Isomer einer Verbindung der Formel I

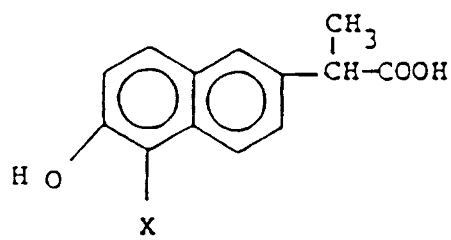

(I)

worin X Wasserstoff, Chlor oder Brom ist.

**17.** Verfahren zur Herstellung von (d)-2-(6-Methoxy-2-naphthyl)propionsäure, umfassend die Überführung eines N-R-D-Glucaminsalzes nach irgendeinem der Ansprüche 1 bis 6, worin das N-R-D-Glucaminsalz das des d-Isomers einer Verbindung der Formel I ist, in (d)-2-(6-Methoxy-2-naphthyl)propionsäure.

**18.** Verfahren nach Anspruch 17, worin (d)-2-(6-Methoxy-2-naphthyl)propionsäure in ein pharmazeutisch annehmbares Salz davon überführt wird.

**19.** Verfahren zur Auftrennung in optische Isomere von Mischungen von (d)- und (l)-2-(6-Methoxy-2-naphthyl)propionsäure-Vorläufern der Formel I

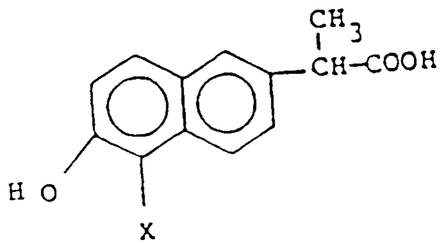

(I)

worin X Wasserstoff, Chlor oder Brom ist; oder von Salzen davon; in die Enantiomere davon, dadurch gekennzeichnet, daß N-R-D-Glucamine oder Salze davon als das Auftrennungsmittel, worin R Methyl ist, verwendet werden.

**20.** Verfahren zur Auftrennung in optische Isomere von Mischungen von (d)- und (l)-2-(6-Methoxy-2-naphthyl)propionsäure-Vorläufern der Formel I

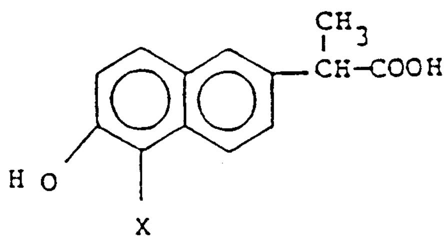

(I)

worin X Wasserstoff, Chlor oder Brom ist; oder von Salzen davon; in die Enantiomere davon, dadurch gekennzeichnet, daß N-R-D-Glucamine oder Salze davon als das Auftrennungsmittel, worin R Octyl ist, verwendet werden.

**21.** Verfahren zur Auftrennung in optische Isomere von Mischungen von (d)- und (l)-2-(6-Methoxy-2-naphthyl)propionsäure-Vorläufern der Formel I

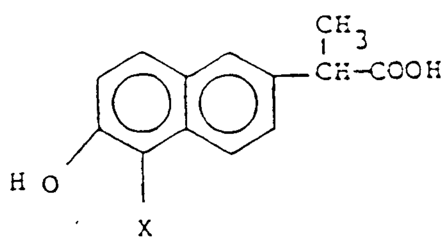

(I)

worin X Wasserstoff, Chlor oder Brom ist; oder von Salzen davon; in die Enantiomere davon, dadurch gekennzeichnet, daß N-R-D-Glucamine oder Salze davon als das Auftrennungsmittel, worin R Alkyl mit 2 bis 18 Kohlenstoffatomen oder Cycloalkyl mit 3 bis 8 Kohlenstoffatomen ist, verwendet werden.

**22.** Verfahren zur Auftrennung in optische Isomere von Mischungen von (d)- und (l)-2-(6-Methoxy-2-naphthyl)propionsäure-Vorläufern der Formel I

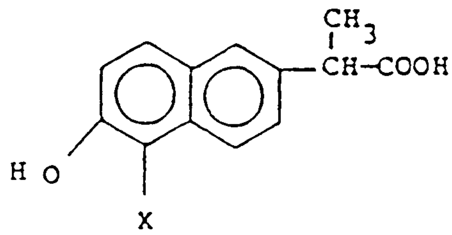

(I)

worin X Wasserstoff, Chlor oder Brom ist; oder von Salzen davon;
in die Enantiomere davon, dadurch gekennzeichnet, daß N-R-D-Glucamine oder Salze davon als das Auftrennungsmittel, worin R Alkyl mit 6 bis 18 Kohlenstoffatomen oder Cycloalkyl mit 3 bis 8 Kohlenstoffatomen ist, verwendet werden.

**23.** Verfahren nach irgendeinem der Ansprüche 19 bis 22, worin das resultierende Paar von (d)- und (l)-Säure-N-R-D-Glucaminsalzen durch fraktionierte Kristallisation aufgetrennt wird.

**24.** Verfahren zur Herstellung von (d)-2-(6-Methoxy-2-naphthyl)propionsäure oder pharmazeutisch annehmbaren Salzen davon, umfassend:
(a) das Auftrennen in optische Isomere eines Vorläufers der Formel I

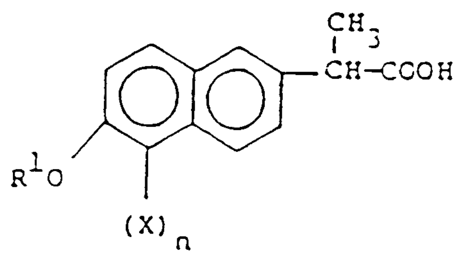

(I)

worin $R^1$ Wasserstoff oder Methyl ist, wenn X Halogen, ausgewählt aus Brom und Chlor, ist und n 1 ist, oder worin $R^1$ Wasserstoff ist, wenn n 0 ist, oder eines Salzes einer Verbindung der Formel (I), mit einem N-R-D-Glucamin, worin R Methyl ist, oder einem Salz davon; und
(b) das Überführen des resultierenden d-2-(6-Methoxy-2-naphthyl)propionsäure-Vorläufers in d-2-(6-Methoxy-naphthyl)propionsäure;
außer dem Verfahren, worin das Auftrennungsmittel N-Methyl-D-glucamin und der Vorläufer dl-2-(5-Halogen-6-methoxy-2-naphthyl)propionsäure in einem Lösungsmittelmedium, das ausgewählt ist aus Mischungen von Toluol und Methanol, in Gegenwart einer optisch inaktiven organischen oder anorganischen Base bei einer Temperatur zwischen Raumtemperatur und 65 °C umgesetzt werden, wobei das weniger lösliche N-Methyl-D-glucaminsalz des d-Isomers erhalten wird und mit einer starken Mineralsäure in Kontakt gebracht wird, um d-2-(5-Halogen-6-methoxy-2-naphthyl)propionsäure zu erhalten, die durch Behandlung mit einem geeigneten Hydriersystem, das in der Lage ist, das 5-Halogen vollständig durch Wasserstoff zu ersetzen, in einem alkalischen Medium bei einer Temperatur zwischen Raumtemperatur und 100 °C über einen Zeitraum zwischen 1 und 4 Stunden katalytisch dehalogeniert wird.

**25.** Verfahren zur Herstellung von (d)-2-(6-Methoxy-2-naphthyl)propionsäure oder pharmazeutisch annehmbaren Salzen davon, umfassend:
(a) das Auftrennen in optische Isomere einer Verbindung der Formel I

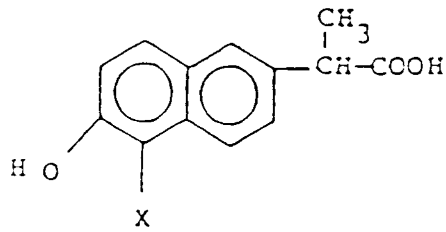

(I)

worin X Wasserstoff, Brom oder Chlor ist, oder eines Salzes einer Verbindung der Formel (I), mit einem N-R-D-Glucamin, worin R Methyl ist, oder einem Salz davon; und
(b) das Überführen des resultierenden d-2-(6-Methoxy-2-naphthyl)propionsäure-Vorläufers in d-2-(6-Methoxy-2-naphthyl)propionsäure.

**26.** Verfahren zur Herstellung von (d)-2-(6-Methoxy-2-naphthyl)propionsäure oder pharmazeutisch annehmbaren Salzen davon, umfassend:
(a) das Auftrennen in optische Isomere einer Verbindung der Formel I

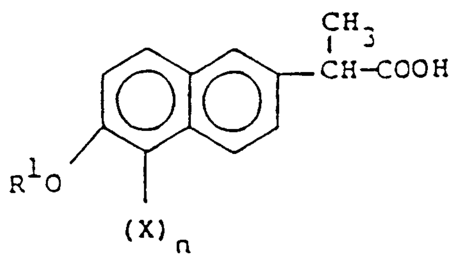

(I)

worin R Wasserstoff oder Methyl ist, wenn X Halogen, ausgewählt aus Brom oder Chlor, ist und n 1 ist, oder worin $R^1$ Wasserstoff ist, wenn n 0 ist, oder eines Salzes einer Verbindung der Formel (I), mit einem N-R-D-Glucamin, worin R Alkyl mit 2 bis 18 Kohlenstoffatomen oder Cycloalkyl mit 3 bis 8 Kohlenstoffatomen ist, oder einem Salz davon; und
(b) das Überführen des resultierenden d-2-(6-Methoxy-2-naphthyl)propionsäure-Vorläufers in d-2-(6-Methoxy-2-naphthyl)propionsäure.

**27.** Verfahren zur Herstellung von (d)-2-(6-Methoxy-2 naphthyl)propionsäure oder pharmazeutisch annehmbaren Salzen davon, umfassend:
(a) das Auftrennen in optische Isomere einer Verbindung der Formel I

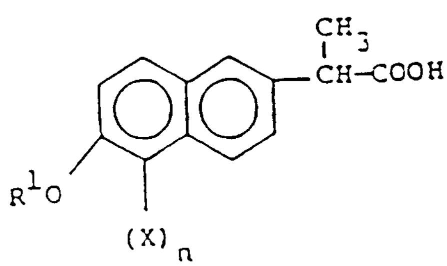

(I)

worin R Wasserstoff oder Methyl ist, wenn X Halogen, ausgewählt aus Brom oder Chlor, ist und n 1 ist, oder worin $R^1$ Wasserstoff ist, wenn n 0 ist, oder eines Salzes einer Verbindung der Formel (I), mit einem N-R-D-Glucamin, worin R Alkyl mit 6 bis 18 Kohlenstoffatomen oder Cycloalkyl mit 3 bis 8 Kohlenstoffatomen ist, oder einem Salz davon; und
(b) das Überführen des resultierenden d-2-(6-Methoxy-2-naphthyl)propionsäure-Vorläufers in d-2-(6-Methoxy-2-naphthyl)propionsäure.

**28.** Verfahren nach irgendeinem der Ansprüche 24 bis 27, worin d-2-(6-Hydroxy-2-naphthyl)propionsäure

oder ein Salz davon in d-2-(6-Methoxy-2-naphthyl)propionsäure überführt wird.

**29.** Verfahren nach irgendeinem der Ansprüche 24 bis 27, worin d-2-(5-Halogen-6-methoxy-2-naphthyl)-propionsäure, d-2-(5-Halogen-6-hydroxy-2-naphthyl)propionsäure oder ein Salz davon in d-2-(6-Methoxy-2-naphthyl)propionsäure überführt wird.

**30.** Verfahren nach Anspruch 28 oder 29, worin d-2-(6-Methoxy-2-naphthyl)propionsäure in ein pharmazeutisch annehmbares Salz davon überführt wird.

**31.** Verfahren zur Auftrennung in optische Isomere eines (d)- und (l)-2-(6-Methoxy-2-naphthyl)propionsäure-Vorläufers der Formel I

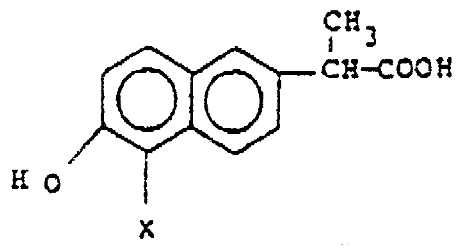

(I)

worin X Wasserstoff, Chlor oder Brom ist, oder von Salzen davon,
in die Enantiomere davon, dadurch gekennzeichnet, daß ein N-R-D-Glucamin oder Salz davon, worin R Alkyl mit 1 bis 18 Kohlenstoffatomen oder Cycloalkyl mit 3 bis 8 Kohlenstoffatomen ist, als das Auftrennungsmittel verwendet wird, um den (d)-Säurevorläufer zu isolieren, und daß der (l)-Säurevorläufer racemisiert wird, gefolgt von Auftrennung in optische Isomere.

**32.** Verfahren zur Herstellung von (d)-2-(6-Methoxy-2-naphthyl)propionsäure oder pharmazeutisch annehmbaren Salzen davon, umfassend:
(a) das Auftrennen in optische Isomere einer Vorläuferverbindung der Formel I

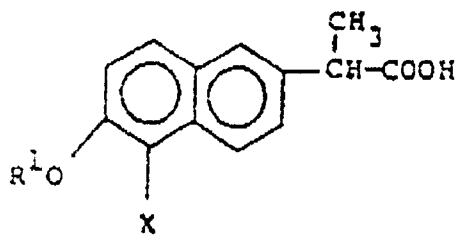

(I)

worin $R^1$ Wasserstoff oder Methyl ist, wenn X Brom oder Chlor ist; oder $R^1$ Wasserstoff ist, denn X Wasserstoff ist; oder eines Salzes einer Verbindung der Formel I, mit einem N-R-D-Glucamin, worin R Alkyl mit 1 bis 18 Kohlenstoffatomen oder Cycloalkyl mit 3 bis 8 Kohlenstoffatomen ist, oder einem Salz des Glucamins, um die (d)-Form der Vorläuferverbindung zu isolieren;
(b) das Überführen der (d)-Form in (d)-2-(6-Methoxy-2-naphthyl)propionsäure oder ein pharmazeutisch annehmbares Salz davon; und
(c) das Racemisieren der (l)-Form der Vorläuferverbindung und das Auftrennen in optische Isomere der resultierenden racemischen Form der Vorläuferverbindung.

**33.** Verfahren zur Auftrennung in optische Isomere eines (d)- und (l)-2-(6-Methoxy-2-naphthyl)propionsäure-Vorläufers der Formel I

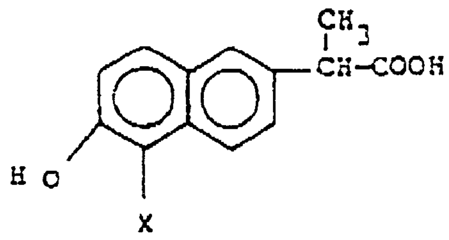

(I)

worin X Wasserstoff, Chlor oder Brom ist, oder von Salzen davon, in die Enantiomere davon, dadurch gekennzeichnet, daß ein N-R-D Glucamin oder Salz davon, worin R Alkyl mit 2 bis 8 Kohlenstoffatomen oder Cycloalkyl mit 3 bis 8 Kohlenstoffatomen ist, als das Auftrennungsmittel verwendet wird, um den (d)-Säurevorläufer zu isolieren, und daß der (l)-Säurevorläufer racemisiert wird, gefolgt von Auftrennung in optische Isomere.

**34.** Verfahren zur Herstellung von (d)-2-(6-Methoxy-2-naphthyl)propionsäure oder pharmazeutisch annehmbaren Salzen davon, umfassend:
(a) das Auftrennen in optische Isomere einer Vorläuferverbindung der Formel I

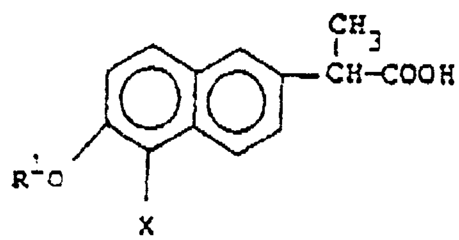

(I)

worin $R^1$ Wasserstoff oder Methyl ist, wenn X Brom oder Chlor ist; oder
$R^1$ Wasserstoff ist, wenn X Wasserstoff ist,
oder eines Salzes einer Verbindung der Formel I,
mit einem N-R-D-Glucamin, worin R Alkyl mit 2 bis 8 Kohlenstoffatomen oder Cycloalkyl mit 3 bis 8 Kohlenstoffatomen ist,
oder einem Salz des Glucamins,
um die (d)-Form der Vorläuferverbindung zu isolieren;
(b) das Überführen der (d)-Form in (d)-2-(6-Methoxy-2-naphthyl)propionsäure oder ein pharmazeutisch annehmbares Salz davon; und
(c) das Racemisieren der (l)-Form der Vorläuferverbindung und das Auftrennen in optische Isomere der resultierenden racemischen Form der Vorläuferverbindung.

**Revendications**
**Revendications pour les Etats contractants suivants : BE, DE, SE, NL, GB, FR, IT, LU**

**1.** Sel de N-R-D-glucamine d'un composé d ou l de formule I

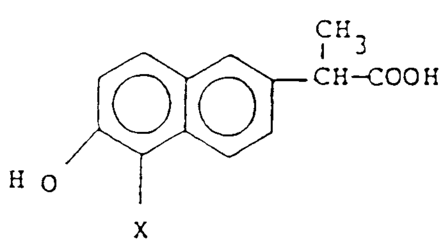

(I)

où R représente un groupe méthyle et X représente l'hydrogène, un groupe chloro ou bromo.

**2.** Sel de N-R-D-glucamine d'un composé d ou l de formule I

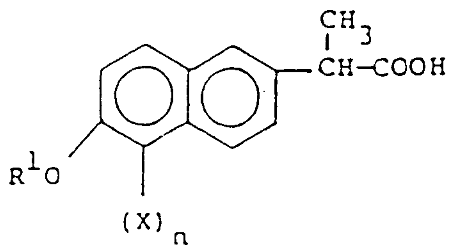

(I)

où R représente un groupe alkyle ayant 2 à 18 atomes de carbone, ou cycloalkyle ayant 3 à 8 atomes de carbone, et $R^1$ représente l'hydrogène ou un groupe méthyle lorsque X représente un halogène choisi entre le chlore et le brome et n est égal à 1 ; ou bien $R^1$ représente l'hydrogène lorsque n est égal à 0.

3. Sel de N-R-D-glucamine d'un composé d ou l de formule I

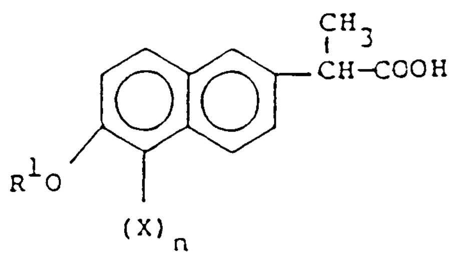

(I)

où R représente un groupe alkyle ayant 6 à 18 atomes de carbone ou cycloalkyle ayant 3 à 8 atomes de carbone, et $R^1$ représente l'hydrogène ou un groupe méthyle lorsque X représente un halogène choisi entre le chlore et le brome et n est égal à 1 ; ou bien $R^1$ représente l'hydrogène lorsque n est égal à 0.

4. Sel de N-R-D-glucamine suivant la revendication 1, dans lequel R représente un groupe méthyle et X représente un groupe bromo.

5. Sel de N-R-D-glucamine suivant la revendication 2 ou la revendication 3, dans lequel R représente un groupe n-octyle.

6. Mélange de sels de N-R-D-glucamine des acides (d,l) de formule I

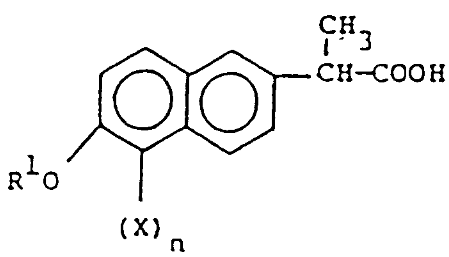

(I)

où $R^1$ représente l'hydrogène ou un groupe méthyle lorsque X représente un halogène choisi entre le brome et le chlore et n est égal à 1; ou bien $R^1$ représente l'hydrogène lorsque n est égal à 0, et R représente un groupe méthyle ; sous réserve que, lorsque $R^1$ représente un groupe méthyle et X représente un halogène, le mélange soit autre qu'un mélange dans un solvant consistant en un mélange de toluène et de méthanol.

7. Mélange de sels de N-R-D-glucamine des acides (d,l) de formule I

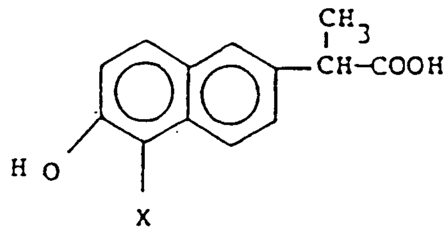

(I)

où X représente l'hydrogène, un groupe bromo ou chloro et R représente un groupe méthyle.

**8.** Mélange des sels de N-R-D-glucamine des acides (d,l) de formule I

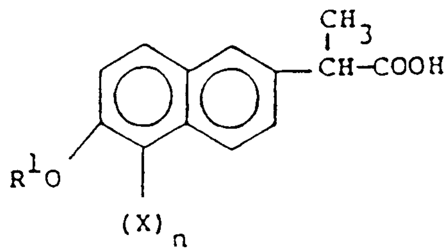

(I)

où $R^1$ représente l'hydrogène ou un groupe méthyle lorsque X représente un halogène choisi entre le brome et le chlore et n est égal à 1 ; ou bien $R^1$ représente l'hydrogène lorsque n est égal à 0, et R représente un groupe alkyle ayant 2 à 18 atomes de carbone ou cycloalkyle ayant 3 à 8 atomes de carbone.

**9.** Mélange des sels de N-R-D-glucamine des acides (d,l) de formule I

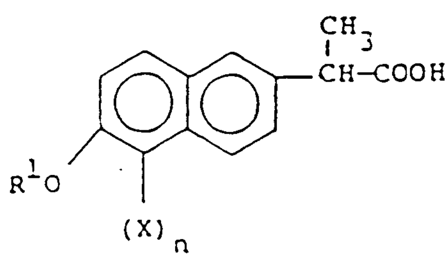

(I)

où $R^1$ représente l'hydrogène ou un groupe méthyle lorsque X représente un halogène choisi entre le brome et le chlore et n est égal à 1 ; ou bien $R^1$ représente l'hydrogène lorsque n est égal à 0, et R représente un groupe alkyle ayant 6 à 18 atomes de carbone ou cycloalkyle ayant 3 à 8 atomes de carbone.

**10.** Milieu de résolution comprenant un mélange des sels de N-R-D-glucamine des acides (d,l) de formule I

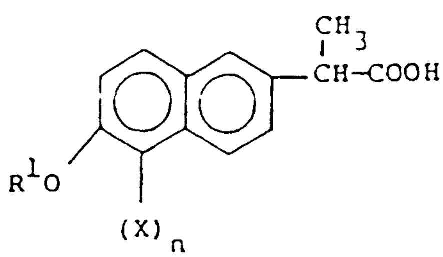

(I)

où $R^1$ représente l'hydrogène ou un groupe méthyle lorsque X représente un halogène choisi entre le

brome et le chlore et n est égal à 1 ; ou bien $R^1$ représente l'hydrogène lorsque n est égal à 0, et R représente un groupe méthyle, et un solvant dans lequel la solubilité du sel précurseur de l'acide d-2-(6-méthoxy-2-naphtyl)propionique correspondant est considérablement inférieure à la solubilité de l'autre sel diastéréo-isomère à la température de résolution ; sous réserve que, lorsque $R^1$ représente un groupe méthyle et X représente un halogène, le mélange soit alors autre qu'un mélange dans un solvant consistant en un mélange de toluène et de méthanol.

**11.** Milieu de résolution comprenant un mélange des sels de N-R-D-glucamine des acides (d,l) de formule I

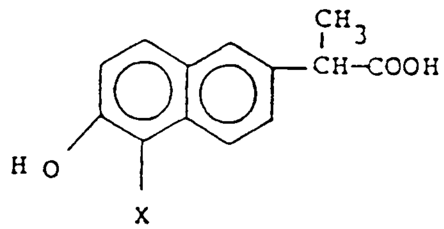

(I)

où X représente l'hydrogène, un groupe bromo ou chloro, et R représente un groupe méthyle, et un solvant dans lequel la solubilité du sel précurseur de l'acide d-2-(6-méthoxy-2-naphtyl)propionique est considérablement inférieure à la solubilité de l'autre sel diastéréo-isomère à la température de résolution.

**12.** Milieu de résolution comprenant un mélange des sels de N-R-D-glucamine des acides (d,l) de formule I

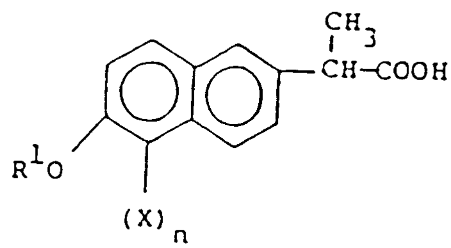

(I)

où $R^1$ représente l'hydrogène ou un groupe méthyle lorsque X représente un halogène choisi entre le brome et le chlore et n est égal à 1, ou bien $R^1$ représente l'hydrogène lorsque n est égal à 0, et R représente un groupe alkyle ayant 2 à 18 atomes de carbone ou un groupe cycloalkyle ayant 3 à 8 atomes de carbone, et un solvant dans lequel la solubilité du sel précurseur d'acide d-2-(6-méthoxy-2-naphtyl)propionique correspondant est considérablement inférieure à la solubilité de l'autre sel diastéréo-isomère à la température de résolution.

**13.** Milieu de résolution comprenant un mélange des sels de N-R-D-glucamine des acides (d,l) de formule I

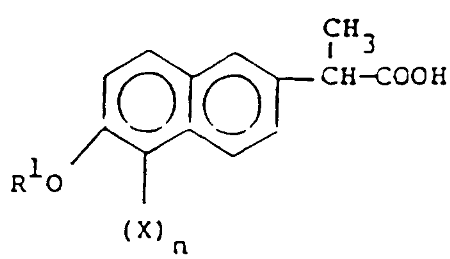

(I)

où $R^1$ représente l'hydrogène ou un groupe méthyle lorsque X représente un halogène choisi entre le brome et le chlore et n est égal à 1, ou bien $R^1$ représente l'hydrogène lorsque n est égal à 0, et R représente un groupe alkyle ayant 6 à 18 atomes de carbone ou un groupe cycloalkyle ayant 3 à 8 atomes de carbone, et un solvant dans lequel la solubilité du sel précurseur d'acide d-2-(6-méthoxy-2-naphtyl)propionique correspondant est considérablement inférieure à la solubilité de l'autre sel diastéréo-isomère à la température de résolution.

**14.** Isomère d d'un composé de formule I

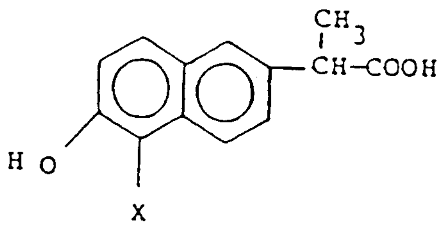

(I)

où X représente l'hydrogène, un groupe chloro ou bromo.

**15.** Isomère l d'un composé de formule I

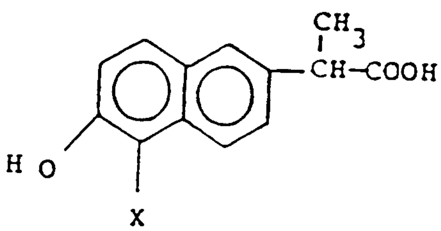

(I)

où X représente l'hydrogène, un groupe chloro ou bromo.

**16.** Procédé de production d'acide (d)-2-(6-méthoxy-2-naphtyl)propionique, qui consiste à transformer un sel de N-R-D-glucamine suivant l'une quelconque des revendications 1 à 5, dans lequel le sel de N-R-D-glucamine est celui de l'isomère d d'un composé de formule I, en acide (d)-2-(6-méthoxy-2-naphtyl)-propionique.

**17.** Procédé suivant la revendication 16, dans lequel l'acide (d)-2-(6-méthoxy-2-naphtyl)propionique est transformé en un de ses sels pharmaceutiquement acceptables.

**18.** Procédé de résolution de mélanges de précurseurs d'acides (d)- et (l)-2-(6-méthoxy-2-naphtyl)-propioniques de formule I

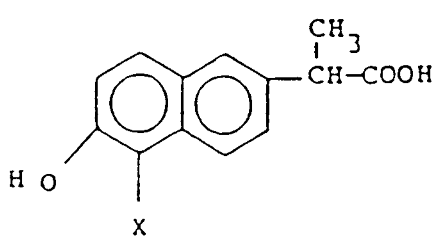

(I)

où X représente l'hydrogène, un groupe chloro ou bromo ; ou de leurs sels ;
en leurs énantiomères, caractérisé en ce que des N-R-D-glucamines ou leurs sels sont utilisés comme agent de résolution, R représentant un groupe méthyle.

**19.** Procédé de résolution de mélanges de précurseurs d'acides (d)- et (l)-2-(6-méthoxy-2-naphtyl)-propioniques de formule I

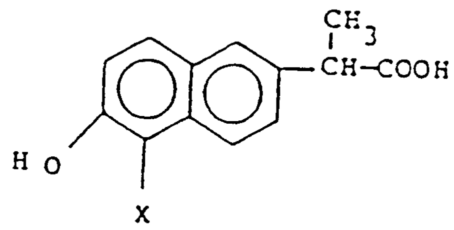

(I)

où X représente l'hydrogène, un groupe chloro ou bromo ; ou de leurs sels ;
en leurs énantiomères, caractérisé en ce que des N-R-D-glucamines ou leurs sels sont utilisés comme agent de résolution, R représentant un groupe octyle.

**20.** Procédé de résolution de mélanges de précurseurs d'acides (d)- et (l)-2-(6-méthoxy-2-naphtyl)-propioniques de formule I

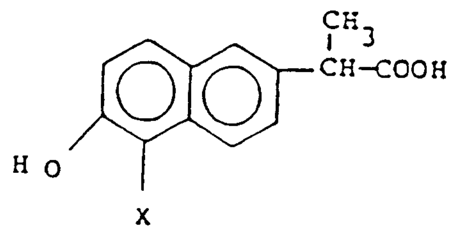

(I)

où X représente l'hydrogène, un groupe chloro ou bromo ; ou de leurs sels ;
en leurs énantiomères, caractérisé en ce que des N-R-D-glucamines ou leurs sels sont utilisés comme agent de résolution, R représentant un groupe alkyle ayant 2 à 18 atomes de carbone ou cycloalkyle ayant 3 à 8 atomes de carbone.

**21.** Procédé de résolution de mélanges de précurseurs d'acides (d)- et (l)-2-(6-méthoxy-2-naphtyl)-propioniques de formule I

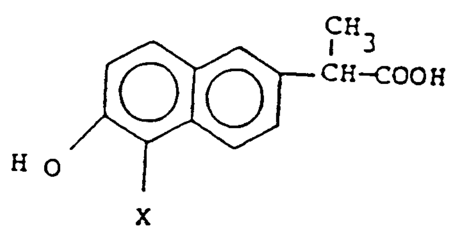

(I)

où X représente l'hydrogène, un groupe chloro ou bromo ; ou de leurs sels ;
en leurs énantiomères, caractérisé en ce que des N-R-D-glucamines ou leurs sels sont utilisés comme agent de résolution, R représentant un groupe alkyle ayant 6 à 18 atomes de carbone ou cycloalkyle ayant 3 à 8 atomes de carbone.

**22.** Procédé suivant l'une quelconque des revendications 18 à 21, dans lequel la paire résultante de sels de N-R-D-glucamine d'acides (d) et (l) est soumise à une séparation par cristallisation fractionnée.

**23.** Procédé de préparation d'acide (d)-2-(6-méthoxy-2-naphtyl)propioniqueou de ses sels pharmaceutiquement acceptables, qui consiste :

(a) à soumettre à une résolution un précurseur de formule I

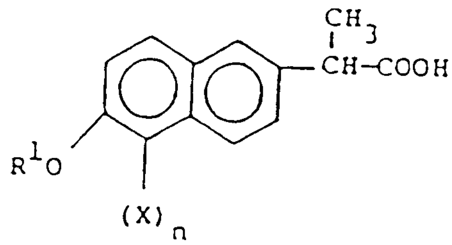

(I)

où $R^1$ représente l'hydrogène ou un groupe méthyle lorsque X représente un halogène choisi entre le brome et le chlore et n est égal à 1, ou dans laquelle $R^1$ représente l'hydrogène lorsque n est égal à 0, ou un sel d'un composé de formule (I) , avec une N-R-D-glucamine dans laquelle R représente un groupe méthyle ou un de ses sels ; sous réserve que, lorsque l'agent de résolution est la N-méthyl-D-glucamine et ledit précurseur est l'acide dl-2-(5-halogéno-6-méthoxy-2-naphtyl)-propionique, qui sont amenés à réagir l'un avec l'autre dans un mélange de solvants duquel le d-2-(5-halogéno-6-méthoxy-2-naphtyl)propionate de N-méthyl-D-glucamine est séparé, ledit mélange de solvants soit alors différent d'un mélange de toluène et de méthanol ; et

(b) à transformer le précurseur d'acide d-2-(6-méthoxy-2-naphtyl)propionique résultant en acide d-2-(6-méthoxy-2-naphtyl)propionique.

**24.** Procédé de préparation d'acide (d)-2-(6-méthoxy-2-naphtyl)propioniqueou de ses sels pharmaceutiquement acceptables, qui consiste :

(a) à soumettre à une résolution un composé de formule I

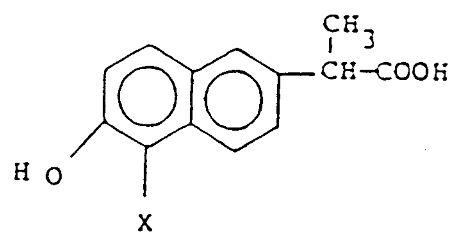

(I)

où X représente l'hydrogène, un groupe bromo ou chloro, ou un sel d'un composé de formule (I), avec une N-R-D-glucamine dans laquelle R représente un groupe méthyle, ou un de ses sels ; et

(b) à transformer le précurseur d'acide d-2-(6-méthoxy-2-naphtyl)propionique résultant en acide d-2-(6-méthoxy-2-naphtyl)propionique.

**25.** Procédé de préparation d'acide (d)-2-(6-méthoxy-2-naphtyl)propioniqueou de ses sels pharmaceutiquement acceptables, qui consiste :

(a) à soumettre à une résolution un composé de formule (I)

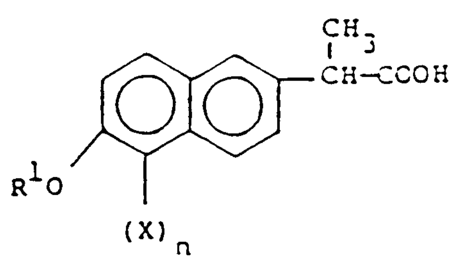

(I)

où R représente l'hydrogène ou un groupe méthyle lorsque X représente un halogène choisi entre le brome et le chlore et n est égal à l, ou bien dans laquelle $R^1$ représente l'hydrogène lorsque n est égal à 0, ou un sel d'un composé de formule (I) , avec une N-R-D-glucamine dans laquelle R représente un groupe alkyle ayant 2 à 18 atomes de carbone ou cycloalkyle ayant 3 à 8 atomes de carbone ou un de ses sels ; et

(b) à transformer le précurseur d'acide d-2-(6-méthoxy-2-naphtyl)propionique résultant en acide d-2-(6-méthoxy-2-naphtyl)propionique.

**26.** Procédé de préparation d'acide (d)-2-(6-méthoxy-2-naphtyl)propioniqueou de ses sels pharmaceutiquement acceptables, qui consiste :

(a) à soumettre à une résolution un composé de formule I

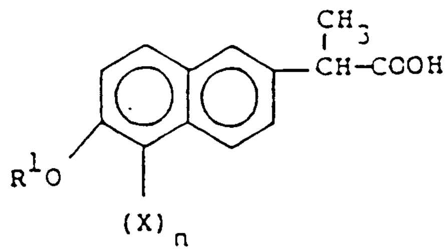

(I)

où R représente l'hydrogène ou un groupe méthyle lorsque X représente un halogène choisi entre le brome et le chlore et n est égal à 1, ou bien dans laquelle $R^1$ représente l'hydrogène lorsque n est égal à 0, ou un sel d'un composé de formule (I), avec une N-R-D-glucamine dans laquelle R représente un groupe alkyle ayant 6 à 18 atomes de carbone ou cycloalkyle ayant 3 à 8 atomes de carbone ou un de ses sels ; et

(b) a transformer le précurseur d'acide d-2-(6-méthoxy-2-naphtyl)propionique résultant en acide d-2-(6-méthoxy-2-naphtyl)propionique.

**27.** Procédé suivant l'une quelconque des revendications 23 à 26, dans lequel l'acide d-2-(6-hydroxy-2-naphtyl)propionique ou un de ses sels est transformé en un acide d-2-(6-méthoxy-2-naphtyl)-propionique.

**28.** Procédé suivant l'une quelconque des revendications 23 à 26, dans lequel un acide d-2-(5-halogéno-6-méthoxy-2-naphtyl)propionique, un acide d-2-(5-halogéno-6-hydroxy-2-naphtyl)propionique ou un de leurs sels est transformé en acide d-2-(6-méthoxy-2-naphtyl)propionique.

**29.** Procédé suivant la revendication 27 ou 28, dans lequel l'acide d-2-(6-méthoxy-2-naphtyl)propionique est transformé en un de ses sels pharmaceutiquement acceptables.

**30.** Procédé de résolution d'un précurseur d'acides (d)- et (l)-2-(6-méthoxy-2-naphtyl)propioniques de formule I

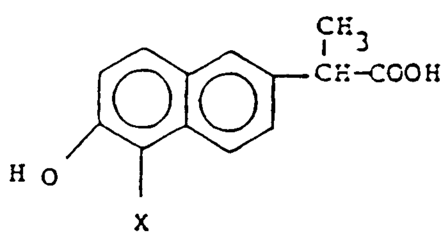

(I)

où X représente l'hydrogène, un groupe chloro ou bromo, ou d'un de ses sels,

en ses énantiomères, caractérisé en ce qu'une N-R-D-glucamine ou un de ses sels, dans lequel R représente un groupe alkyle ayant 1 à 18 atomes de carbone ou cycloalkyle ayant 3 à 8 atomes de carbone, est utilisé comme agent de résolution pour isoler ledit précurseur d'acide (d), et en ce que ledit précurseur d'acide (l) est racémisé, puis soumis à une résolution.

**31.** Procédé de préparation d'acide (d)-2-(6-méthoxy-2-naphtyl)propioniqueou de ses sels pharmaceutiquement acceptables, qui consiste :

(a) à soumettre à une résolution un composé précurseur de formule I

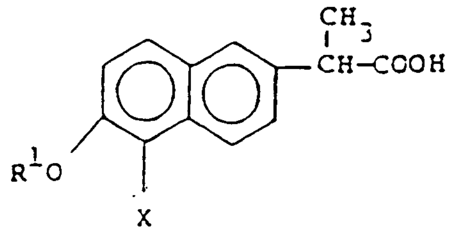

(I)

où $R^1$ représente l'hydrogène ou un groupe méthyle lorsque X représente un groupe bromo ou chloro ; ou bien $R^1$ représente l'hydrogène lorsque X représente l'hydrogène ; ou un sel d'un composé de formule I, avec une N-R-D-glucamine dans laquelle R représente un groupe alkyle ayant 1 à 18 atomes de carbone ou cycloalkyle ayant 3 à 8 atomes de carbone, ou un sel de ladite glucamine, pour isoler la forme (d) dudit composé précurseur ;

(b) à transformer ladite forme (d) en acide (d)-2-(6-méthoxy-2-naphtyl)propionique ou un de ses sels pharmaceutiquement acceptables ; et

(c) à racémiser la forme (l) dudit composé précurseur et à soumettre à une résolution la forme racémique résultante dudit composé précurseur.

**32.** Procédé de résolution d'un précurseur d'acides (d)- et (l)-2-(6-méthoxy-2-naphtyl)propioniques de formule I

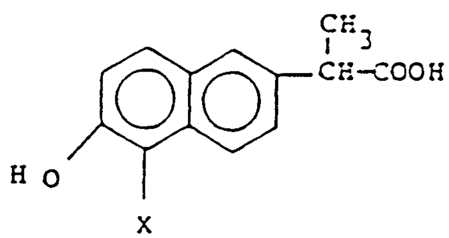

(I)

où X représente l'hydrogène, un groupe chloro ou bromo, ou de ses sels,
en ses énantiomères, caractérisé en ce qu'une N-R-D-glucamine ou un de ses sels, dans lequel R représente un groupe alkyle ayant 2 à 8 atomes de carbone ou cycloalkyle ayant 3 à 8 atomes de carbone, est utilisé comme agent de résolution pour isoler ledit précurseur d'acide (d), et en ce que ledit précurseur d'acide (l) est racémisé, puis est soumis à une résolution.

**33.** Procédé de préparation d'acide (d)-2-(6-méthoxy-2-naphtyl)propioniqueou de ses sels pharmaceutiquement acceptables, qui consiste:

(a) à soumettre à une résolution un composé précurseur de formule I

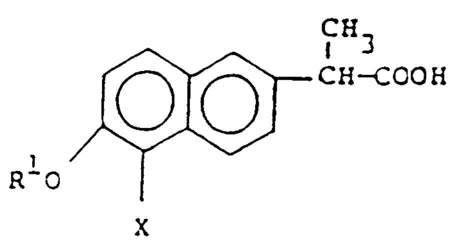

(I)

où $R^1$ représente l'hydrogène ou un groupe méthyle lorsque X représente un groupe bromo ou chloro ; ou
$R^1$ représente l'hydrogène lorsque X représente l'hydrogène,
ou un sel d'un compose de formule I,
avec une N-R-D-glucamine dans laquelle R représente un groupe alkyle ayant 2 à 8 atomes de carbone ou cycloalkyle ayant 3 à 8 atomes de carbone,
ou un sel de ladite glucamine,
pour isoler la forme (d) dudit composé précurseur ;

(b) à transformer ladite forme (d) en acide (d)-2-(6-méthoxy-2-naphtyl)propionique ou un de ses sels pharmaceutiquement acceptables ; et

(c) à racémiser la forme (l) dudit composé précurseur et à soumettre à une résolution la forme racémique résultante dudit composé précurseur.

**Revendications pour l'Etat contractant suivant : AT**

1. Procédé de séparation des isomères d et l de formule I

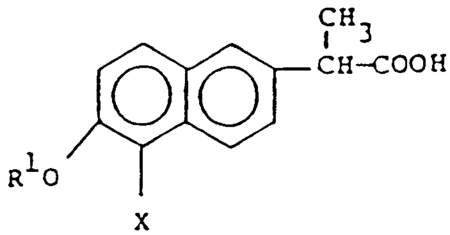

(I)

où $R^1$ représente un groupe méthyle et X représente un groupe bromo ;
procédé qui est caractérisé en ce qu'il consiste à effectuer la résolution de sels diastéréo-isomères de N-R-D-glucamine, dans laquelle R représente un groupe méthyle, et d'un mélange d'isomères d et l de formule I.

2. Procédé qui consiste à effectuer la résolution des sels diastéréo-isomères de N-R-D-glucamine et d'un mélange d'isomères d et l de formule I

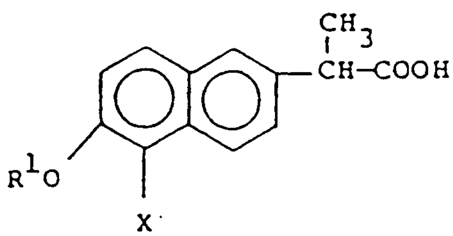

(I)

où R représente un groupe méthyle, $R^1$ représente un groupe méthyle et X représente un groupe chloro.

3. Procédé qui consiste à effectuer la résolution de sels diastéréo-isomères de N-R-D-glucamine et d'un mélange d'isomères d et l de formule I

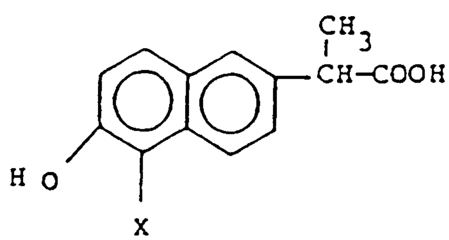

(I)

où R représente un groupe méthyle et X représente l'hydrogène, un groupe chloro ou bromo.

4. Procédé qui consiste à effectuer la résolution de sels diastéréo-isomères de N-R-D-glucamine et d'un mélange d'isomères d et l de formule I

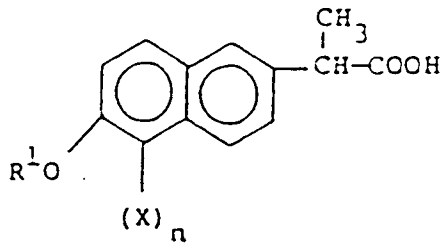

(I)

où R représente un groupe alkyle ayant 2 à 18 atomes de carbone, ou cycloalkyle ayant 3 à 8 atomes de carbone et $R^1$ représente l'hydrogène ou un groupe méthyle lorsque X représente un halogène choisi entre le chlore et le brome et n est égal à 1 ; ou bien $R^1$ représente l'hydrogène lorsque n est égal à 0.

**5.** Procédé qui consiste à effectuer la résolution de sels diastéréo-isomères de N-R-D-glucamine et d'un mélange d'isomères d et l de formule I

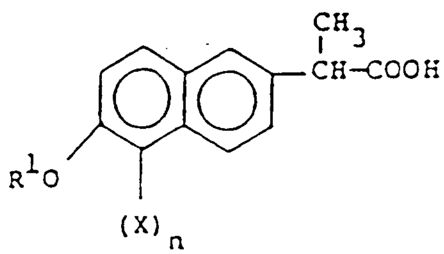

(I)

où R représente un groupe alkyle ayant 6 à 18 atomes de carbone ou cycloalkyle ayant 3 à 8 atomes de carbone, et $R^1$ représente l'hydrogène ou un groupe méthyle lorsque X représente un halogène choisi entre le chlore et le brome et n est égal à 1 ; ou bien $R^1$ représente l'hydrogène lorsque n est égal à 0.

**6.** Procédé suivant la revendication 3, dans lequel X représente un groupe bromo.

**7.** Procédé suivant la revendication 5, dans lequel R représente un groupe n-octyle.

**8.** Procédé de séparation des isomères d et l de formule I

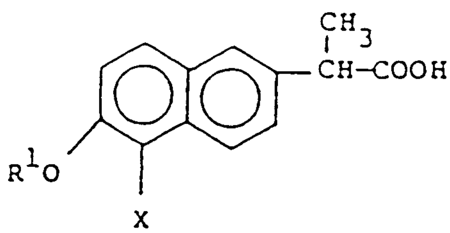

(I)

où $R^1$ représente un groupe méthyle et X représente un groupe bromo ; procédé caractérisé en ce qu'il consiste à faire réagir de la N-R-D-glucamine, dans laquelle R représente un groupe méthyle, avec un mélange d'isomères d et l de formule I.

**9.** Procédé qui consiste à faire réagir la N-R-D-glucamine avec un mélange d'isomères d et l de formule I

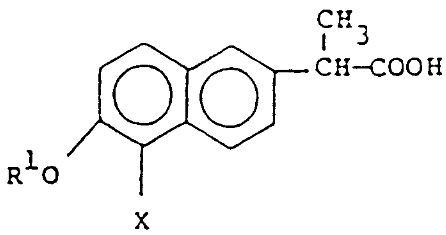

(I)

où $R^1$ représente un groupe méthyle, X représente un groupe chloro et R représente un groupe méthyle.

**10.** Procédé qui consiste à faire réagir la N-R-D-glucamine avec un mélange d'isomères d et l de formule I

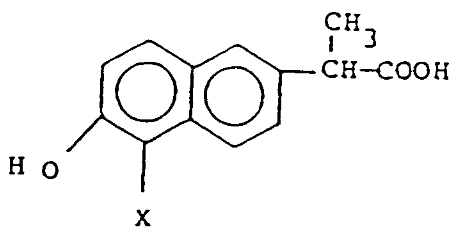

(I)

où X représente l'hydrogène, un groupe bromo ou chloro et R représente un groupe méthyle.

**11.** Procédé qui consiste à faire réagir la N-R-D-glucamine avec un mélange d'isomères d et l de formule I

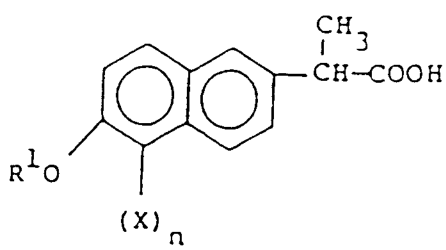

(I)

où $R^1$ représente l'hydrogène ou un groupe méthyle lorsque X représente un halogène choisi entre le brome et le chlore et n est égal à 1 : ou bien $R^1$ représente l'hydrogène lorsque n est égal à 0, et R représente un groupe alkyle ayant 2 à 18 atomes de carbone ou cycloalkyle ayant 3 à 8 atomes de carbone.

**12.** Procédé qui consiste à faire réagir la N-R-D-glucamine avec un mélange d'isomères d et l de formule I

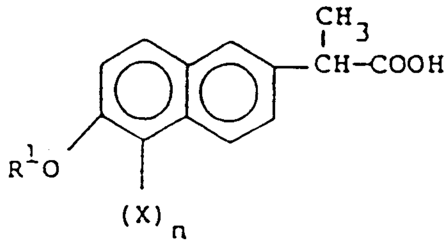

(I)

où $R^1$ représente l'hydrogène ou un groupe méthyle lorsque X représente un halogène choisi entre le brome et le chlore et n est égal à 1 ; ou bien $R^1$ représente l'hydrogène lorsque n est égal à 0, et R représente un groupe alkyle ayant 6 à 18 atomes de carbone ou cycloalkyle ayant 3 à 8 atomes de carbone.

**13.** Procédé de séparation des isomères d et l de formule I

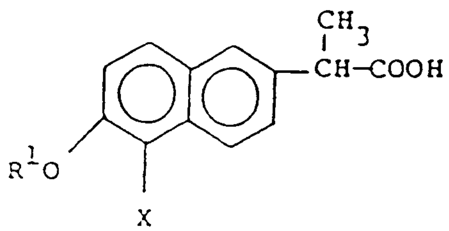

(I)

où R[1] représente un groupe méthyle lorsque X représente un groupe bromo, procédé caractérisé en ce qu'il consiste à préparer un milieu de résolution comprenant un mélange de sels diastéréo-isomères de N-R-D-glucamine, R représentant un groupe méthyle, et des isomères d et l de formule I, et un solvant dans lequel la solubilité du sel précurseur d'acide d-2-(6-méthoxy-2-naphtyl)propionique correspondant est considérablement inférieure à la solubilité de l'autre sel diastéréo-isomère à la température de résolution.

**14.** Procédé qui consiste à préparer un milieu de résolution comprenant un mélange des sels de N-R-D-glucamine des acides (d,l) de formule I

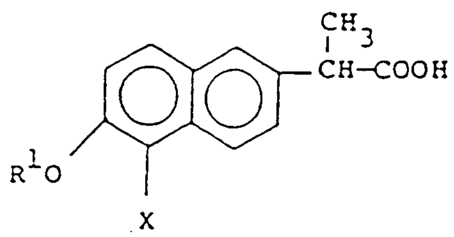

(I)

où R[1] représente un groupe méthyle, X représente un groupe chloro et R représente un groupe méthyle, et un solvant dans lequel la solubilité du sel précurseur d'acide d-2-(6-méthoxy-2-naphtyl)-propionique est considérablement inférieure à la solubilité de l'autre sel diastéréo-isomère à la température de résolution.

**15.** Procédé qui consiste à préparer un mélange de résolution comprenant un mélange des sels de N-R-D-glucamine des acides (d,l) de formule I

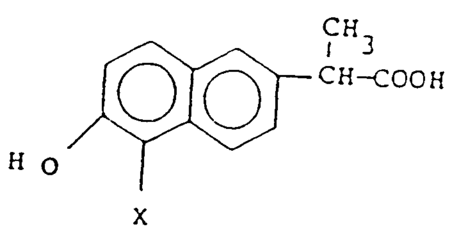

(I)

où X représente l'hydrogène, un groupe bromo ou chloro, et R représente un groupe méthyle, et un solvant dans lequel la solubilité du sel précurseur d'acide d-2-(6-méthoxy-2-naphtyl)propionique correspondant est considérablement inférieure à la solubilité de l'autre sel diastéréo-isomère à la température de résolution.

**16.** Procédé qui consiste à préparer un milieu de résolution comprenant un mélange des sels de N-R-D-glucamine des acides (d,l) de formule I

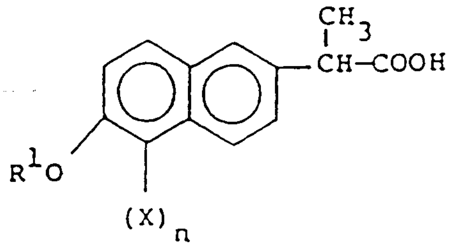

(I)

où $R^1$ représente l'hydrogène ou un groupe méthyle lorsque X représente un halogène choisi entre le brome et le chlore et n est égal à 1, ou bien $R^1$ représente l'hydrogène lorsque n est égal à 0, et R représente un groupe alkyle ayant 2 à 18 atomes de carbone ou cycloalkyle ayant 3 à 8 atomes de carbone, et un solvant dans lequel la solubilité du sel précurseur d'acide d-2-(6-méthoxy-2-naphtyl)-propionique correspondant est considérablement inférieure à la solubilité de l'autre sel diastéréo-isomère à la température de résolution.

**17.** Procédé qui consiste à préparer un milieu de résolution comprenant un mélange des sels de N-R-D-glucamine des acides (d,l) de formule I

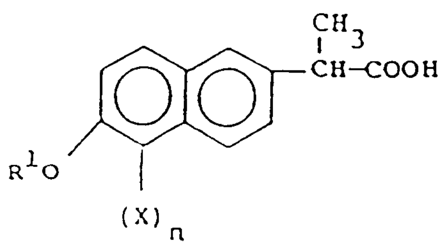

(I)

où $R^1$ représente l'hydrogène ou un groupe méthyle lorsque X représente un halogène choisi entre le brome et le chlore et n est égal à 1, ou bien $R^1$ représente l'hydrogène lorsque n est égal à 0, et R représente un groupe alkyle ayant 6 à 18 atomes de carbone ou cycloalkyle ayant 3 à 8 atomes de carbone, et un solvant dans lequel la solubilité du sel précurseur d'acide d-2-(6-méthoxy-2-naphtyl)-propionique correspondant est considérablement inférieure à la solubilité de l'autre sel diastéréo-isomère à la température de résolution.

**18.** Procédé qui consiste à utiliser de la N-R-D-glucamine, dans laquelle R représente un groupe alkyle ayant 2 à 18 atomes de carbone ou cycloalkyle ayant 3 à 8 atomes de carbone, comme agent de résolution dans la préparation d'un isomère d d'un composé de formule I

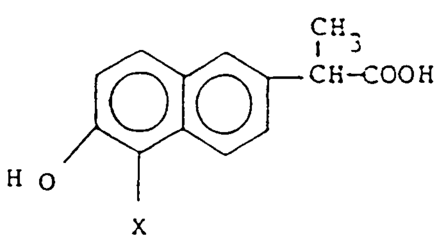

(I)

où X représente l'hydrogène, un groupe chloro ou bromo.

**19.** Procédé qui consiste à utiliser de la N-R-D-glucamine, dans laquelle R représente un groupe alkyle ayant 2 à 18 atomes de carbone ou cycloalkyle ayant 3 à 8 atomes de carbone, comme agent de résolution dans la préparation de l'isomère l d'un composé de formule I

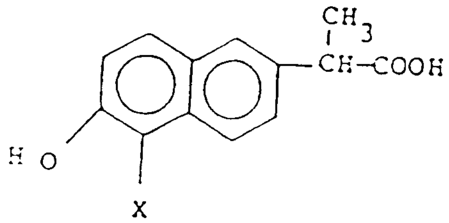

(I)

où X représente l'hydrogène, un groupe chloro ou bromo.

**20.** Procédé de production d'acide (d)-2-(6-méthoxy-2-naphtyl)propionique, qui consiste à transformer un sel de N-R-D-glucamine suivant l'une quelconque des revendications 1 à 7, ledit sel de N-R-D-glucamine étant celui de l'isomère d d'un composé de formule I, en acide (d)-2-(6-méthoxy-2-naphtyl)-propionique.

**21.** Procédé suivant la revendication 20, dans lequel l'acide (d)-2-(6-méthoxy-2-naphyl)propionique est transformé en un de ses sels pharmaceutiques acceptables.

**22.** Procédé de résolution de mélanges de précurseurs d'acides (d)- et (l)-2-(6-méthoxy-2-naphtyl)-propioniques de formule I

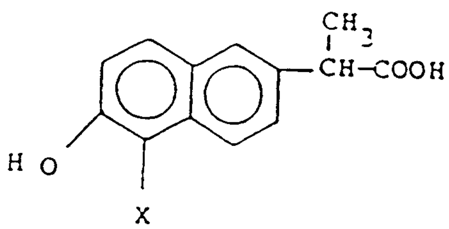

(I)

où X représente l'hydrogène, un groupe chloro ou bromo ; ou de leurs sels ;
en leurs énantiomères, caractérisé en ce que des N-R-D-glucamine ou leurs sels sont utilisés comme agent de résolution, R représentant un groupe méthyle.

**23.** Procédé de résolution de mélanges de précurseurs d'acides (d)- et (l)-2-(6-méthoxy-2-naphtyl)-propioniques de formule I

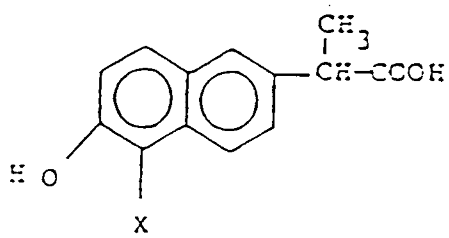

(I)

où X représente l'hydrogène, un groupe chloro ou bromo ; ou de leurs sels ;
en leurs énantiomères, caractérisé en ce que des N-R-D-glucamines ou leurs sels sont utilisés comme agent de résolution, R représentant un groupe octyle.

**24.** Procédé de résolution de mélanges de précurseurs d'acides (d)- et (l)-2-(6-méthoxy-2-naphtyl)-propioniques de formule I

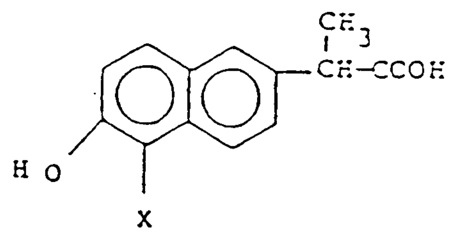

(I)

où X représente l'hydrogène, un groupe chloro ou bromo ; ou de leurs sels ;
en leurs énantiomères, caractérisé en ce que des N-R-D-glucamines ou leurs sels sont utilisés comme agent de résolution, R représentant un groupe alkyle ayant 2 à 18 atomes de carbone ou cycloalkyle ayant 3 à 8 atomes de carbone.

**25.** Procédé de résolution de mélanges de précurseurs d'acides (d)- et (l)-2-(6-méthoxy-2-naphtyl)-propioniques de formule I

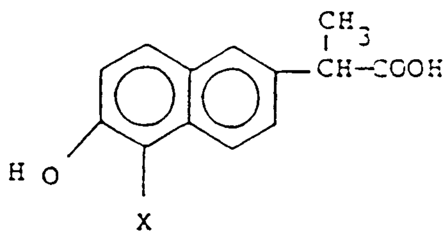

(I)

où X représente l'hydrogène, un groupe chloro ou bromo ; ou de leurs sels ;
en leurs énantiomères, caractérisé en ce que des N-R-D-glucamines ou leurs sels sont utilisés comme agent de résolution, R représentant un groupe alkyle ayant 6 à 18 atomes de carbone ou cycloalkyle ayant 3 à 8 atomes de carbone.

**26.** Procédé suivant l'une quelconque des revendications 22 à 25, dans lequel la paire résultante de sels de N-R-D-glucamine d'acides (d) et (l) est soumise à une séparation par cristallisation fractionnée.

**27.** Procédé de préparation d'acide (d)-2-(6-méthoxy-2-naphtyl)propioniqueou de ses sels pharmaceutiquement acceptables, qui consiste :
(a) à soumettre à une résolution un précurseur de formule I

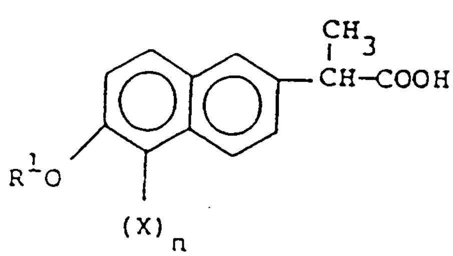

(I)

où $R^1$ représente l'hydrogène ou un groupe méthyle lorsque X représente un halogène choisi entre le brome et le chlore et n est égal à 1, ou bien dans laquelle $R^1$ représente l'hydrogène lorsque n est égal à 0, ou d'un sel d'un composé de formule (I), avec une N-R-D-glucamine dans laquelle R représente un groupe méthyle ou un de ses sels ; et
(b) à transformer le précurseur d'acide d-2-(6-méthoxy-2-naphtyl)propionique résultant en acide d-2-(6-méthoxy-2-naphtyl)propionique ;
sous réserve que, lorsque R et $R^1$ représentent des groupes méthyle et X représente un groupe bromo et n est égal à 1, la résolution soit alors effectuée dans un solvant autre qu'un mélange de toluène et de méthanol.

**28.** Procédé de préparation d'acide (d)-2-(6-méthoxy-2-naphtyl)propioniqueou de ses sels pharmaceutiquement acceptables, qui consiste :

(a) à soumettre à une résolution un composé de formule I

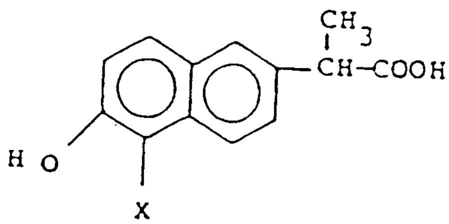

(I)

où X représente l'hydrogène, un groupe bromo ou chloro, ou un sel d'un composé de formule (I), avec une N-R-D-glucamine dans laquelle R représente un groupe méthyle, ou un de ses sels ; et

(b) à transformer le précurseur d'acide d-2-(6-méthoxy-2-naphtyl)propionique en acide d-2-(6-méthoxy-2-naphtyl)propionique.

**29.** Procédé de préparation d'acide (d)-2-(6-méthoxy-2-naphtyl)propioniqueou de ses sels pharmaceutiquement acceptables, qui consiste :

(a) à soumettre à une résolution un composé de formule I

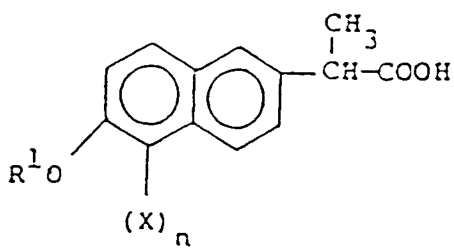

(I)

où $R^1$ représente l'hydrogène ou un groupe méthyle lorsque X représente un halogène choisi entre le brome et le chlore et n est égal à 1, ou bien dans laquelle $R^1$ représente l'hydrogène lorsque n est égal à 0, ou un sel d'un composé de formule (I), avec une N-R-D-glucamine dans laquelle R représente un groupe alkyle ayant 2 à 18 atomes de carbone ou cycloalkyle ayant 3 à 8 atomes de carbone ou un de ses sels ; et

(b) à transformer le précurseur d'acide d-2-(6-méthoxy-2-naphtyl)propionique résultant en acide d-2-(6-méthoxy-2-naphtyl)propionique.

**30.** Procédé de préparation d'acide (d)-2-(6-méthoxy-2-naphtyl)propioniqueou de ses sels pharmaceutiquement acceptables, qui consiste :

(a) à soumettre à une résolution un composé de formule I

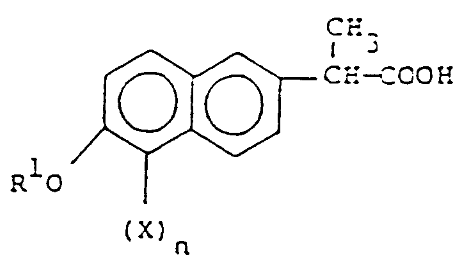

(I)

où $R^1$ représente l'hydrogène ou un groupe méthyle lorsque X représente un halogène choisi entre le brome et le chlore et n est égal à 1, ou bien dans laquelle $R^1$ représente l'hydrogène lorsque n est égal à 0, ou un sel d'un composé de formule (I), avec une N-R-D-glucamine dans laquelle R représente un groupe alkyle ayant 6 à 18 atomes de carbone ou cycloalkyle ayant 3 à 8 atomes de

carbone, ou un de ses sels ; et

(b) à transformer le précurseur d'acide d-2-(6-méthoxy-2-naphtyl)propionique résultant en acide d-2-(6-méthoxy-2-naphtyl)propionique.

**31.** Procédé suivant l'une quelconque des revendications 27 à 30, dans lequel l'acide d-2-(6-hydroxy-2-naphtyl)propionique ou un de ses sels est transformé en un acide d-2-(6-méthoxy-2-naphtyl)-propionique.

**32.** Procédé suivant l'une quelconque des revendications 27 à 30, dans lequel un acide d-2-(5-halogéno-6-méthoxy-2-naphtyl)propionique, un acide d-2-(5-halogéno-6-hydroxy-2-naphtyl)propionique ou un de leurs sels est transformé en acide d-2-(6-méthoxy-2-naphtyl)propionique.

**33.** Procédé suivant la revendication 31 ou 32, dans lequel l'acide d-2-(6-méthoxy2-naphtyl)propionique est transformé en un de ses sels pharmaceutiquement acceptables.

**34.** Procédé de résolution d'un précurseur d'acides (d)- et (l)-2-(6-méthoxy-2-napthyl)propioniques de formule I

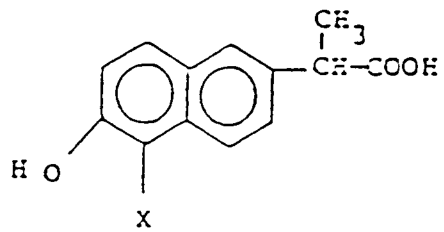

(I)

où X représente l'hydrogène, un groupe chloro ou bromo, ou de ses sels,

en leurs énantiomères, caractérisé en ce qu'une N-R-D-glucamine ou un de ses sels, dans lequel R représente un groupe alkyle ayant 1 à 18 atomes de carbone ou cycloalkyle ayant 3 à 8 atomes de carbone, est utilisé comme agent de résolution pour isoler ledit précurseur d'acide (d), et en ce que ledit précurseur d'acide (l) est racémisé, puis est soumis à une résolution.

**35.** Procédé de préparation d'acide (d)-2-(6-méthoxy-2-naphtyl)propioniqueou de ses sels pharmaceutiquement acceptables, qui consiste :

(a) à soumettre à une résolution un composé précurseur de formule I

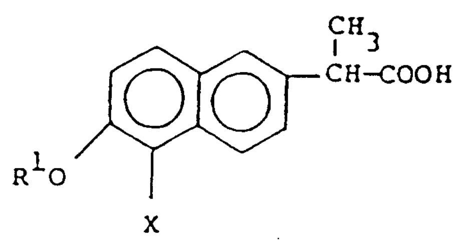

(I)

où $R^1$ représente l'hydrogène ou un groupe méthyle lorsque X représente un groupe bromo ou chloro ; ou bien $R^1$ représente l'hydrogène lorsque X représente l'hydrogène ; ou un sel d'un composé de formule I, avec une N-R-D-glucamine dans laquelle R représente un groupe alkyle ayant 1 à 18 atomes de carbone ou cycloalkyle ayant 3 à 8 atomes de carbone, ou un sel de ladite glucamine, pour isoler la forme (d) dudit composé précurseur ;

(b) à transformer ladite forme (d) en acide (d)-2-(6-méthoxy-2-naphtyl)propionique ou un de ses sels pharmaceutiquement acceptables ; et

(c) à racémiser la forme (l) dudit composé précurseur et à soumettre à une résolution la forme racémique résultante dudit composé précurseur.

**36.** Procédé de résolution d'un précurseur d'acides (d)- et (l)-2-(6-méthoxy-2-naphtyl)propioniques de formule I

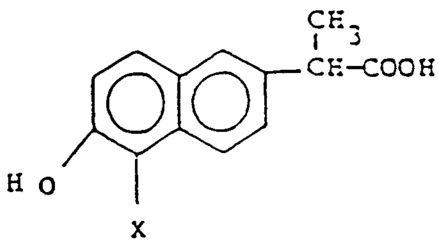

(I)

où X représente l'hydrogène, un groupe chloro ou bromo, ou de ses sels,
en leurs énantiomères, caractérisé en ce qu'une N-R-D-glucamine ou un de ses sels, dans lequel R représente un groupe alkyle ayant 2 à 8 atomes de carbone ou cycloalkyle ayant 3 à 8 atomes de carbone, est utilisé comme agent de résolution pour isoler ledit précurseur d'acide (d), et en ce que ledit précurseur d'acide (l) est racémisé, puis est soumis à une résolution.

**37.** Procédé de préparation d'acide (d)-2-(6-méthoxy-2-naphtyl)propioniqueou de ses sels pharmaceutiquement acceptables, qui consiste:
(a) à soumettre à une résolution un composé précurseur de formule I

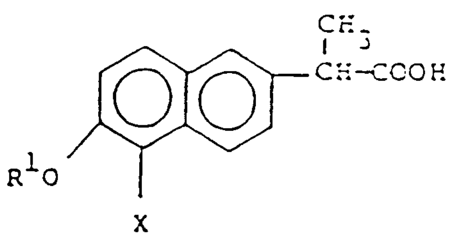

(I)

où $R^1$ représente l'hydrogène ou un groupe méthyle lorsque X représente un groupe bromo ou chloro ; ou
$R^1$ représente l'hydrogène lorsque X représente l'hydrogène,
ou un sel d'un composé de formule I,
avec une N-R-D-glucamine dans laquelle R représente un groupe alkyle ayant 2 à 8 atomes de carbone ou cycloalkyle ayant 3 à 8 atomes de carbone,
ou un sel de ladite glucamine,
pour isoler la forme (d) dudit composé précurseur ;
(b) à transformer ladite forme (d) en acide (d)-2-(6-méthoxy-2-naphtyl)propionique ou un de ses sels pharmaceutiquement acceptables ; et
(c) à racémiser la forme (l) dudit composé précurseur et à soumettre à une résolution la forme racémique résultante dudit composé précurseur.

**Revendications pour l'Etat contractant suivant : CH**

**1.** Sel de N-R-D-glucamine d'un composé d ou l de formule I

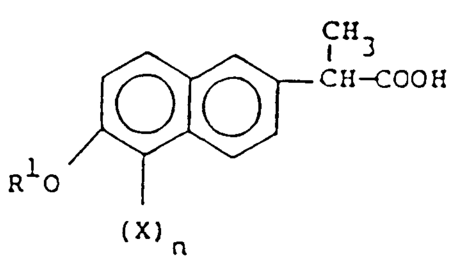

(I)

où R représente un groupe méthyle et $R^1$ représente l'hydrogène ou un groupe méthyle lorsque X représente un halogène choisi entre le chlore et le brome et n est égal à 1 ; ou bien $R^1$ représente l'hydrogène lorsque n est égal à 0.

**2.** Sel de N-R-D-glucamine d'un composé d ou l de formule I

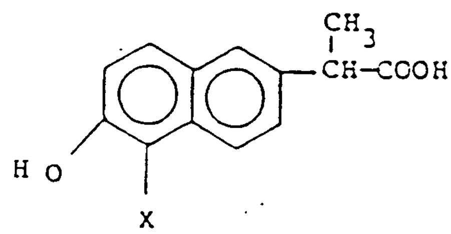

(I)

où R représente un groupe méthyle et X représente l'hydrogène, un groupe chloro ou bromo.

**3.** Sel de N-R-D-glucamine d'un composé d ou l de formule I

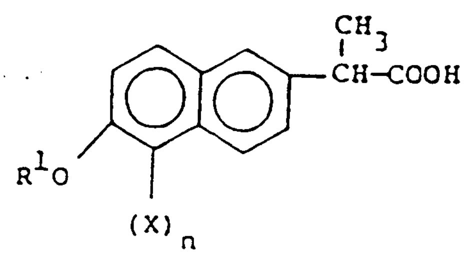

(I)

où R représente un groupe alkyle ayant 2 à 18 atomes de carbone ou cycloalkyle ayant 3 à 8 atomes de carbone, et $R^1$ représente l'hydrogène ou un groupe méthyle lorsque X représente un halogène choisi entre le chlore et le brome et n est égal à 1 ; ou bien $R^1$ représente l'hydrogène lorsque n est égal à 0.

**4.** Sel de N-R-D-glucamine d'un composé d ou l de formule I

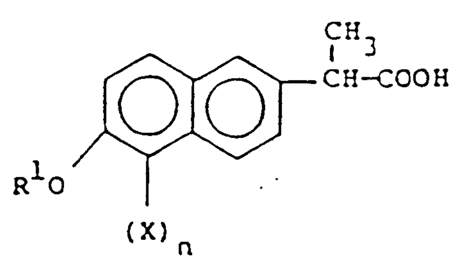

(I)

où R représente un groupe alkyle ayant 6 à 18 atomes de carbone ou cycloalkyle ayant 3 à 8 atomes de carbone, et $R^1$ représente l'hydrogène ou un groupe méthyle lorsque X représente un halogène choisi entre le chlore et le brome et n est égal à 1 ; ou bien $R^1$ représente l'hydrogène lorsque n est égal à 0.

**5.** Sel de N-R-D-glucamine suivant la revendication 1 ou la revendication 2, dans lequel R représente un groupe méthyle ; et X représente un groupe bromo.

**6.** Sel de N-R-D-glucamine suivant la revendication 3 ou la revendication 4, dans lequel R représente un groupe n-octyle.

**7.** Mélange des sels de N-R-D-glucamine des acides (d,l) de formule I

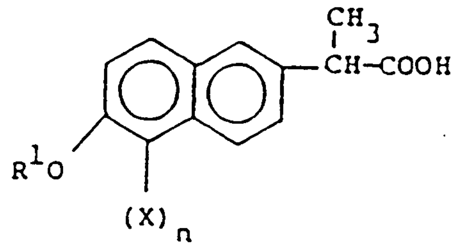

(I)

où $R^1$ représente l'hydrogène ou un groupe méthyle lorsque X représente un halogène choisi entre le brome et le chlore et n est égal à 1 ; ou bien $R^1$ représente l'hydrogène lorsque n est égal à 0 ; et R représente un groupe méthyle.

**8.** Mélange des sels de N-R-D-glucamine des acides (d,l) de formule I

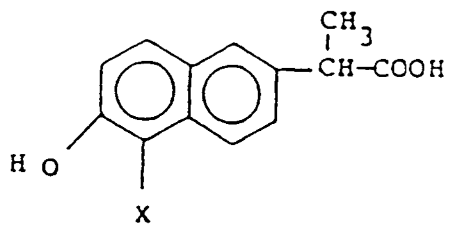

(I)

où X représente l'hydrogène, un groupe bromo ou chloro et R représente un groupe méthyle.

**9.** Mélange des sels de N-R-D-glucamine des acides (d,l) de formule I

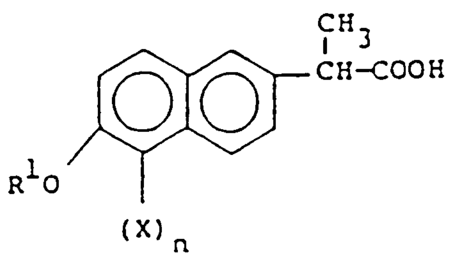

(I)

où $R^1$ représente l'hydrogène ou un groupe méthyle lorsque X représente un halogène choisi entre le brome et le chlore et n est égal à 1 ; ou bien $R^1$ représente l'hydrogène lorsque n est égal à 0, et R représente un groupe alkyle ayant 2 à 18 atomes de carbone ou cycloalkyle ayant 3 à 8 atomes de carbone.

**10.** Mélange des sels de N-R-D-glucamine des acides (d,l) de formule I

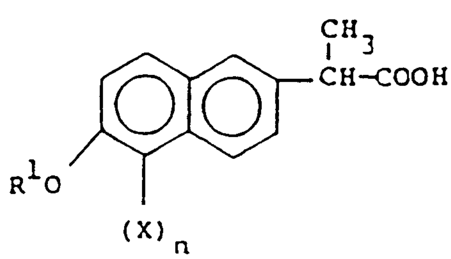

(I)

où $R^1$ représente l'hydrogène ou un groupe méthyle lorsque X représente un halogène choisi entre le brome et le chlore et n est égal à 1 ; ou bien $R^1$ représente l'hydrogène lorsque n est égal à 0 et R représente un groupe alkyle ayant 6 à 18 atomes de carbone ou cycloalkyle ayant 3 à 8 atomes de carbone.

**11.** Milieu de résolution comprenant un mélange des sels de N-R-D-glucamine des acides (d,l) de formule I

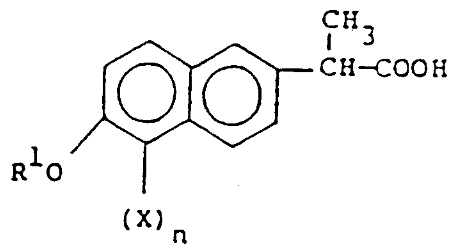

(I)

où $R^1$ représente l'hydrogène ou un groupe méthyle lorsque X représente un halogène choisi entre le brome et le chlore et n est égal à 1 ; ou bien $R^1$ représente l'hydrogène lorsque n est égal à 0, et R représente un groupe méthyle, et un solvant dans lequel la solubilité du sel précurseur d'acide d-2-(6-méthoxy-2-naphtyl)propionique correspondant est considérablement inférieure à la solubilité de l'autre sel diastéréo-isomère à la température de résolution.

**12.** Milieu de résolution comprenant un mélange des sels de N-R-D-glucamine des acides (d,l) de formule I

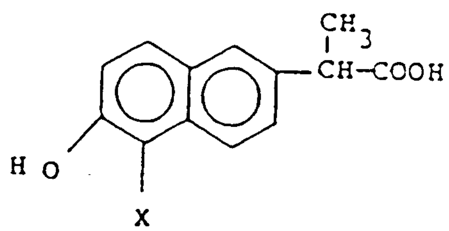

(I)

où X représente l'hydrogène, un groupe bromo ou chloro et R représente un groupe méthyle, et un solvant dans lequel la solubilité du sel précurseur d'acide d-2-(6-méthoxy-2-naphtyl)propionique correspondant est considérablement inférieure à la solubilité de l'autre sel diastéréo-isomère à la température de résolution.

**13.** Milieu de résolution comprenant un mélange des sels de N-R-D-glucamine des acides (d,l) de formule I

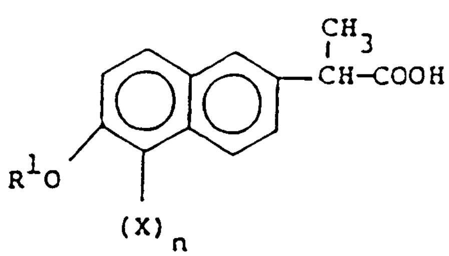

(I)

où $R^1$ représente l'hydrogène ou un groupe méthyle lorsque X représente un halogène choisi entre le brome et le chlore et n est égal à 1, ou bien $R^1$ représente l'hydrogène lorsque n est égal à 0, et R représente un groupe alkyle ayant 2 à 18 atomes de carbone ou cycloalkyle ayant 3 à 8 atomes de carbone, et un solvant dans lequel la solubilité du sel précurseur d'acide d-2-(6-méthoxy-2-naphtyl)-propionique correspondant est considérablement inférieure à la solubilité de l'autre sel diastéréo-isomère à la température de résolution.

**14.** Milieu de résolution comprenant un mélange des sels de N-R-D-glucamine des acides (d,l) de formule I

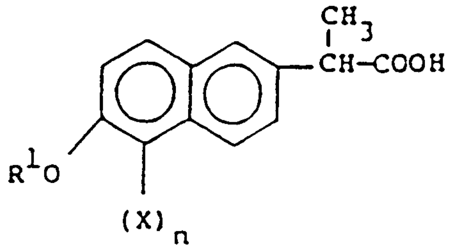

(I)

où $R^1$ représente l'hydrogène ou un groupe méthyle lorsque X représente un halogène choisi entre le brome et le chlore et n est égal à 1, ou bien $R^1$ représente l'hydrogène lorsque n est égal à 0, et R représente un groupe alkyle ayant 6 à 18 atomes de carbone ou cycloalkyle ayant 3 à 8 atomes de carbone, et un solvant dans lequel la solubilité du sel précurseur d'acide d-2-(6-méthoxy-2-naphtyl)-propionique correspondant est considérablement inférieure à la solubilité de l'autre sel diastéréoisomère à la température de résolution.

**15.** Isomère d d'un composé de formule I

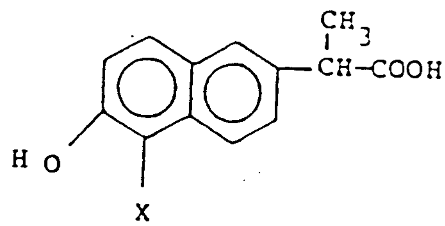

(I)

où X représente l'hydrogène, un groupe chloro ou bromo.

**16.** Isomère l d'un composé de formule I

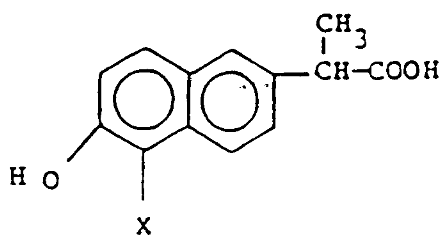

(I)

où X représente l'hydrogène, un groupe chloro ou bromo.

**17.** Procédé de production d'acide (d)-2-(6-méthoxy-2-naphtyl)propionique, qui consiste à transformer un sel de N-R-D-glucamine suivant l'une quelconque des revendications 1 à 6, ledit sel de N-R-D-glucamine étant celui de l'isomère d d'un composé de formule I, en acide (d)-2-(6-méthoxy-2-naphtyl)-propionique.

**18.** Procédé suivant la revendication 17, dans lequel l'acide (d)-2-(6-méthoxy-2-naphtyl)propionique est transformé en un de ses sels pharmaceutiquement acceptables.

**19.** Procédé de résolution de mélanges de précurseurs d'acides (d)- et (l)-2-(6-méthoxy-2-naphtyl)-propioniques de formule I

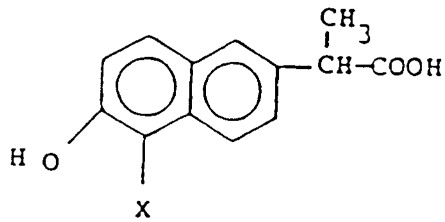

(I)

où X représente l'hydrogène, un groupe chloro ou bromo ; ou de leurs sels ;
en leurs énantiomères, caractérisé en ce que des N-R-D-glucamines ou leurs sels sont utilisés comme agent de résolution, R représentant un groupe méthyle.

**20.** Procédé de résolution de mélanges de précurseursd'acides (d) et (l)-2-(6-méthoxy-2-naphtyl)-propioniques de formule I

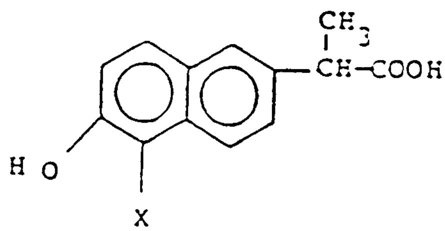

(I)

où X représente l'hydrogène, un groupe chloro ou bromo ; ou de leurs sels ;
en leurs énantiomères, caractérisé en ce que des N-R-D-glucamines ou leurs sels sont utilisés comme agent de résolution, R représentant un groupe octyle.

**21.** Procédé de résolution de mélanges de précurseurs d'acides (d)- et (l)-2-(6-méthoxy-2-naphtyl)-propioniques de formule I

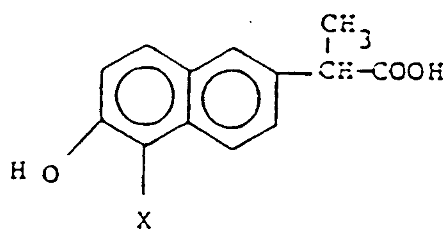

(I)

où X représente l'hydrogène, un groupe chloro ou bromo ; ou de leurs sels ;
en leurs énantiomères, caractérisé en ce que des N-R-D-glucamines ou leurs sels sont utilisés comme agent de résolution, R représentant un groupe alkyle ayant 2 à 18 atomes de carbone ou cycloalkyle ayant 3 à 8 atomes de carbone.

**22.** Procédé de résolution de mélanges de précurseurs d'acides (d)- et (l)-2-(6-méthoxy-2-naphtyl)-propioniques de formule I

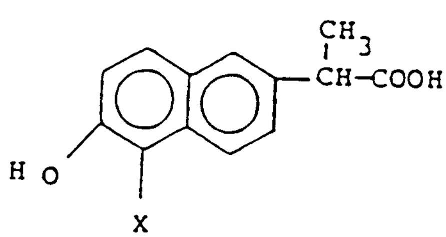

(I)

où X représente l'hydrogène, un groupe chloro ou bromo ; ou de leurs sels ;
en leurs énantiomères, caractérisé en ce que des N-R-D-glucamines ou leurs sels sont utilisés comme agent de résolution, R représentant un groupe alkyle ayant 6 à 18 atomes de carbone ou cycloalkyle ayant 3 à 8 atomes de carbone.

**23.** Procédé suivant l'une quelconque des revendications 19 à 22, dans lequel la paire résultante de sels de N-R-D-glucamine d'acides (d) et (l) est séparée par cristallisation fractionnée.

**24.** Procédé de préparation d'acide (d)-2-(6-méthoxy-2-naphtyl)propioniqueou de ses sels pharmaceutiquement acceptables, qui consiste :
(a) à soumettre à une résolution un précurseur de formule I

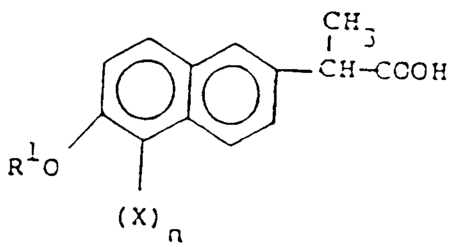

(I)

où $R^1$ représente l'hydrogène ou un groupe méthyle lorsque X représente un halogène choisi entre le brome et le chlore et n est égal à 1, ou bien $R^1$ représente l'hydrogène lorsque n est égal à 0, ou un sel d'un composé de formule (I), avec une N-R-D-glucamine dans laquelle R représente un groupe méthyle ou un de ses sels ; et
(b) à transformer le précurseur d'acide d-2-(6-méthoxy-2-naphtyl)propionique résultant en acide d-2-(6-méthoxy-2-naphtyl)propionique ;
à l'exception du procédé dans lequel l'agent de résolution consistant en N-méthyl-D-glucamine et le précurseur consistant en acide dl-2-(5-halogéno-6-méthoxy-2-naphtyl)propionique sont amenés à réagir dans un milieu servant de solvant choisi entre des mélanges de toluène et de méthanol en présence d'une base organique ou inorganique optiquement inactive à une température comprise dans l'intervalle de la température ambiante à 65 ° C, le sel moins soluble de N-méthyl-D-glucamine de l'isomère d étant obtenu et mis en contact avec un acide minéral fort pour obtenir un acide d-2-(5-halogéno-6-méthoxy-2-naphtyl)propionique, qui est soumis à une déshalogénation catalytique par traitement avec un agent d'hydrogénation convenable qui est capable de remplacer totalement le groupe 5-halogéno par l'hydrogène dans un milieu alcalin à une température comprise dans l'intervalle de la température ambiante à 100 ° C pendant un temps de 1 à 4 heures.

**25.** Procédé de préparation d'acide (d)-2-(6-méthoxy-2-naphtyl)propioniqueou de ses sels pharmaceutiquement acceptables, qui consiste :
(a) à soumettre à une résolution un composé de formule I

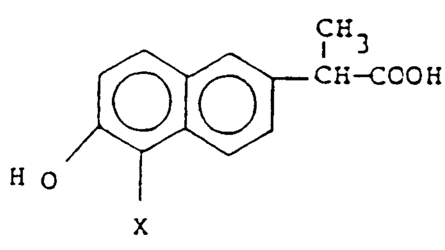

(I)

où X représente l'hydrogène, un groupe bromo ou chloro, ou bien un sel d'un composé de formule (I), avec une N-R-D-glucamine dans laquelle R représente un groupe méthyle, ou un de ses sels ; et
(b) à transformer le précurseur d'acide d-2-(6-méthoxy-2-naphtyl)propionique résultant en acide d-2-(6-méthoxy-2-naphtyl)propionique.

**26.** Procédé de préparation d'acide (d)-2-(6-méthoxy-2-naphtyl)propioniqueou de ses sels pharmaceutiquement acceptables, qui consiste :

(a) à soumettre à une résolution un composé de formule (I)

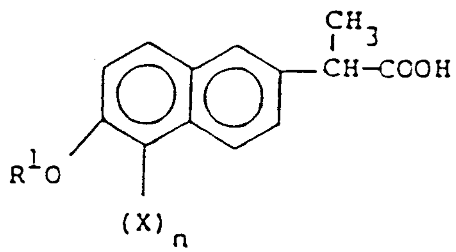

(I)

où $R^1$ représente l'hydrogène ou un groupe méthyle lorsque X représente un halogène choisi entre le brome ou le chlore et n est égal à 1, ou bien $R^1$ représente l'hydrogène lorsque n est égal à 0, ou un sel d'un composé de formule (I), avec une N-R-D-glucamine dans laquelle R représente un groupe alkyle ayant 2 à 18 atomes de carbone ou cycloalkyle ayant 3 à 8 atomes de carbone ou un de ses sels ; et

(b) à transformer le précurseur d'acide d-2-(6-méthoxy-2-naphtyl)propionique résultant en acide d-2-(6-méthoxy-2-naphtyl)propionique.

**27.** Procédé de préparation d'acide (d)-2-(6-méthoxy-2-naphtyl)propioniqueou de ses sels pharmaceutiquement acceptables, qui consiste :

(a) à soumettre à une résolution un composé de formule I

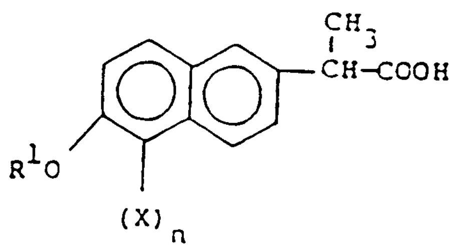

(I)

où R représente l'hydrogène ou un groupe méthyle lorsque X représente un halogène choisi entre le brome et le chlore et n est égal à 1, ou bien où $R^1$ représente l'hydrogène lorsque n est égal à 0, ou un sel d'un composé de formule (I), avec une N-R-D-glucamine dans laquelle R représente un groupe alkyle ayant 6 à 18 atomes de carbone ou cycloalkyle ayant 3 à 8 atomes de carbone, ou un de ses sels ; et

(b) à transformer le précurseur d'acide d-2-(6-méthoxy-2-naphtyl)propionique résultant en acide d-2-(6-méthoxy-2-naphtyl)propionique.

**28.** Procédé suivant l'une quelconque des revendications 24 à 27, dans lequel l'acide d-2-(6-hydroxy-2-naphtyl)propionique ou un de ses sels est transformé en un acide d-2-(6-méthoxy-2-naphtyl)-propionique.

**29.** Procédé suivant l'une quelconque des revendications 24 à 27, dans lequel un acide d-2-(5-halogéno-6-méthoxy-2-naphtyl)propionique, un acide d-2-(5-halogéno-6-hydroxy-2-naphtyl)propionique ou un de leurs sels est transformé en acide d-2-(6-méthoxy-2-naphtyl)propionique.

**30.** Procédé suivant la revendication 28 ou 29, dans lequel l'acide d-2-(6-méthoxy-2-naphtyl)propionique est transformé en un de ses sels pharmaceutiquement acceptables.

**31.** Procédé de résolution d'un précurseur d'acides (d)- et (l)-2-(6-méthoxy-2-naphtyl)propioniques de formule I

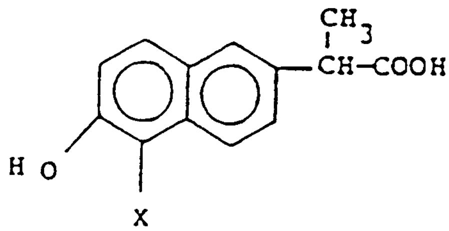

(I)

où X représente l'hydrogène, un groupe chloro ou bromo, ou de ses sels,

en leurs énantiomères, caractérisé en ce qu'une N-R-D-glucamine ou un de ses sels, dans lequel R représente un groupe alkyle ayant 1 à 18 atomes de carbone ou cycloalkyle ayant 3 à 8 atomes de carbone, est utilisé comme agent de résolution pour isoler ledit précurseur d'acide (d), et en ce que ledit précurseur d'acide (l) est racémisé, puis est soumis à une résolution.

**32.** Procédé de préparation d'acide (d)-2-(6-méthoxy-2-naphtyl)propioniqueou de ses sels pharmaceutiquement acceptables, qui consiste :

(a) à soumettre à une résolution un composé précurseur de formule I

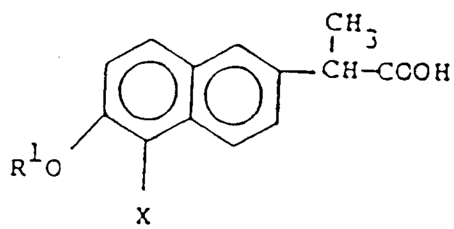

(I)

où $R^1$ représente l'hydrogène ou un groupe méthyle lorsque X représente un groupe bromo ou chloro ; ou bien $R^1$ représente l'hydrogène lorsque X représente l'hydrogène ; ou un sel d'un composé de formule I, avec une N-R-D-glucamine dans laquelle R représente un groupe alkyle ayant 1 à 18 atomes de carbone ou cycloalkyle ayant 3 à 8 atomes de carbone, ou un sel de ladite glucamine, pour isoler la forme (d) dudit composé précurseur ;

(b) à transformer ladite forme (d) en acide (d)-2-(6-méthoxy-2-naphtyl)propionique ou un de ses sels pharmaceutiquement acceptables ; et

(c) à racémiser la forme (l) dudit composé précurseur et à soumettre à une résolution la forme racémique résultante dudit composé précurseur.

**33.** Procédé de résolution d'un précurseur d'acides (d)- et (l)-2-(6-méthoxy-2-naphtyl)propioniques de formule I

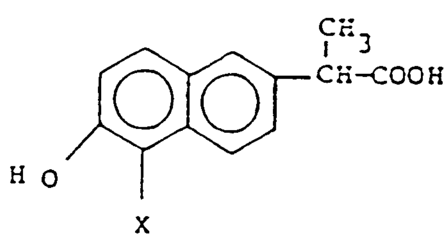

(I)

où X représente l'hydrogène, un groupe chloro ou bromo, ou de ses sels,

en leurs énantiomères, caractérisé en ce qu'une N-R-D-glucamine ou un de ses sels, dans lequel R représente un groupe alkyle ayant 2 à 8 atomes de carbone ou cycloalkyle ayant 3 à 8 atomes de carbone, est utilisé comme agent de résolution pour isoler ledit précurseur d'acide (d), et en ce que ledit précurseur d'acide (l) est racémisé, puis est soumis à une résolution.

**34.** Procédé de préparation d'acide (d)-2-(6-méthoxy-2-naphtyl)propioniqueou de ses sels pharmaceutiquement acceptables, qui consiste:

(a) à soumettre à une résolution un composé précurseur de formule I

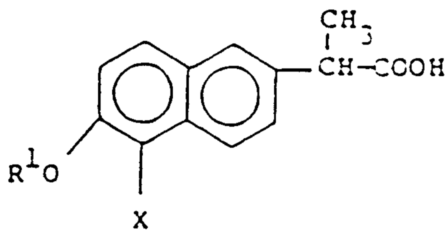

(I)

où $R^1$ représente l'hydrogène ou un groupe méthyle lorsque X représente un groupe bromo ou chloro ; ou bien
$R^1$ représente l'hydrogène lorsque X représente l'hydrogène,
ou un sel d'un composé de formule I,
avec une N-R-D-glucamine dans laquelle R représente un groupe alkyle ayant 2 à 8 atomes de carbone ou cycloalkyle ayant 3 à 8 atomes de carbone,
ou un sel de ladite glucamine,
pour isoler la forme (d) dudit composé précurseur ;
(b) à transformer ladite forme (d) en acide (d)-2-(6-méthoxy-2-naphtyl)propionique ou un de ses sels pharmaceutiquement acceptables ; et
(c) à racémiser la forme (l) dudit composé précurseur et à soumettre à une résolution la forme racémique résultante dudit composé précurseur.